# EUROPEAN PATENT SPECIFICATION

(11) **EP 3 079 695 B1**
(45) Date of publication and mention of the grant of the patent: **17.03.2021**
(21) Application number: 14824245.6
(22) Date of filing: 09.12.2014
(51) Int. Cl.: A61K 31/439, A61P 9/00

(54) **GLUCOSYLCERAMIDE SYNTHASE INHIBITORS**
GLUCOSYLCERAMID-SYNTHASE-HEMMER
INHIBITEURS DE LA GLUCOSYLCÉRAMIDE SYNTHASE

(30) Priority: 11.12.2013 US 201361914842 P
(43) Date of publication of application: 19.10.2016
(73) Proprietor: Genzyme Corporation, Cambridge, MA 02142 (US)
(72) Inventor: LEONARD, John P., Bridgewater, New Jersey 08807 (US); DELEUZE, Jean-François, F-75008 Paris (FR); ITIER, Jean-Michel, F-75008 Paris (FR); ORSINI, Cécile, F-75008 Paris (FR); RET-LECUELLE, Gwénaëlle, F-75008 Paris (FR); VIALE, Sandra, F-75008 Paris (FR)
(74) Representative: Garner, Stephen
(86) International application number: PCT/US2014/069338
(87) International publication number: WO 2015/089067

(56) References cited:
- WO-A1-2014/043068
- WO-A1-2014/152215
- WO-A2-2012/129084
- URBANELLI LORENA ET AL: "Therapeutic approaches for lysosomal storage diseases: a patent update.", RECENT PATENTS ON CNS DRUG DISCOVERY AUG 2013, vol. 8, no. 2, August 2013 (2013-08), pages 91-109, XP002735910, ISSN: 2212-3954
- GAMBARIN ET AL.: "When Should Cardiologists Suspect Anderson-Fabry disease ?", AMERICAN JOURNAL OF CARDIOLOGY, vol. 106, no. 10, November 2010 (2010-11), pages 1492-1499, XP002735911, DOI: 10.1016/J.AMJCARD.2010.07.016
- Paula A. Rozenfeld ET AL: "Myocardial Alterations in the Murine Model of Fabry Disease Can Be Reversed by Enzyme Replacement Therapy", CANADIAN JOURNAL OF CARDIOLOGY., vol. 27, no. 3, 1 May 2011 (2011-05-01), pages 339-345, XP055458517, CANADA ISSN: 0828-282X, DOI: 10.1016/j.cjca.2010.12.035
- DINESH S. BANGARI ET AL: "[alpha]-Galactosidase A Knockout Mice", AMERICAN JOURNAL OF PATHOLOGY., vol. 185, no. 3, 1 March 2015 (2015-03-01) , pages 651-665, XP055614044, US ISSN: 0002-9440, DOI: 10.1016/j.ajpath.2014.11.004
- Beth L. Thurberg ET AL: "Cardiac Microvascular Pathology in Fabry Disease : Evaluation of Endomyocardial Biopsies Before and After Enzyme Replacement Therapy", Circulation, vol. 119, no. 19, 19 May 2009 (2009-05-19) , pages 2561-2567, XP055614048, US ISSN: 0009-7322, DOI: 10.1161/CIRCULATIONAHA.108.841494

## Description

### FIELD OF THE INVENTION

The present invention relates generally to the field of therapeutics for metabolic diseases. More specifically, the invention relates to inhibitors of glucosylceramide synthase (GCS) useful for the treatment of metabolic diseases, such as lysosomal storage diseases, either alone or in combination with enzyme replacement therapy.

### SUMMARY OF THE INVENTION

The present invention provides compounds for use in a method according to appended Claim 1.

Fabry disease (FD) is a rare X-linked lysosomal storage disorder characterized by deficient activity of the enzyme α-galactosidase A (a-Gal A) encoded by the GLA gene. Enzyme deficiency results in the progressive intracellular accumulation of glycosphingolipids, mostly globotriaosylceramide (GL-3) in a variety of cell types and tissues including kidney, heart, liver, spleen, skin as well as the peripheral and central nervous systems.

Although multiple organ-specific cell types accumulate GL-3 and are important to consider and without being limited as to theory, the systemic accumulation in capillary endothelial cells is believed to play a major role in the general pathological process that leads to the devastating renal, cardiac, and cerebrovascular clinical manifestations of the disease and to substantially decreased life expectancy. FD is a progressive disorder that takes years to advance to end stage organ failure mainly in hemizygous males. Although the αGAL gene mutation is carried by the X chromosome, females have a variable phenotype, ranging from asymptomatic to a presentation as severe as in male patients. The initial signs and symptoms of FD often begin during childhood and frequently include neuropathic pain in the extremities, hypohidrosis, angiokeratomas, and gastrointestinal discomfort. Over a period of decades, the progressive accumulation of glycosphingolipids impairs vital organ function, putting patients with FD at risk of developing renal failure, cardiovascular dysfunction, and stroke. However, it is now clear that tissue damage, such as fibrosis in heart and kidney, starts well before the onset of organ failure.

Cardiac manifestations of FD can include progressive left-ventricular hypertrophy, arrhythmia, conduction defects, valvular dysfunction and angina. As the disease progresses, myocardial fibrosis develops that can lead to life-threatening cardiac events.

Enzyme replacement therapy (ERT) with recombinant α-GalA (agalsidase beta and agalsidase alpha) represents the only approved disease-specific therapeutic approach for the treatment of Fabry disease. Agalsidase beta has been shown to consistently clear GL-3 deposits in capillary endothelium in all tissues evaluated, in renal mesangial and interstitial cells, and to clear GL-3 in podocytes in a dose dependent manner. In contrast, GL-3 deposits in cardiomyocytes were not reduced by agalsidase beta after 5 and 12 months of treatment in the patients that underwent cardiac biopsies, despite effective clearance of GL-3 from interstitial capillary endothelial cells in endomyocardial biopsies. Thus, clearance of GL-3 from cardiomyocytes of Fabry patients has not been shown to date.

Substrate reduction therapy (SRT) is an approach that reduces the synthesis of lipids reaching the lysosome through inhibition of glucosylceramide synthase (GCS), the rate limiting enzyme in glycosphingolipid synthesis. As GL-1 serves as the central building block for the synthesis of more complex glycosphingolipids, SRT with GCS inhibitors offers a potential therapeutic strategy for other glycosphingolipidoses, including Fabry disease.

SRT has been evaluated in α-Gal A deficient mice (murine model of Fabry disease) that accumulates GL-3 in a variety of tissues. Published reports have shown that GCS inhibition can delay the accumulation of tissue GL-3 and lower GL-3 in both plasma and urine of Fabry mice. However, the cellular distribution of GL-3 in Fabry mice does not mirror that observed in Fabry patients as GL-3 does not accumulate in cardiomyocytes and these mice fail to develop cardiac dysfunction with age.

Accordingly, there is a need for developing novel therapies to clear GL-3 accumulation in Fabry cardiomyocytes. In particular, it would be desirable to develop a SRT with a small molecule targeting cardiomyocytes in the heart, and capable of preventing cardiomyocyte GL-3 accumulation. There is also a need for developing new models of Fabry disease to evaluate the effects of therapeutic candidates on cellular GL3 accumulation.

As shown in the examples, the inventors of the present application have found that an inhibitor of glucosylceramide synthase (GCS), further described herein, is capable of preventing GL-3 accumulation in cardiomyocytes (e.g., Fabry-disease induced pluripotent stem cell (iPSC)-derived cardiomyocytes). The present invention is therefore based, at least in part, on the finding that inhibitors of glucosylceramide synthase (GCS) are effective in treating cardiomyocytes in a subject having or at risk of having a glycosphingolipid disease or disorder. Accordingly, the invention features methods for using GCS inhibitors to treat cardiomyocytes. The invention also features compositions for use in treating cardiomyocytes.

In aspects, the invention relates to methods for treating a GL-3 disease or disorder in a cardiomyocyte using a compound described herein.

In aspects, the invention relates to methods for treating increased GL-3 in a cardiomyocyte using a compound described herein.

In aspects, the invention relates to methods for treating GL-3 accumulation in a cardiomyocyte using a compound described herein.

In embodiments, the glycosphingolipid disease or disorder is associated with increased globotriaosylceramide (GL-3) in the cardiomyocyte as compared to a healthy cardiomyocyte (e.g. cardiomyocyte from a healthy, or non-Fabry subject). In related embodiments, increased GL-3 is present, at least in part, in the lysosome of the cardiomyocyte.

In some embodiments, administration of the compound maintains (e.g., prevents further increase in GL-3 levels or prevents further GL-3 accumulation) GL-3 levels in the cardiomyocyte. In related embodiments, the compound maintains (e.g., prevents further increase in GL-3 levels or prevents further GL-3 accumulation) GL-3 levels in the lysosome of the cardiomyocyte.

In some embodiments, administration of the compound reduces GL-3 in the cardiomyocyte. In related embodiments, the compound reduces GL-3 in the lysosome of the cardiomyocyte.

In embodiments, the glycosphingolipid disease or disorder is associated with globotriaosylceramide (GL-3) accumulation in the cardiomyocyte. In related embodiments, GL-3 accumulation is present in the lysosome of the cardiomyocyte.

In some embodiments, administration of the compound inhibits GL-3 accumulation in the cardiomyocyte. In related embodiments, administration of the compound inhibits GL-3 accumulation in the lysosome of the cardiomyocyte.

In some embodiments, administration of the compound reduces GL-3 accumulation in the cardiomyocyte. In related embodiments, administration of the compound reduces GL-3 accumulation in the lysosome of the cardiomyocyte.

In some embodiments, administration of the compound prevents GL-3 accumulation in the cardiomyocyte. In related embodiments, administration of the compound prevents GL-3 accumulation in the cardiomyocyte.

In the above aspects and embodiments, administration of the compound can inhibit glucosylceramide synthase (GCS) activity.

In the above aspects and embodiments, the cardiomyocyte can be in the heart of a subject.

In embodiments, the subject has or is at risk of having the glycosphingolipid disease or disorder. In some embodiments, the glycosphingolipid disease or disorder is a metabolic disorder. In some embodiments, the glycosphingolipid disease or disorder is a metabolic disorder. In some embodiments, the glycosphingolipid disease or disorder is a lysosomal storage disease (e.g., Gaucher, Fabry, G_{M1}-gangliosidosis, G_{M2} Activator Deficiency, Tay-Sachs and Sandhoff). In some embodiments, the glycosphingolipid disease or disorder is a GL-3 disease or disorder. In some embodiments, the glycosphingolipid disease or disorder is Fabry disease.

In the above aspects and embodiments, the method can further involve administering a therapeutically effective amount of a lysosomal enzyme (e.g., glucocerebrosidase, alpha-galactosidase A, Hexosaminidase A, Hexosaminidase B and G_{M1}-ganglioside-β-galactosidase) to the subject. In embodiments, the lysosomal enzyme is alpha-galactosidase A.

In embodiments, the subject has elevated levels of a lysosomal substrate (e.g., globotriaosylceramide and lyso-globotriaosylceramide, or a combination thereof) prior to treatment. In some embodiments, the subject undergoing treatment has lower combined amounts of the lysosomal substrate in the urine and plasma than a subject treated with either the lysosomal enzyme or the compound alone.

In aspects, the invention relates to methods for treating globotriaosylceramide (GL-3) accumulation in the heart of a subject having or at risk of having Fabry disease. The methods involve administering an effective amount of a compound described herein to the subject.

In some embodiments, administration of the compound inhibits GL-3 accumulation in a cardiomyocyte of the subject. In related embodiments, administration of the compound inhibits GL-3 accumulation in the lysosome of the cardiomyocyte.

In some embodiments, administration of the compound reduces GL-3 accumulation in a cardiomyocyte of the subject. In related embodiments, administration of the compound reduces GL-3 accumulation in the lysosome of the cardiomyocyte.

In some embodiments, administration of the compound prevents GL-3 accumulation in a cardiomyocyte of the subject. In related embodiments, administration of the compound prevents GL-3 accumulation in the lysosome of the cardiomyocyte.

In the above aspects and embodiments, administration of the compound can inhibit glucosylceramide synthase (GCS) activity.

In aspects, the invention relates to methods for reducing globotriaosylceramide (GL-3) in the heart of a subject having or at risk of having Fabry disease. The methods involve administering an effective amount of a compound described herein to the subject. In embodiments, the subject has increased GL-3 as compared to healthy cardiomyocytes. In related embodiments, the increased GL-3 is present, at least in part, in the lysosomes of the cardiomyocytes.

In some embodiments, administration of the compound maintains (e.g., prevents further increase in GL-3 levels or prevents further GL-3 accumulation) GL-3 levels in the cardiomyocytes. In related embodiments, administration of the compound maintains (e.g., prevents further increase in GL-3 levels or prevents further GL-3 accumulation) GL-3 levels in the lysosome of the cardiomyocytes.

In some embodiments, administration of the compound reduces GL-3 in the cardiomyocytes. In related embodiments, administration of the compound reduces GL-3 in the lysosome of the cardiomyocytes.

In the above aspects and embodiments, the cardiomyocytes of the subject can contain accumulated GL-3. In embodiments, the accumulated GL-3 is present in the lysosomes of the cardiomyocytes.

In the above aspects and embodiments, administration of the compound can inhibit glucosylceramide synthase (GCS) activity.

In the above aspects and embodiments, the methods can further involve administering a therapeutically effective amount of alpha-galactosidase A to the subject. In embodiments, the subject can have elevated levels of globotriaosylceramide, lyso-globotriaosylceramide, or combinations thereof prior to treatment. In some embodiments, the subject undergoing treatment has lower combined amounts of globotriaosylceramide or lyso-globotriaosylceramide in the urine and plasma than a subject treated with either the alpha-galactosidase A or the compound alone.

In the above aspects and embodiments, administration of the compound slows progression of the disease or disorder, reduces intensity of symptoms associated with the disease or disorder, delays onset of symptoms associated with the disease or disorder, delays mortality, or combinations thereof.

In the above aspects and embodiments, administration of the compound reduces GL-3 levels or GL-3 accumulation by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or any number therebetween in the cardiomyocyte (or the lysosome of the cardiomyocyte).

In aspects, the invention provides pharmaceutical composition for use in any of the methods described herein. In embodiments, the pharmaceutical composition further comprises a pharmaceutically acceptable carrier, diluent, or excipient.

In aspects, the invention provides a compound described herein for use in any of the methods described herein.

In the above aspects and embodiments, the compound is represented by the following structural formula, 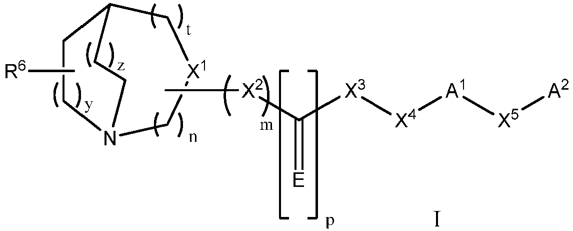 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR² ;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂ -(C₁-C₆) alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
   with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

In some embodiments, the compound can be

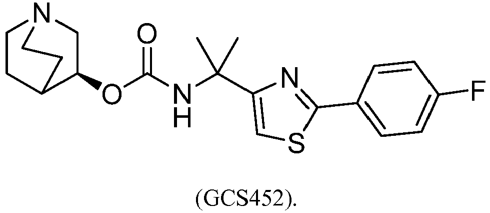

In some embodiments, the compound can be

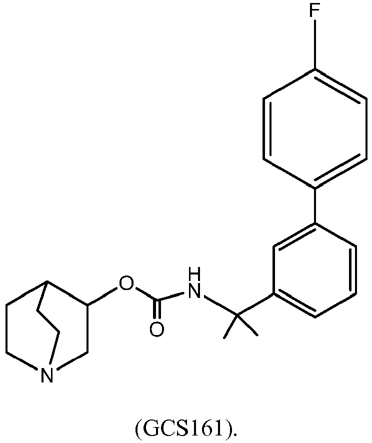

The present invention refers to a compound represented by the following structural formula, 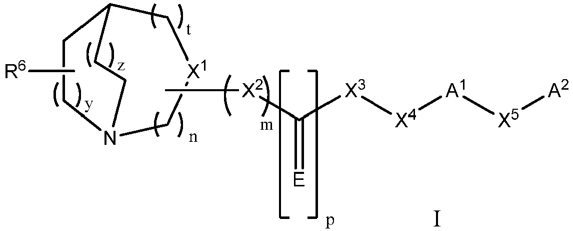 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR² ;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂ -(C₁-C₆) alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆) dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl. Certain aspects of the invention include administering the foregoing compound to a patient as part of combination therapy that includes an enzyme replacement therapy (ERT) and small molecule therapy (SMT) to reduce the amount of and/or inhibit substrate accumulation in a patient diagnosed with a lysosomal storage disease.

The present invention further relates to the compound of Formula I, wherein n is 1; t is 0; y is 1 and z is 1.

The present invention further relates to the compound of Formula I, wherein n is 1; t is 1; y is 1 and z is 1.

The present invention further relates to the compound of Formula I, wherein n is 2; t is 0; y is 1 and z is 1.

The present invention further relates to the compound of Formula I, wherein n is 2; t is 1; y is 1 and z is 1.

The present invention further relates to the compound of Formula I, wherein n is 3; t is 0; y is 1 and z is 1.

The present invention further relates to the compound of Formula I, wherein n is 1; t is 2; y is 1 and z is 1.

The present invention further relates to the compound of Formula I, wherein n is 1; t is 0; y is 1 and z is 0.

The present invention further relates to the compound of Formula I, wherein n is 1; t is 1; y is 1 and z is 0.

The present invention further relates to the compound of Formula I, wherein n is 2; t is 0; y is 1 and z is 0.

The present invention further relates to the compound of Formula I, wherein n is 2; t is 1; y is 1 and z is 0.

The present invention further relates to the compound of Formula I, wherein n is 3; t is 0; y is 1 and z is 0.

The present invention further relates to the compound of Formula I, wherein n is 1; t is 2; y is 1 and z is 0.

The present invention further relates to the compound of Formula I, wherein n is 1; t is 1; y is 2 and z is 0.

The present invention further relates to the compound of Formula I, wherein n is 2; t is 0; y is 2 and z is 0.

The present invention further relates to the compound of Formula I, wherein m is 1 and X¹ is CR¹.

The present invention further relates to the compound of Formula I, wherein m is 0 and X¹ is N.

The present invention further relates to the compound of Formula I, wherein m is 1; E is O; X² is O and X³ is NH.

The present invention further relates to the compound of Formula I, wherein m is 1; E is O; X² is NH and X³ is NH.

The present invention further relates to the compound of Formula I, wherein m is 1; E is O; X² is CH² and X³ is NH.

The present invention further relates to the compound of Formula I, wherein m is 1; E is O; X² is NH and X³ is CH².

The present invention further relates to the compound of Formula I, wherein m is 1; E is S; X² is NH and X³ is NH.

The present invention further relates to the compound of Formula I, wherein m is 0; E is O; X¹ is NH and X³ is NH.

The present invention further relates to the compound of Formula I, wherein m is 1; E is O; X² is NH and X³ is CO-NH.

The present invention further relates to the compound of Formula I, wherein m is 1; p is 0; X² is NH-SO₂ and X³ is NH.

The present invention further relates to the compound of Formula I, wherein R⁴ and R⁵ are each (C₁-C₆)alkyl or taken together with the carbon to which they are attached to form a spiro (C₃-C₁₀)cyclo- alkyl ring or a spiro (C₃-C₁₀)cycloalkoxy ring.

The present invention further relates to the compound of Formula I, wherein R⁴ and R⁵ are each methyl.

The present invention further relates to the compound of Formula I, wherein R⁴ and R⁵ are taken together with the carbon to which they are attached to form a spiro (C₃-C₁₀)cycloalkyl ring.

The present invention further relates to the compound of Formula I, wherein R⁴ and R⁵ are taken together with the carbon to which they are attached to form a spiro cyclopropyl ring.

The present invention further relates to the compound of Formula I, wherein R⁴ and R⁵ are taken together with the carbon to which they are attached to form a spiro (C₃-C₁₀)cycloalkoxy ring.

The present invention further relates to the compound of Formula I, wherein A¹ is (C₂-C₆)alkynyl or (C₆-C₁₂)aryl.

The present invention further relates to the compound of Formula I, wherein A¹ is (C₂-C₉)heteroaryl.

The present invention further relates to the compound of Formula I, wherein A¹ is thiophene, thiazole, isothiazole, furane, oxazole, isoxazole, pyrrole, imidazole, pyrazole, triazole, pyridine, pymiridine, pyridazine, indole, benzotiazole, benzoisoxazole, benzopyrazole, benzoimidazole, benzofuran, benzooxazole or benzoisoxazole.

The present invention further relates to the compound of Formula I, wherein A¹ is (C₂-C₉)heterocycloalkyl.

The present invention further relates to the compound of Formula I, wherein A¹ is pyrrolidinyl, tetrahydrofuranyl, dihydrofuranyl, tetrahydropyranyl, pyranyl, thiopyranyl, aziridinyl, azetidinyl, oxiranyl, methylenedioxyl, chromenyl, barbituryl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, piperizin-2-onyl, piperizin-3-onyl, chromanyl, 2-pyrrolinyl, 3-pyrrolinyl, imidazolidinyl, 2-imidazolidinyl, 1,4-dioxanyl, 8-azabicyclo[3.2.1]octanyl, 3-azabicyclo[3.2.1]octanyl, 3,8-diazabicyclo[3.2.1]octanyl, 2,5-diazabicyclo[2.2.1]heptanyl, 2,5-diazabicyclo[2.2.2]octanyl, octahydro-2H-pyrido[1,2-a]pyrazinyl, 3-azabicyclo[4.1.0]heptanyl, 3-azabicyclo[3.1.0]hexanyl 2-azaspiro[4.4]nonanyl, 7-oxa-1-aza-spiro[4.4]nonanyl, 7-azabicyclo[2.2.2]heptanyl or octahydro-1H-indolyl.

The present invention further relates to the compound of Formula I, wherein A¹ is benzo(C₂-C₉)heterocycloalkyl.

The present invention further relates to the compound of Formula I, wherein A¹ is 2,3-dihydrobenzo[b][1,4] dioxine or 2,2-difluorobenzo[d][1,3]dioxole.

The present invention further relates to the compound of Formula I, wherein R⁶ is H.

The present invention further relates to the compound of Formula I, X⁵ is a direct bond.

The present invention further relates to the compound of Formula I, X⁵ is a CR⁴R⁵.

The present invention further relates to the compound of Formula I, wherein R⁴ and R⁵ are each methyl.

The present invention further relates to the compound of Formula I, wherein R⁴ and R⁵ are taken together with the carbon to which they are attached to form a spiro (C₃-C₁₀)cycloalkyl ring.

The present invention further relates to the compound of Formula I, wherein R⁴ and R⁵ are taken together with the carbon to which they are attached to form a spiro cyclopropyl ring.

The present invention further relates to the compound of Formula I, wherein R⁴ and R⁵ are taken together with the carbon to which they are attached to form a spiro (C₃-C₁₀)cycloalkoxy ring.

The present invention further relates to the compound of Formula I, wherein A² is (C₆-C₁₂)aryl.

The present invention further relates to the compound of Formula I, wherein A² is (C₂-C₉)heteroaryl.

The present invention further relates to the compound of Formula I, wherein A² is pyridine.

The present invention further relates to the compound of Formula I, wherein A² is (C₂-C₉)heterocycloalkyl.

The present invention further relates to the compound of Formula I, wherein A² is pyrrolidinyl, tetrahydrofuranyl, dihydrofuranyl, tetrahydropyranyl, pyranyl, thiopyranyl, aziridinyl, azetidinyl, oxiranyl, methylenedioxyl, chromenyl, barbituryl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, piperizin-2-onyl, piperizin-3-onyl, chromanyl, 2-pyrrolinyl, 3-pyrrolinyl, imidazolidinyl, 2-imidazolidinyl, 1,4-dioxanyl, 8-azabicyclo[3.2.1]octanyl, 3-azabicyclo[3.2.1]octanyl, 3,8-diazabicyclo[3.2.1]octanyl, 2,5-diazabicyclo[2.2.1]heptanyl, 2,5-diazabicyclo[2.2.2]octanyl, octahydro-2H-pyrido[1,2-a]pyrazinyl, 3-azabicyclo[4.1.0]heptanyl, 3-azabicyclo[3.1.0]hexanyl 2-azaspiro[4.4]nonanyl, 7-oxa-1-aza-spiro[4.4]nonanyl, 7-azabicyclo[2.2.2]heptanyl or octahydro-1H-indolyl.

The present invention further relates to the compound of Formula I, wherein A² is benzo(C₂-C₉)heterocycloalkyl.

The present invention further relates to the compound of Formula I, where R¹ is hydrogen or methyl.

The present further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heterocycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heterocycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₆-C₁₂)aryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₆-C₁₂)aryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; p is 1; E is O; X² is O; X³ is NH; R¹ is H; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CH₂; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is CH₂; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is S; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is SO₂; X² is NH; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is N; m is 0; E is O; X³ is NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₂-C₉)heteroaryl; X⁵ is a direct bond, O or CR⁴R⁵ and A² is (C₃-C₁₀)cycloalkyl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are taken together with the carbon to which they are attached to form a spiro (C₃-C₁₀)cycloalkyl ring or spiro (C₃-C₁₀)cycloalkoxy ring; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein n is 1; 2 or 3; t is 0, 1 or 2; y is 0 or 1; z is 0, 1 or 2; X¹ is CR¹; m is 1; E is O; X² is NH; X³ is CO-NH; R⁴ and R⁵ are each independently methyl; R⁶ is a hydrogen or methyl; A¹ is (C₃-C₁₀)cycloalkyl; X⁵ is a direct bond, O or CR⁴R⁵ and A² is (C₂-C₉)heteroaryl.

The present invention further relates to the compound of Formula I, wherein A¹ is (C₃-C₁₀)cycloalkyl.

The present invention further relates to the compound of Formula I, wherein A² is (C₃-C₁₀)cycloalkyl.

The present invention further relates to the compound of Formula I, or a pharmaceutically acceptable salt or prodrug thereof, selected from the group consisting of:
1-azabicyclo[2.2.2]oct-3-yl [2-(2,4'-difluorobiphenyl-4-yl)propan-2-yl]carbamate;
1-azabicyclo[2.2.2]oct-3-yl {2-[4-(1,3-benzothiazol-6-yl)phenyl]propan-2-yl}carbamate;
1-azabicyclo[3.2.2]non-4-yl {1-[5-(4-fluorophenyl)pyridin-2-yl]cyclopropyl}carbamate;
1-azabicyclo[2.2.2]oct-3-yl {1-[3-(4-fluorophenoxy)phenyl]cyclopropyl}carbamate;
1-azabicyclo[2.2.2]oct-3-yl {1-[4-(1,3-benzothiazol-5-yl)phenyl]cyclopropyl}carbamate;
1-azabicyclo[2.2.2]oct-3-yl [1-(4'-fluoro-3'-methoxybiphenyl-4yl)cyclopropyl]carbamate;
1-azabicyclo[2.2.2]oct-3-yl [3-(4'-fluorobiphenyl-4-yl)oxetan-3-yl]carbamate;
1-azabicyclo[2.2.2]oct-3-yl {1-[6-(4-fluorophenoxy)pyridin-2-yl]cyclopropyl}carbamate;
1-azabicyclo[2.2.2]oct-3-yl [3-(4'-fluorobiphenyl-4-yl)pentan-3-yl]carbamate;
1-azabicyclo[2.2.2]oct-3-yl {2-[2-(4-fluorophenyl)-2H-indazol-6-yl]propan-2 yl}carbamate;
1-azabicyclo[2.2.2]oct-3-yl {2-[2-(1H-pyrrol-1-yl)pyridin-4-yl]propan-2-yl}carbamate;
1-(3-ethyl-1-azabicyclo[2.2.2]oct-3-yl)-3-[1-(4'-fluorobiphenyl-4-yl)cyclopropyl]urea;
N-(1-azabicyclo[2.2.2]oct-3-yl)-N'-[1-(4'-fluorobiphenyl-4yl)cyclopropyl] ethanediamide;
1-azabicyclo[2.2.2]oct-3-yl (1-{4[(4,4difluorocyclohexyl)oxy]phenyl}cyclopropyl) carbamate;
1-(4-methyl-1-azabicyclo[3.2.2]non-4-yl)-3-[1-(5-phenylpyridin-2-yl)cyclopropyl]urea; 1-[1-(4'-fluorobiphenyl-4-yl)cyclopropyl]-1-methyl-3-(3-methyl-1-azabicyclo[2.2.2]oct-3-yl)urea;
1-[1-(4'-fluorobiphenyl-4-yl)cyclopropyl]-1-methyl-3-(3-methyl-1-azabicyclo[2.2.2]oct-3-yl)urea;
1-{2-[4'-(2-methoxyethoxy)biphenyl-4-yl]propan-2-yl}-3-(3-methyl-1-azabicyclo[2.2.2]oct-3-yl)urea;
2-(1-azabicyclo[3.2.2]non-4-yl)-N-[1-(5-phenylpyridin-2-yl)cyclopropyl]acetamide; 3-(4'-fluorobiphenyl-4-yl)-3-methyl-N-(4-methyl-1-azabicyclo[3.2.2]non-4-yl)butanamide;
N-[2-(biphenyl-4-yl)propan-2-yl]-N'-(3-methyl-1-azabicyclo[2.2.2]oct-3-yl)sulfuric diamide;
N-[2-(4'-fluorobiphenyl-4-yl)propan-2-yl]-N'-(3-methyl-1-azabicyclo[2.2.2]oct-3-yl)sulfuric diamide;
1-(3-butyl-1-azabicyclo[2.2.2]oct-3-yl)-3-{2-[1-(4-fluorophenyl)-1H-pyrazol-4-yl]propan-2-yl}urea;
1-azabicyclo[2.2.2]oct-3-yl [4-(4-fluorophenyl)-2-methylbut-3-yn-2-yl]carbamate; 1-(3-butyl-1-azabicyclo[2.2.2]oct-3-yl)-3-[4-(4-fluorophenyl)-2-methylbut-3-yn-2-yl]urea;
N-[1-(4'-fluorobiphenyl-4-yl)cyclopropyl]-1,4-diazabicyclo[3.2.2]nonane-4-carboxamide;
1-(2-(4'-fluoro-[1,1'-biphenyl]-4-yl)propan-2-yl)-3-(3-methyl-1-azabicyclo[3.2.2]nonan-3-yl)urea;
1-(2-(4'-fluoro-[1,1'-biphenyl]-4-yl)propan-2-yl)-3-(4-methyl-1-azabicyclo[4.2.2]decan-4-yl)urea;
1-(2-(4'-fluoro-[1,1'-biphenyl]-4-yl)propan-2-yl)-3-(3-methyl-1-azabicyclo[4.2.2]decan-3-yl)urea; and
1-(2-(4'-fluoro-[1,1'-biphenyl]-4-yl)propan-2-yl)-3-(5-methyl-1-azabicyclo[4.2.2]decan-5-yl)urea.

The present invention further relates to a pharmaceutical composition for treating a disease or disorder mediated by glucosylceramide synthase (GCS) or a disease or disorder in which GCS is implicated in a subject in need of such treatment comprising administering to the subject an effective amount of the compound of Formula I.

The present invention further relates to a method for treating a disease or disorder mediated by glucosylceramide synthase (GCS) or a disease or disorder in which GCS is implicated in a subject in need of such treatment comprising administering to the subject an effective amount of the compound of Formula I.

The present invention further relates to a method for treating a disease or disorder such as cancer.

The present invention further relates to a method for treating a disease or disorder such as a metabolic disorder.

The present invention further relates to a method for treating a disease or disorder such as a neuropathic disease.

The present invention further relates to a method wherein the neuropathic disease is Alzheimer's disease.

The present invention further relates to a method wherein the neuropathic disease is Parkinson's disease.

The present invention further relates to the method for inducing decreased glucosylceramide synthase catalytic activity in a cell, in vitro, comprising contacting the cell with an effect amount of the compound of Formula I.

The present invention further relates to the compound of Formula I, (S)-quinuclidin-3-yl (2-(2-(4-fluorophenyl)thiazol-4-yl)propan-2-yl)carbamate, represented by the following structural formula, 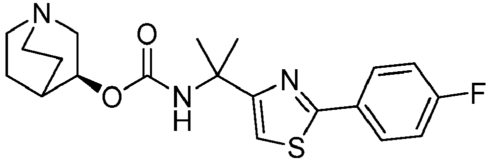 or a pharmaceutically acceptable salt or prodrug thereof.

The present invention further relates to the compound of Formula I, Quinuclidin-3-yl (2-(4'-fluoro-[1,1'-biphenyl]-3-yl)propan-2-yl)carbamate, represented by the following structural formula, 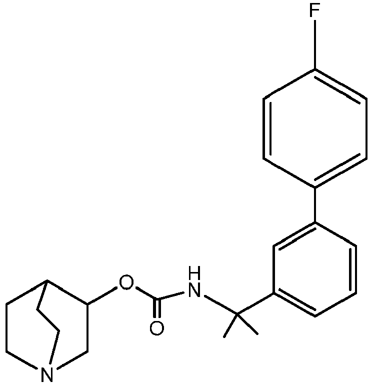 or a pharmaceutically acceptable salt or prodrug thereof.

The present invention further relates to a method of treating a subject diagnosed as having a lysosomal storage disease, the method including administering to the subject an effective amount of the compound of Formula I, and in certain embodiments the compound is represented by following structural formulas, 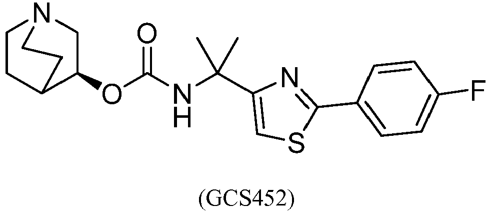 or a pharmaceutically acceptable salt or prodrug thereof, or 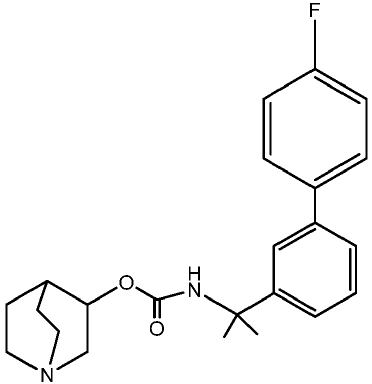 or a pharmaceutically acceptable salt or prodrug thereof.

In certain embodiments of the invention, the lysosomal storage disease results from a defect in the glycosphingolipid pathway.

In certain embodiments of the invention, the lysosomal storage disease is Gaucher, Fabry, G_{M1}-gangliosidosis, G_{M2} Activator deficiency, Tay-Sachs or Sandhoff.

The present invention further relates to a method of treating a subject diagnosed as having a lysosomal storage disease, the method including administering to the subject an effective amount of the compound of Formula I and administering to the subject a therapeutically effective amount of a lysosomal enzyme.

In certain embodiments of the invention, the lysosomal enzyme is glucocerebrosidase, alpha-galactosidase A, Hexosaminidase A, Hexosaminidase B or G_{M1}-ganglioside-β-galactosidase.

In certain embodiments of the invention, the subject has elevated levels of a lysosomal substrate prior to treatment and once undergoing treatment the subject has lower combined amounts of the lysosomal substrate in the urine and plasma than a subject treated with either the lysosomal enzyme or compound alone.

In certain embodiments of the invention, the substrate is globotriaosylceramide or lyso-globotriaosylceramide, and combinations thereof.

The present invention further relates to a method of reducing glucosylceramide synthase (GCS) activity in a subject diagnosed as having a lysosomal storage disease, including administering to the patient an effective amount of the compound of Formula I, either alone or as a combination therapy with an enzyme replacement therapy.

The present invention further relates to a method of reducing accumulation of a GCS-derived material in a subject diagnosed as having a lysosomal storage disease, including administering to the patient an effective amount of the compound of Formula I, either alone or as a combination therapy with an enzyme replacement therapy.

The present invention further relates to a method for treating globotriaosylceramide (GL-3) accumulation in the heart of a subject having or at risk of having Fabry disease, wherein the method comprises administering to the subject an effective amount of a compound represented by the following structural formula, 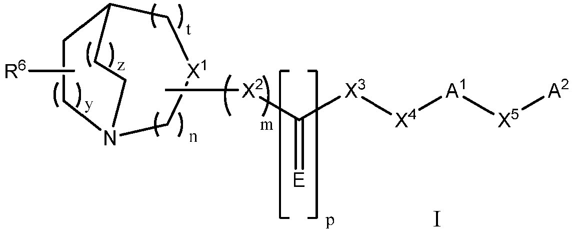 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR²;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂-(C₁-C₆)alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound inhibits GL-3 accumulation in a cardiomyocyte of the subject.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound inhibits GL-3 accumulation in the lysosome of the cardiomyocyte.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound reduces GL-3 accumulation in a cardiomyocyte of the subject.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound reduces GL-3 accumulation in the lysosome of the cardiomyocyte.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound prevents GL-3 accumulation in a cardiomyocyte of the subject.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound prevents GL-3 accumulation in the lysosome of the cardiomyocyte.

The present invention further relates to a method for treating globotriaosylceramide (GL-3) accumulation in the heart of a subject having or at risk of having Fabry disease, wherein the method comprises administering to the subject an effective amount of a compound represented by Formula I, wherein administration of the compound inhibits glucosylceramide synthase (GCS) activity.

The present invention further relates to a method for reducing globotriaosylceramide (GL-3) in the heart of a subject having or at risk of having Fabry disease, wherein the method comprises administering to the subject an effective amount of a compound represented by the following structural formula, 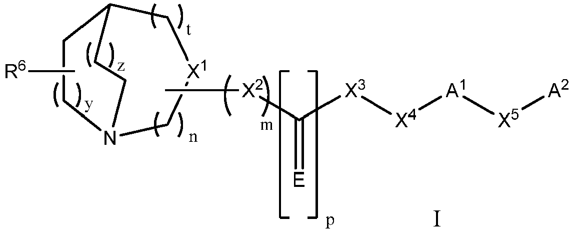 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR²;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂-(C₁-C₆)alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

The present invention further relates to a method for reducing globotriaosylceramide (GL-3) in the heart of a subject having or at risk of having Fabry disease, wherein the method comprises administering to the subject an effective amount of a compound represented by Formula I, wherein the cardiomyocytes of the subject has increased GL-3 as compared to healthy cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein the increased GL-3 is present in the lysosomes of the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein administration of the compound maintains GL-3 levels in the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein administration of the compound maintains GL-3 levels in the lysosome of the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein administration of the compound reduces GL-3 in the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein administration of the compound reduces GL-3 in the lysosome of the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein the cardiomyocytes of the subject comprise accumulated GL-3.

The present invention further relates to a method for reducing globotriaosylceramide (GL-3) in the heart of a subject having or at risk of having Fabry disease, wherein the method comprises administering to the subject an effective amount of a compound represented by Formula I, wherein the accumulated GL-3 is present in the lysosomes of the cardiomyocytes.

The present invention further relates to a method for reducing globotriaosylceramide (GL-3) in the heart of a subject having or at risk of having Fabry disease, wherein the method comprises administering to the subject an effective amount of a compound represented by Formula I, wherein administration of the compound inhibits glucosylceramide synthase (GCS) activity.

The present invention further relates to a method for reducing globotriaosylceramide (GL-3) in the heart of a subject having or at risk of having Fabry disease, wherein the method comprises administering to the subject an effective amount of a compound, wherein the compound is 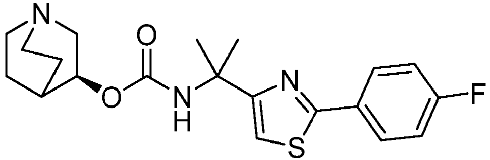 or a pharmaceutically acceptable salt or prodrug thereof.

The present invention further relates to a method for treating globotriaosylceramide (GL-3) accumulation in the heart of a subject having or at risk of having Fabry disease, wherein the method comprises administering to the subject an effective amount of a compound represented by the following structural formula:

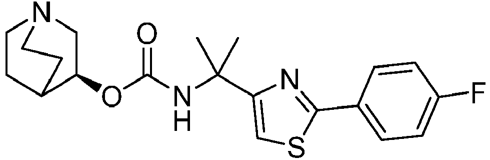

The present invention further relates to a method for reducing globotriaosylceramide (GL-3) in the heart of a subject having or at risk of having Fabry disease, wherein the method comprises administering to the subject an effective amount of a compound, wherein the compound is , wherein the compound is 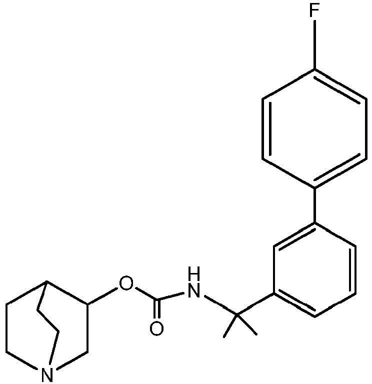 or a pharmaceutically acceptable salt or prodrug thereof.

The present invention further relates to a method for treating globotriaosylceramide (GL-3) accumulation in the heart of a subject having or at risk of having Fabry disease, wherein the method comprises administering to the subject an effective amount of a compound represented by the following structural formula:

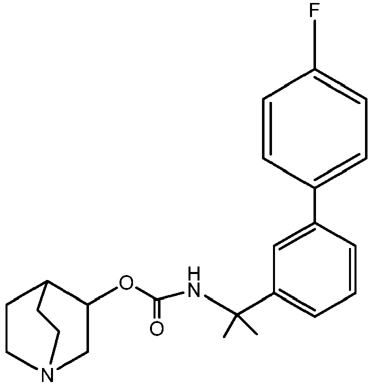

The present invention further relates to a method for reducing globotriaosylceramide (GL-3) in the heart of a subject having or at risk of having Fabry disease, wherein the method comprises administering to the subject an effective amount of a compound of Formula I, wherein the method further comprises administering a therapeutically effective amount of alpha-galactosidase A to the subject.

The present invention further relates to a method for treating globotriaosylceramide (GL-3) accumulation in the heart of a subject having or at risk of having Fabry disease, wherein the method comprises administering to the subject an effective amount of a compound represented by the following structural formula: 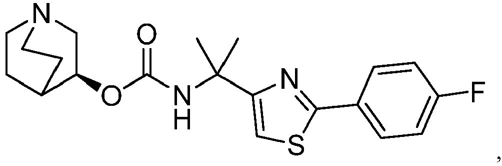 wherein the method further comprises administering a therapeutically effective amount of alpha-galactosidase A to the subject.

The present invention further relates to a method for treating globotriaosylceramide (GL-3) accumulation in the heart of a subject having or at risk of having Fabry disease for reducing globotriaosylceramide (GL-3) in the heart of a subject having or at risk of having Fabry disease, wherein the method comprises administering to the subject an effective amount of a compound of Formula I and wherein prior to treatment the subject has elevated levels of globotriaosylceramide, lyso-globotriaosylceramide, or combinations thereof.

The present invention further relates to a method for treating globotriaosylceramide (GL-3) accumulation in the heart of a subject having or at risk of having Fabry disease for reducing globotriaosylceramide (GL-3) in the heart of a subject having or at risk of having Fabry disease, wherein the method comprises administering to the subject an effective amount of a compound of the following structural formula: 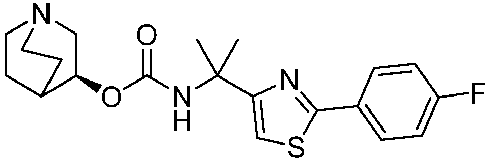 and wherein prior to treatment the subject has elevated levels of globotriaosylceramide, lyso-globotriaosylceramide, or combinations thereof.

The present invention further relates to a method for treating globotriaosylceramide (GL-3) accumulation in the heart of a subject having or at risk of having Fabry disease for reducing globotriaosylceramide (GL-3) in the heart of a subject having or at risk of having Fabry disease, wherein the method comprises administering to the subject an effective amount of a compound of Formula I, wherein the subject undergoing treatment has lower combined amounts of globotriaosylceramide or lyso-globotriaosylceramide in the urine and plasma than a subject treated with either the alpha-galactosidase A or the compound alone.

The present invention further relates to a method for treating globotriaosylceramide (GL-3) accumulation in the heart of a subject having or at risk of having Fabry disease for reducing globotriaosylceramide (GL-3) in the heart of a subject having or at risk of having Fabry disease, wherein the method comprises administering to the subject an effective amount of a compound of the following structrural formula: 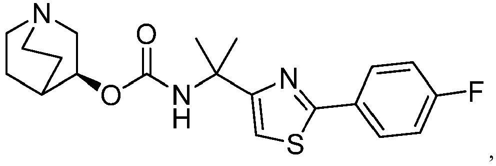 wherein the subject undergoing treatment has lower combined amounts of globotriaosylceramide or lyso-globotriaosylceramide in the urine and plasma than a subject treated with either the alpha-galactosidase A or the compound alone.

The present invention further relates to a method for treating a glycosphingolipid disease or disorder in a cardiomyocyte, wherein the method comprises contacting the cardiomyocyte with an effective amount of a compound represented by the following structural formula, 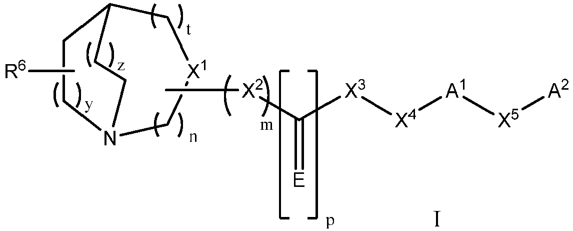 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR² ;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂-(C₁-C₆) alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₆)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

The present invention further relates to a method for treating a glycosphingolipid disease or disorder in a cardiomyocyte, wherein the method comprises contacting the cardiomyocyte with an effective amount of a compound of Formula I, wherein the glycosphingolipid disease or disorder comprises increased globotriaosylceramide (GL-3) in the cardiomyocyte as compared to a healthy cardiomyocyte.

The present invention further relates to a method for treating a glycosphingolipid disease or disorder in a cardiomyocyte, wherein the method comprises contacting the cardiomyocyte with an effective amount of a compound of Formula I, wherein increased GL-3 is present in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound maintains GL-3 levels in the cardiomyocyte.

The present invention further relates to a method for treating a glycosphingolipid disease or disorder in a cardiomyocyte, wherein the method comprises contacting the cardiomyocyte with an effective amount of a compound of Formula I, wherein the compound maintains GL-3 levels in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound reduces GL-3 in the cardiomyocyte.

The present invention further relates to a method for treating a glycosphingolipid disease or disorder in a cardiomyocyte, wherein the method comprises contacting the cardiomyocyte with an effective amount of a compound of Formula I wherein the compound reduces GL-3 in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein the glycosphingolipid disease or disorder comprises globotriaosylceramide (GL-3) accumulation in the cardiomyocyte.

The present invention further relates to a method for treating a glycosphingolipid disease or disorder in a cardiomyocyte, wherein the method comprises contacting the cardiomyocyte with an effective amount of a compound of Formula I, wherein GL-3 accumulation is present in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound inhibits GL-3 accumulation in the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound inhibits GL-3 accumulation in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound reduces GL-3 accumulation in the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound reduces GL-3 accumulation in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound prevents GL-3 accumulation in the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound prevents GL-3 accumulation in the lysosome of the cardiomyocyte.

The present invention further relates to a method for treating a glycosphingolipid disease or disorder in a cardiomyocyte, wherein the method comprises contacting the cardiomyocyte with an effective amount of a compound of Formula I, wherein administration of the compound inhibits glucosylceramide synthase (GCS) activity.

The present invention further relates to a method for treating a glycosphingolipid disease or disorder in a cardiomyocyte, wherein the method comprises contacting the cardiomyocyte with an effective amount of a compound of Formula I, wherein the compound is 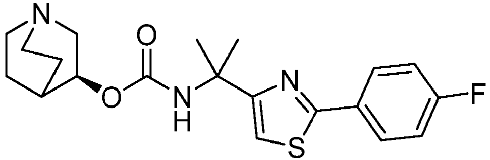 or a pharmaceutically acceptable salt or prodrug thereof.

The present invention further relates to a method for treating a glycosphingolipid disease or disorder in a cardiomyocyte, wherein the method comprises contacting the cardiomyocyte with an effective amount of a compound of Formula I, wherein the compound is 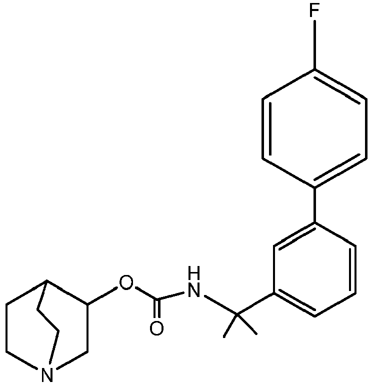 or a pharmaceutically acceptable salt or prodrug thereof.

The present invention further relates to a method for treating a glycosphingolipid disease or disorder in a cardiomyocyte, wherein the method comprises contacting the cardiomyocyte with an effective amount of a compound of Formula I, wherein the cardiomyocyte is in the heart of a subject.

The present invention further relates to a method for treating a glycosphingolipid disease or disorder in a cardiomyocyte, wherein the method comprises contacting the cardiomyocyte with an effective amount of a compound of Formula I, wherein the subject has or is at risk of having the glycosphingolipid disease or disorder.

The present invention further relates to a method for treating a glycosphingolipid disease or disorder in a cardiomyocyte, wherein the method comprises contacting the cardiomyocyte with an effective amount of a compound of Formula I, wherein the glycosphingolipid disease or disorder is a metabolic disorder.

The present invention further relates to a method for treating a glycosphingolipid disease or disorder in a cardiomyocyte, wherein the method comprises contacting the cardiomyocyte with an effective amount of a compound of Formula I, wherein the glycosphingolipid disease or disorder is a lysosomal storage disease.

The present invention further relates to a method for treating a glycosphingolipid disease or disorder in a cardiomyocyte, wherein the method comprises contacting the cardiomyocyte with an effective amount of a compound of Formula I, wherein the lysosomal storage disease is selected from the group consisting of Gaucher, Fabry, G_{M1}-gangliosidosis, G_{M2} Activator Deficiency, Tay-Sachs and Sandhoff.

The present invention further relates to a method for treating a glycosphingolipid disease or disorder in a cardiomyocyte, wherein the method comprises contacting the cardiomyocyte with an effective amount of a compound of Formula I, wherein the glycosphingolipid disease or disorder is a GL-3 disease or disorder.

The present invention further relates to a method for treating a glycosphingolipid disease or disorder in a cardiomyocyte, wherein the method comprises contacting the cardiomyocyte with an effective amount of a compound of Formula I, wherein the lysosomal storage disease is Fabry.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject and wherein the lysosomal enzyme is selected from the group consisting of glucocerebrosidase, alpha-galactosidase A, Hexosaminidase A, Hexosaminidase B and G_{M1}-ganglioside-β-galactosidase.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject, wherein the lysosomal enzyme is alpha-galactosidase A.

The present invention further relates to a method for treating a glycosphingolipid disease or disorder in a cardiomyocyte, wherein the method comprises contacting the cardiomyocyte with an effective amount of a compound of Formula I, wherein the method further comprises administering a therapeutically effective amount of a lysosomal enzyme to the subject, wherein prior to treatment the subject has elevated levels of a lysosomal substrate.

The present invention further relates to a method for treating a glycosphingolipid disease or disorder in a cardiomyocyte, wherein the method comprises contacting the cardiomyocyte with an effective amount of a compound of Formula I, wherein the method further comprises administering a therapeutically effective amount of a lysosomal enzyme to the subject, wherein prior to treatment the subject has elevated levels of a lysosomal substrate.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject, wherein prior to treatment the subject has elevated levels of a lysosomal substrate.

The present invention further relates to a method for treating a glycosphingolipid disease or disorder in a cardiomyocyte, wherein the method comprises contacting the cardiomyocyte with an effective amount of a compound of Formula I, wherein the method further comprises administering a therapeutically effective amount of a lysosomal enzyme to the subject, wherein prior to treatment the subject has elevated levels of a lysosomal substrate and wherein the lysosomal substrate is selected from the group consisting of globotriaosylceramide and lyso-globotriaosylceramide, and combinations thereof.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject, wherein prior to treatment the subject has elevated levels of a lysosomal substrate and wherein the lysosomal substrate is selected from the group consisting of globotriaosylceramide and lyso-globotriaosylceramide, and combinations thereof.

The present invention further relates to a method for treating a glycosphingolipid disease or disorder in a cardiomyocyte, wherein the method comprises contacting the cardiomyocyte with an effective amount of a compound of Formula I, wherein the method further comprises administering a therapeutically effective amount of a lysosomal enzyme to the subject, wherein prior to treatment the subject has elevated levels of a lysosomal substrate and wherein the subject undergoing treatment has lower combined amounts of the lysosomal substrate in the urine and plasma than a subject treated with either the lysosomal enzyme or the compound alone.

The present invention further relates to a method for treating a glycosphingolipid disease or disorder in a cardiomyocyte, wherein the method comprises contacting the cardiomyocyte with an effective amount of a compound GCS452, wherein the method further comprises administering a therapeutically effective amount of a lysosomal enzyme to the subject, wherein prior to treatment the subject has elevated levels of a lysosomal substrate and wherein the subject undergoing treatment has lower combined amounts of the lysosomal substrate in the urine and plasma than a subject treated with either the lysosomal enzyme or the compound alone.

This invention relates to a method of combination therapy for treatment of a subject diagnosed as having a lysosomal storage disease comprising alternating between administration of an enzyme replacement therapy and an SRT or small molecule therapy.

This invention relates to a method of combination therapy for treatment of a subject diagnosed as having a lysosomal storage disease comprising simultaneously administering an enzyme replacement therapy and an SRT or small molecule therapy.

In the various combination therapies of the disclosure, it will be understood that administering small molecule therapy or SRT may occur prior to, concurrently with, or after, administration of enzyme replacement therapy. Similarly, administering enzyme replacement therapy may occur prior to, concurrently with, or after, administration of small molecule therapy.

This invention further provides a compound for use in a method for treating GL-3 accumulation in a cardiomyocyte as defined in appended Claim 1, wherein the method comprises contacting the cardiomyocyte with an effective amount of a compound represented by the following structural formula, 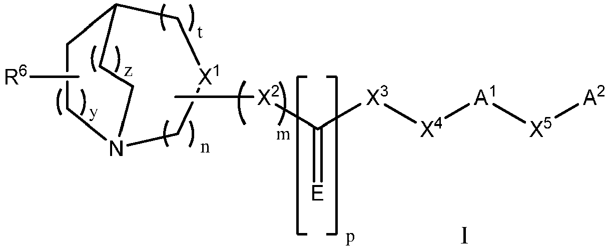 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂; CH(C₁-C₆) alkyl or -NR²;
X³ is O, -NH, -CH₂-, CO, - CH(C₁-C₆)alkyl, SO₂NH, -CO-NH- or -NR³;
X⁴ is CR⁴R⁵, CH₂ CR⁴R⁵ or CH₂-(C₁-C₆)alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

This invention further provides a compound for use in a method for treating GL-3 accumulation in a cardiomyocyte as defined in appended Claim 1, wherein the method comprises contacting the cardiomyocyte with an effective amount of a compound represented by the following structural formula: 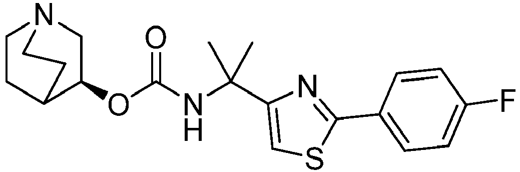 or a pharmaceutically acceptable salt or prodrug thereof.

### Definitions

As used herein, the term "pharmaceutically acceptable salt" means either a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.

As used herein, the term "prodrug" means a pharmacological derivative of a parent drug molecule that requires biotransformation, either spontaneous or enzymatic, within the organism to release the active drug. For example, prodrugs are variations or derivatives of the compounds of Formula I that have groups cleavable under certain metabolic conditions, which when cleaved, become the compounds of Formula I. Such prodrugs then are pharmaceutically active in vivo, when they undergo solvolysis under physiological conditions or undergo enzymatic degradation. Prodrug compounds herein may be called single, double, triple, etc., depending on the number of biotransformation steps required to release the active drug within the organism, and the number of functionalities present in a precursor-type form. Prodrug forms often offer advantages of solubility, tissue compatibility, or delayed release in the mammalian organism (See, Bundgard, Design of Prodrugs, pp. 7-9, 21-24, Elsevier, Amsterdam 1985 and Silverman, The Organic Chemistry of Drug Design and Drug Action, pp. 352-401, Academic Press, San Diego, Calif., 1992). Prodrugs commonly known in the art include well-known acid derivatives, such as, for example, esters prepared by reaction of the parent acids with a suitable alcohol, amides prepared by reaction of the parent acid compound with an amine, basic groups reacted to form an acylated base derivative, etc. Of course, other prodrug derivatives may be combined with other features disclosed herein to enhance bioavailability. As such, those of skill in the art will appreciate that certain of the presently disclosed compounds having free amino, amido, hydroxy or carboxylic groups can be converted into prodrugs. Prodrugs include compounds having an amino acid residue, or a polypeptide chain of two or more (e.g., two, three or four) amino acid residues which are covalently joined through peptide bonds to free amino, hydroxy or carboxylic acid groups of the presently disclosed compounds. The amino acid residues include the 20 naturally occurring amino acids commonly designated by three letter symbols and also include 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone. Prodrugs also include compounds having a carbonate, carbamate, amide or alkyl ester moiety covalently bonded to any of the above substituents disclosed herein.

As used herein, the term "(C₁-C₆)alkyl" means a saturated linear or branched free radical consisting essentially of 1 to 6 carbon atoms and a corresponding number of hydrogen atoms. Exemplary (C₁-C₆)alkyl groups include methyl, ethyl, n-propyl, isopropyl, n-butyl, isobutyl, etc. Of course, other (C₁-C₆)alkyl groups will be readily apparent to those of skill in the art given the benefit of the present disclosure.

As used herein, the term "(C₃-C₁₀)cycloalkyl" means a nonaromatic saturated free radical forming at least one ring consisting essentially of 3 to 10 carbon atoms and a corresponding number of hydrogen atoms. As such, (C₃-C₁₀)cycloalkyl groups can be monocyclic or multicyclic. Individual rings of such multicyclic cycloalkyl groups can have different connectivities, e.g., fused, bridged, spiro, etc. in addition to covalent bond substitution. Exemplary (C₃-C₁₀)cycloalkyl groups include cyclopropyl, cyclobutyl, cyclopentyl, cyclohexyl, norbornanyl, bicyclo[3.2.1]octanyl, octahydro-pentalenyl, spiro[4.5]decanyl, cyclopropyl substituted with cyclobutyl, cyclobutyl substituted with cyclopentyl, cyclohexyl substituted with cyclopropyl, etc. Of course, other (C₃-C₁₀)cycloalkyl groups will be readily apparent to those of skill in the art given the benefit of the present disclosure.

As used herein, the term "(C₂-C₉)heterocycloalkyl" means a nonaromatic free radical having 3 to 10 atoms (i.e., ring atoms) that form at least one ring, wherein 2 to 9 of the ring atoms are carbon and the remaining ring atom(s) (i.e., hetero ring atom(s)) is selected from the group consisting of nitrogen, sulfur, and oxygen. As such, (C₂-C₉)heterocycloalkyl groups can be monocyclic or multicyclic. Individual rings of such multicyclic heterocycloalkyl groups can have different connectivities, e.g., fused, bridged, spiro, etc. in addition to covalent bond substitution. Exemplary (C₂-C₉)heterocycloalkyl groups include pyrrolidinyl, tetrahydrofuranyl, dihydrofuranyl, tetrahydropyranyl, pyranyl, thiopyranyl, aziridinyl, azetidinyl, oxiranyl, methylenedioxyl, chromenyl, barbituryl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, piperizin-2-onyl, piperizin-3-onyl, chromanyl, 2-pyrrolinyl, 3-pyrrolinyl, imidazolidinyl, 2-imidazolidinyl, 1,4-dioxanyl, 8-azabicyclo[3.2.1]octanyl, 3-azabicyclo[3.2.1]octanyl, 3,8-diazabicyclo[3.2.1]octanyl, 2,5-diazabicyclo[2.2.1]heptanyl, 2,5-diazabicyclo[2.2.2]octanyl, octahydro-2H-pyrido[1,2-a]pyrazinyl, 3-azabicyclo[4.1.0]heptanyl, 3-azabicyclo[3.1.0]hexanyl 2-azaspiro[4.4]nonanyl, 7-oxa-1-aza-spiro[4.4]nonanyl, 7-azabicyclo[2.2.2]heptanyl, octahydro-1H-indolyl, etc. In general, the (C₂-C₉)heterocycloalkyl group typically is attached to the main structure via a carbon atom or a nitrogen atom. Of course, other (C₂-C₉)heterocycloalkyl groups will be readily apparent to those of skill in the art given the benefit of the present disclosure.

As used herein, the term "(C₂-C₉)heteroaryl" means an aromatic free radical having 5 to 10 atoms (i.e., ring atoms) that form at least one ring, wherein 2 to 9 of the ring atoms are carbon and the remaining ring atom(s) (i.e., hetero ring atom(s)) is selected from the group consisting of nitrogen, sulfur, and oxygen. As such, (C₂-C₉)heteroaryl groups can be monocyclic or multicyclic. Individual rings of such multicyclic heteroaryl groups can have different connectivities, e.g., fused, etc. in addition to covalent bond substitution. Exemplary (C₂-C₉)heteroaryl groups include furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyrindinyl, benzo[b]thiophenyl, 5,6,7,8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl and benzoxazinyl, etc. In general, the (C₂-C₉)heteroaryl group typically is attached to the main structure via a carbon atom, however, those of skill in the art will realize when certain other atoms, e.g., hetero ring atoms, can be attached to the main structure. Of course, other (C₂-C₉)heteroaryl groups will be readily apparent to those of skill in the art given the benefit of the present disclosure.

As used herein, the term "(C₆-C₁₂)aryl" means a functional group or substituent having 6 to 12 carbon atoms that form at least one ring.

As used herein, the term "(C₆-C₁₀)aryl" means means a functional group or substituent having 6 to 12 carbon atoms that form at least one ring, including phenyl or naphthyl.

As used herein, the term "halo" means fluorine, chlorine, bromine, or iodine.

As used herein, the term "amino" means a free radical having a nitrogen atom and 1 to 2 hydrogen atoms. As such, the term amino generally refers to primary and secondary amines. In that regard, as used herein and in the appended claims, a tertiary amine is represented by the general formula RR'N-, wherein R and R' are carbon radicals that may or may not be identical. Nevertheless, the term "amino" generally may be used herein to describe a primary, secondary, or tertiary amine, and those of skill in the art will readily be able to ascertain the identification of which in view of the context in which this term is used in the present disclosure.

As used herein, the term "combination therapy" means treating a patient with two or more therapeutic platforms (e.g., enzyme replacement therapy and small molecule therapy) in rotating, alternating and/or simultaneous treatment schedules. Examples of treatment schedules may include, but are not limited to: (1) enzyme replacement therapy, then small molecule therapy; (2) small molecule therapy, then enzyme replacement therapy; (3) enzyme replacement therapy concurrent with small molecule therapy, and (4) and any combination of the foregoing. Combination therapy may provide a temporal overlap of therapeutic platforms, as needed, depending on the clinical course of a given storage disease in a given subject.

As used herein, the term "enzyme replacement therapy", or "ERT" means administering an exogenously-produced natural or recombinant enzyme to a patient who is in need thereof. In the case of a lyosomal storage disease, for example, the patient accumulates harmful levels of a substrate (i.e., material stored) in lysosomes due to a deficiency or defect in an enzyme responsible for metabolizing the substrate, or due to a deficiency in an enzymatic activator required for proper enzymatic function. Enzyme replacement therapy is provided to the patient to reduce the levels of (i.e., debulk) accumulated substrate in affected tissues. Table 1 provides a list of lysosomal storage diseases and identifies the corresponding enzyme deficiency and accumulated substrate for each disease. Enzyme replacement therapies for treating lysosomal storage diseases are known in the art. In accordance with a combination therapy of the invention, the lysosomal enzymes identified in Table 1 can be used for enzyme replacement therapy to reduce the levels of corresponding substrate in a patient diagnosed with the respective lysosomal storage disease.

As used herein, "effective amount" of an enzyme or small molecule, when delivered to a subject in a combination therapy of the invention, is an amount sufficient to improve the clinical course of a lysosomal storage disease, where clinical improvement is measured by any of the variety of defined parameters well known to the skilled artisan.

### Chemical Abbreviations

ACN refers to acetonitrile.
DMF refers to N,N-dimethylformamide.
DMSO refers to dimethylsulfoxide.
EtOAc refers to ethyl acetate.
EtOH refers to ethanol.
Hunig's Base refers to diisopropylethyl amine ("DIPEA").
MeOH refers to methanol.
NaOH refers to sodium hydroxide.
THF refers to tetrahydrofuran.
TFA refers to trifluoroacetic acid.

Additional features and advantages of compounds disclosed herein will be apparent from the following detailed description of certain embodiments.

### BRIEF DESCRIPTION OF THE DRAWINGS

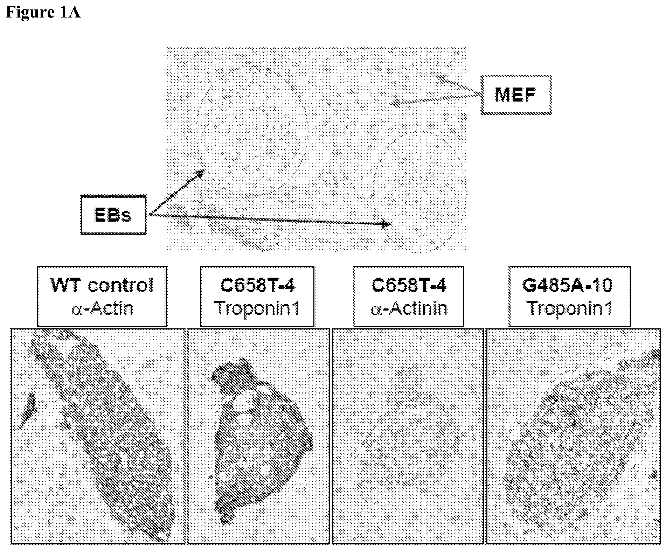

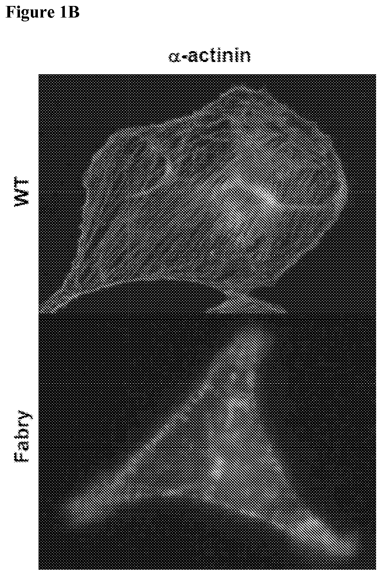

**Figure 1A** **and** **1B****.** Expression of cardiac contractile proteins in one month old wild type (WT) and Fabry Disease (FD) beating embryoid bodies (EBs) and dissociated cells. (A) Immunohistochemistry of beating EBs. Mouse fibroblasts (large cells, green arrows) were added to the EBs (small and compacted cells, brown arrows) to facilitate handling of the samples. Antibodies directed against actin, troponin I and a-actinin were used to identify the cardiomyocytes contained in the EBs. In WT EBs and in Fabry EBs from the three FD induced pluripotent stem cells (iPSC) lines, more than 90% of the cells express high level of cardiac contractile proteins. (B) Contractile fibers, revealed by an anti-a-actinin, are less numerous, more disorganized and localized at the periphery in FD iPSC-derived cell.

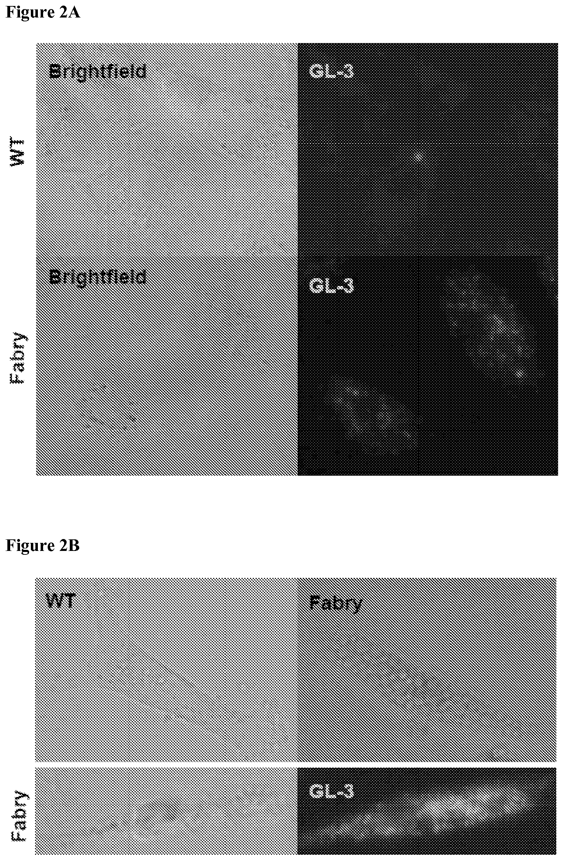

**Figure 2A-2D****.** Lysosomal globotriaosylceramide (GL-3) accumulation in one month old Fabry iPSC-derived cardiomyocytes. (A) GL-3 accumulation in dissociated cells from beating Fabry EBs. Anti-GL-3 reveal spots of the glycosphingolipid scattered in the cell cytoplasm. (B) Frequent observable vacuoles at the bright-field microscopic examination and more elevated level of GL-3 in Fabry iPSC-derived cells. (C) GL-3 accumulation in Fabry iPSC-derived cardiomyocytes. Expression of α-actinin and GL-3 in the same cell. (D) GL-3 is accumulated in lysosomes. Co-localization of GL-3 and Lamp2, a marker of lysosomes membranes (see merged picture and compare yellow arrows at the maximun magnification). The GL-3 and the Lamp2 signals are higher close to the nuclei and decrease at the periphery of the cell

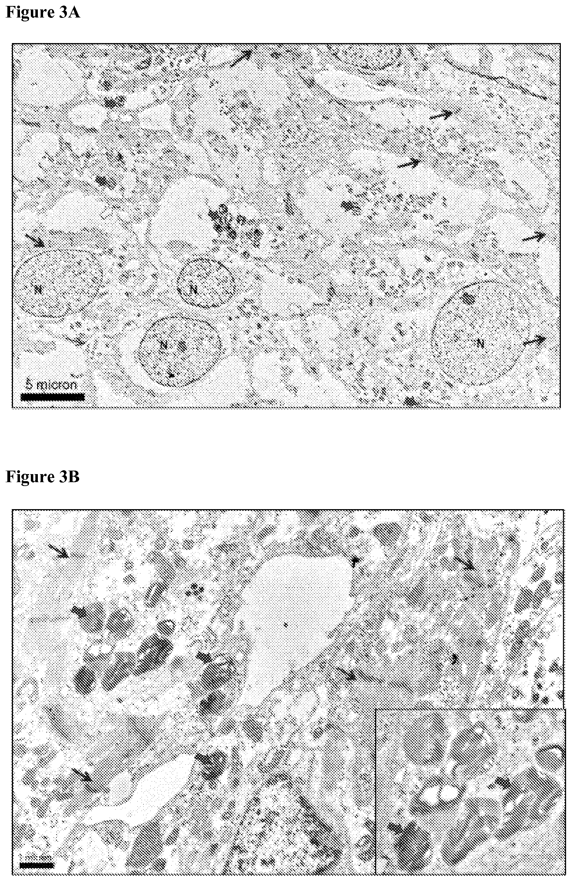

**Figures 3A and 3B****.** Electron microscopy of one month old Fabry iPSC-derived EBs. (A) Cardiomyocyte differentiation and lysosomal storage inclusions in Fabry iPSC-derived EBs. Blue arrows indicate myofibrils with prominent Z bands. Red arrows indicate multi-lamellar, membranous inclusions (lysosomes with substrate storage), yellow arrows indicate intercellular junctions, and N = nucleus. (B) Higher magnification view of a differentiated cardiomyocyte. Inset in Figure 3B is a higher magnification view of lysosomal storage inclusions

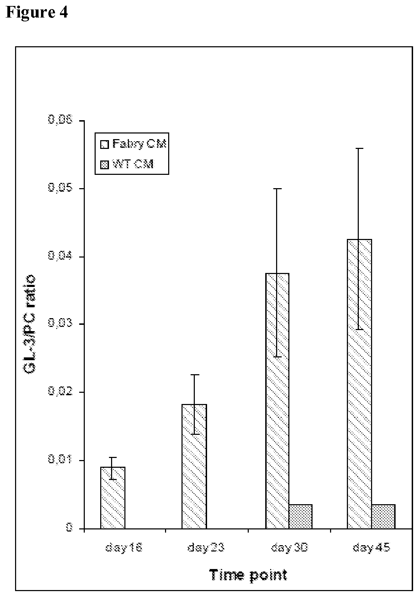

**Figure 4****.** Kinetic of GL-3 accumulation in beating Fabry EBs determined by mass spectrometry. Days are computed from the start of cardiac differentiation. GL-3 content was determined by quantitating nine distinct GL-3 isoforms and normalizing total GL-3 levels to total phosphatidylcholine (PC). Each sample was treated in replicate. The GL-3/PC ratios were averaged across all of the Fabry clones. Number of Fabry samples: day 16, n=4; day 23, n=8; day 30, n=17; day 45, n=8. Number of WT samples: day 30, n=4; day 45, n=5.

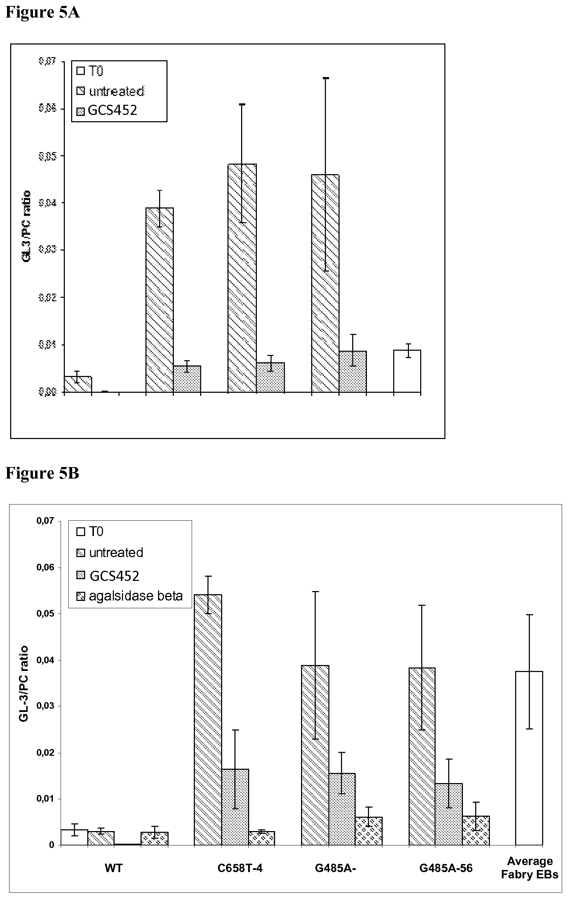

**Figures 5A-5C****.** Effect of µM GCS452 or 3µg/mL agalsidase beta treatment on GL-3 accumulation and clearance in Fabry cardiomyocytes. (A, B) Mass spectrometry of GL-3 content in EBs. Each sample was treated in replicate. T0: EBs before the start of treatment. (A) Prevention against accumulation: treatment at day 18 post-start of cardiac differentiation. Number of untreated/ GCS452 treated samples: WT, n=3/5; C658T-4, n=4/6; G485A-10, n=2/3; G485A-56, n=3/5. T0 average in Fabry EBs of all three iPSC at day 16, n=4. (B) Clearance: treatment at day 28 post-start of cardiac differentiation. Number of untreated/GCS452 treated/agalsidase beta treated samples: WT, n=3/5/2; C658T-4, n=2/5/3; G485A-10, n=3/7/3; G485A-56, n=3/9/4. T0 WT, n=4; T0 average in Fabry EBs of the three iPSC, n=17. (C) GL-3 content and Lamp2 co-localization were determined by immunocytochemistry in one month old treated Fabry iPSC-derived cardiomyocytes and compared with untreated controls cells. GL-3 was not or was very slightly detectable at the end of the treatment. At the same time, the number of Lamp2 vacuoles and the intensity of the signal were reduced.

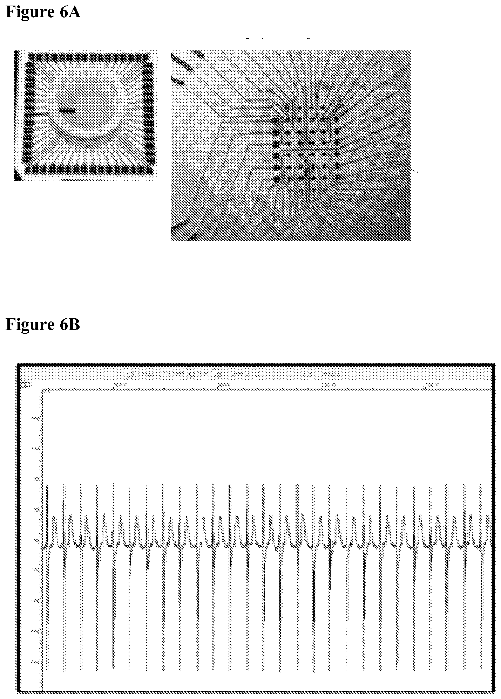

**Figures 6A-6C****.** Electrophysiology of Fabry cardiomyocytes. (A) Depicts MicroElectrode Array (MEA) used to evaluate electrophysiology of cells. (B) Shows the electrophysiology of WT iPSC-derived cardiomyocytes. (C) Shows the electrophysiology of Fabry iPSC-derived cardiomyocytes.

### DETAILED DESCRIPTION

The invention is based, at least in part, on the finding that substrate reduction therapy via glucosylceramide synthase inhibition is able to prevent accumulation and to clear accumulated lysosomal GL-3 in cardiomyocytes (e.g., a non-dividing cardiomycyte). The invention features methods for using glucosylceramide synthase inhibitors to reduce or prevent GL-3 accumulation in a cardiomyocyte of a subject. The invention also features compositions for use in reducing or preventing GL-3 accumulation in a cardiomyocyte of a subject.

Although specific embodiments of the present disclosure will now be described with reference to the preparations and schemes, it should be understood that such embodiments are by way of example only and merely illustrative of but a small number of the many possible specific embodiments which can represent applications of the principles of the present disclosure. Various changes and modifications will be obvious to those of skill in the art given the benefit of the present disclosure and are deemed to be within the spirit and scope of the present disclosure as further defined in the appended claims.

### Glucosylceramide synthase inhibitors

Glucosylceramide synthase (GCS) is a pivotal enzyme which catalyzes the initial glycosylation step in the biosynthesis of glucosylceramide-base glycosphingolipids (GSLs) namely via the pivotal transfer of glucose from UDP-glucose (UDP-Glc) to ceramide to form glucosylceramide. GCS is a transmembrane, type III integral protein localized in the cis/medial Golgi. Glycosphingolipids (GSLs) are believed to be integral for the dynamics of many cell membrane events, including cellular interactions, signaling and trafficking. Synthesis of GSL structures has been shown (see, Yamashita et al., Proc. Natl. Acad. Sci. USA 1999, 96(16), 9142-9147) to be essential for embryonic development and for the differentiation of some tissues. Ceramide plays a central role in sphingolipid metabolism and downregulation of GCS activity has been shown to have marked effects on the sphingolipid pattern with diminished expression of glycosphingolipids. Sphingolipids (SLs) have a biomodulatory role in physiological as well as pathological cardiovascular conditions. In particular, sphingolipids and their regulating enzymes appear to play a role in adaptive responses to chronic hypoxia in the neonatal rat heart (see, El Alwanit et al., Prostaglandins & Other Lipid Mediators 2005, 78(1-4), 249-263).

GCS inhibitors have been proposed for the treatment of a variety of diseases (see for example, WO2005068426). Such treatments include treatment of glycolipid storage diseases (e.g., Tay Sachs, Sandhoffs, GM2 Activator deficiency, GM1 gangliosidosis and Fabry diseases), diseases associated with glycolipid accumulation (e.g., Gaucher disease; Miglustat (Zavesca), a GCS inhibitor, has been approved for therapy in type 1 Gaucher disease patients, see, Treiber et al., Xenobiotica 2007, 37(3), 298-314), diseases that cause renal hypertrophy or hyperplasia such as diabetic nephropathy; diseases that cause hyperglycemia or hyperinsulemia; cancers in which glycolipid synthesis is abnormal, infectious diseases caused by organisms which use cell surface glycolipids as receptors, infectious diseases in which synthesis of glucosylceramide is essential or important, diseases in which synthesis of glucosylceramide is essential or important, diseases in which excessive glycolipid synthesis occurs (e.g., atherosclerosis, polycystic kidney disease, and renal hypertrophy), neuronal disorders, neuronal injury, inflammatory diseases or disorders associated with macrophage recruitment and activation (e.g., rheumatoid arthritis, Crohn's disease, asthma and sepsis) and diabetes mellitus and obesity (see, WO 2006053043).

For example, it has been shown that overexpression of GCS is implicated in multidrug resistance and disrupts ceramide-induced apoptosis. For example, Turzanski et al., Experimental Hematology 2005, 33(1), 62-72 have shown that ceramide induces apoptosis in acute myeloid leukemia (AML) cells and that P-glycoprotein (p-gp) confers resistance to ceramide-induced apoptosis, with modulation of the ceramide-glucosylceramide pathway making a marked contribution to this resistance in TF-1 cells. Thus, GCS inhibitors can be useful for treatment of proliferative disorders by inducing apoptosis in diseased cells.

### Compounds

Unless defined otherwise, all technical and scientific terms used herein have the same meaning as commonly understood by one having ordinary skill in the art to which this disclosure belongs. Although other compounds or methods can be used in practice or testing, certain preferred methods are now described in the context of the following preparations and schemes.

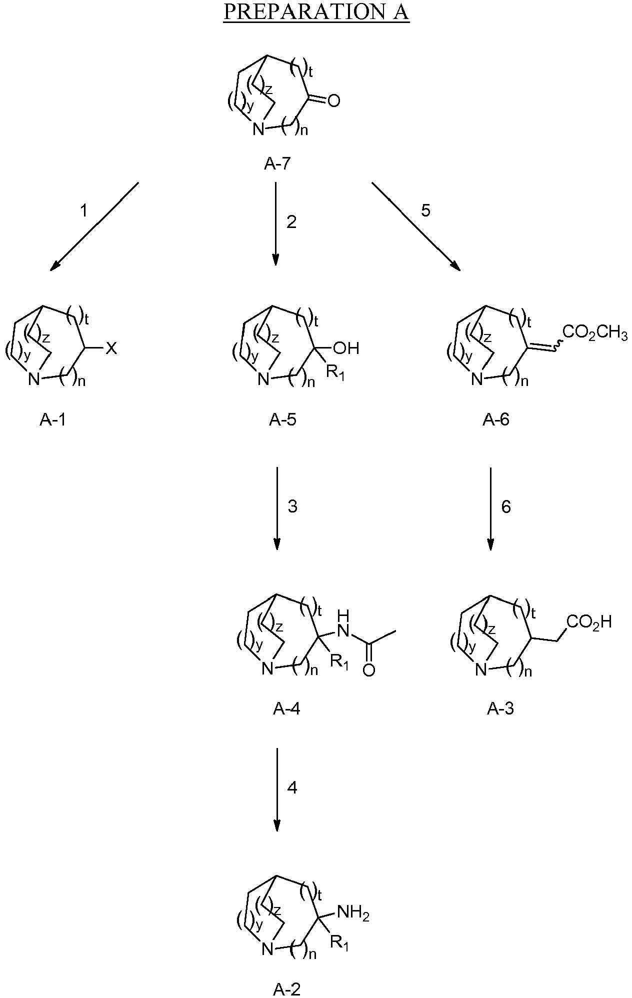

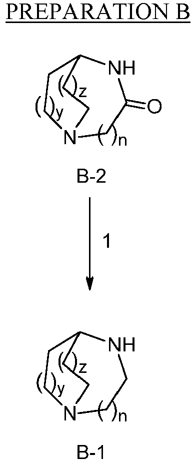

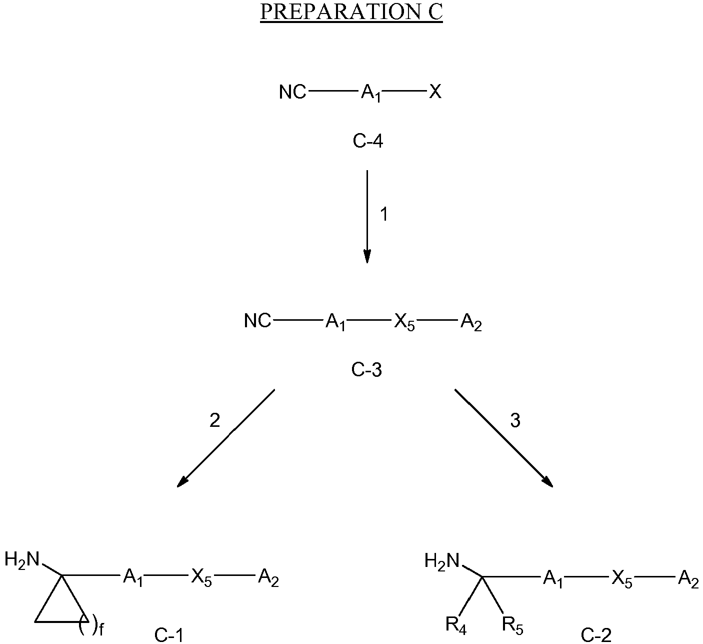

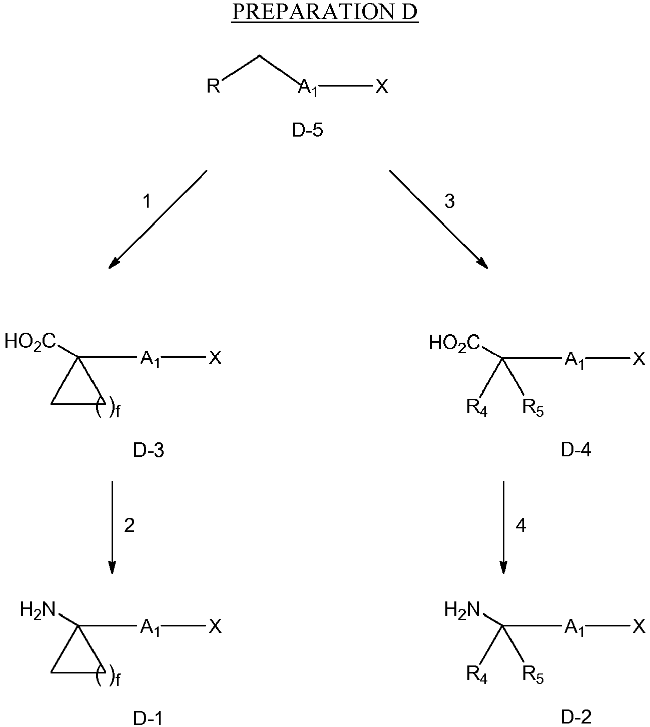

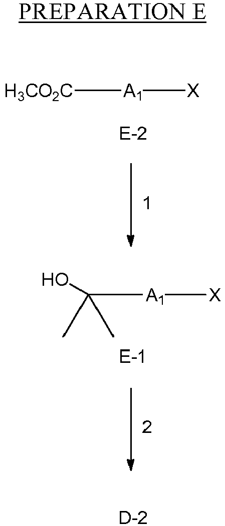

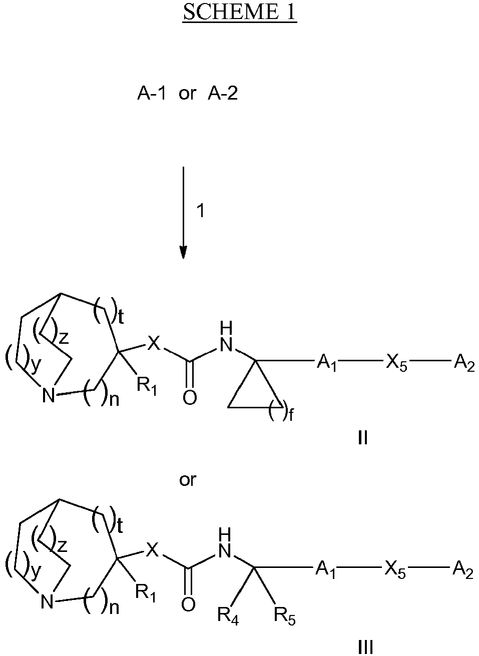

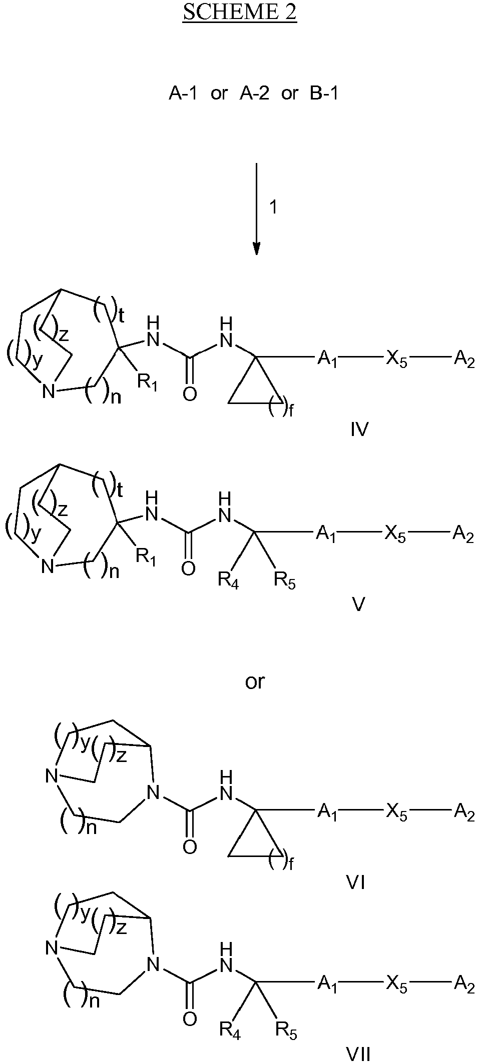

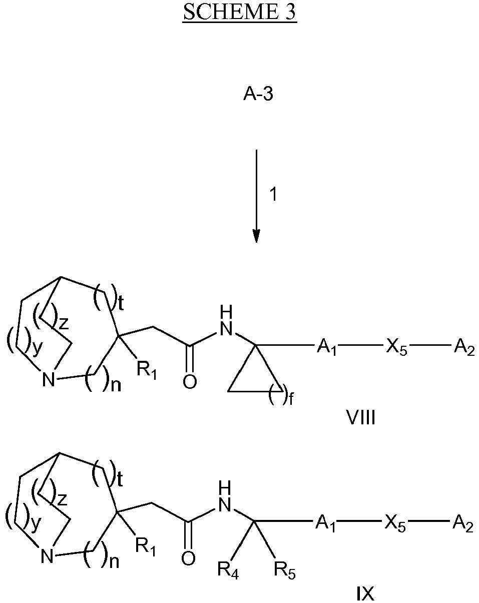

In reaction 1 of Preparation A, the compound of formula A-7 is converted to the corresponding compound of formula A-1, wherein X is OH, by reducing A-7 with a reducing agent, preferably lithium aluminum hydride in aprotic solvent such tetrahydrofuran. The reaction is stirred at a temperature between 0°C and room temperature for a time period between about 15 minutes to about 2 hours, preferably about 30 minutes. Alternatively, the compound of formula A-7 is converted to the corresponding compound of formula A-1, wherein X is OH, by reducing A-7 under approximately 1 atmosphere of hydrogen in presence of a catalyst, preferably platinum oxide, and a polar solvent such methanol or ethanol for a period of 2 hours to 6 hours, preferably 4 hours. Alternatively, the compound of formula A-7 is converted to the corresponding compound of formula A-1, wherein X is NH, by reacting A-7 with hydroxylamine hydrochloride and sodium acetate in a polar solvent such ethanol, methanol, isopropanol, preferably isopropanol. The reaction mixture is stirred at a temperature between 50-80°C for a period of 2 hours to 7 hours, preferably 3 hours. Subsequently, the compound so formed above is converted to compound of formula A-1 with a reducing agent, preferably sodium metallic in a polar protic solvent such ethanol, methanol, propanol, preferably n-propanol. The reaction is stirred overnight at 50-80°C, preferably solvent reflux temperature.

In reaction 2 of Preparation A, the compound of formula A-7 is converted to the corresponding compound of formula A-5, wherein R1, n and z are as defined above, by adding a solution of R1-magnesium bromide in ether to a solution of A-7 in a aprotic solvent, such as ether, at a temperature between about -60° C to about -90° C, preferably about -78° C for a time period between about 1 hour to about 4 hours, preferably about 2 hours. Alternatively, the compound of formula A-7 can be reacted with R1-lithium to afford the compound of formula A-5.

In reaction 3 of Preparation A, the compound of formula A-5 is converted to the corresponding compound of formula A-4, wherein R1, n and z are as defined above, by treating A-5 with a strong acid, preferably sulfuric acid, in the presence of acetonitrile. The reaction is stirred overnight at room temperature.

In reaction 4 of Preparation A, the compound of formula A-4 is converted to the corresponding compound of formula A-3, wherein R1, n and z are as defined above, by treating A-4 with an acid, preferably hydrochloric acid. The reaction is stirred at reflux for a period of 18 hours to 72 hours, preferably 24 hours and basified to pH=8 by treatment with an inorganic base in aqueous solution, such as sodium hydroxide.

In reaction 5 of Preparation A, the compound of formula A-7 is converted to the corresponding compound of formula A-6, wherein R1, n and z are as defined above, by reacting A-7 with a triphenyl phosphonium ylide to give the corresponding alkene compound of formula A-6. The reaction is stirred at room temperature for overnight.

In reaction 6 of Preparation A, the compound of formula A-6 is converted to the corresponding compound of formula A-3, wherein R1, n and z are as defined above, by reducing A-6 under approximately 1 atmosphere of hydrogen in the presence of a catalyst, preferably palladium on carbon, and a polar solvent, such as methanol, ethanol or ethyl acetate. The reaction is stirred at room temperature for a time period between about 2 hours to about 24 hour, preferably about 18 hours. Subsequently, the compound so formed is treated with a base, preferably lithium hydroxide, in a mixture of solvent such tetrahydrofuran, methanol and water to afford the compound of A-3. The reaction is stirred overnight at room temperature.

In reaction 1 of Preparation B, the compound of formula B-2 is converted to the corresponding compound of formula B-1, by reducing B-2 with a reducing agent, preferably lithium aluminum hydride in aprotic solvent such tetrahydrofuran. The reaction is stirred at a temperature between 0°C and room temperature for a time period between about 15 minutes to about 2 hours, preferably about 30 minutes.

In reaction 1 of Preparation C, the compound of C-4 is converted to the corresponding compound of formula C-3, wherein X is bromine or chloride, by reacting C-4 with boronic acid in the presence of a catalyst, preferably 1,1'-bis(diphenylphosphino)ferrocene-palladium(II)-dichloride, and potassium carbonate. The reaction is microwaved in a mixture of dimethoxyethane and water at a temperature between about 130° C to about 170° C, preferably about 150° C, for a time period between about 15 min to about 1 hour, preferably about 30 min. Alternatively, the reaction can be performed using solvent such dioxane and stirred overnight at 100° C under conventional heating.

In reaction 2 of Preparation C, the compound of C-3 is converted to the corresponding compound of formula C-1, wherein f is 1 to 8 and A1, X5 and A2 are as defined above, by adding ethyl magnesium bromide dropwise to a mixture of C-3 and titanium isopropoxide in ether. The reaction is stirred at a temperature between about-50° C to about -90° C, preferably about -70° C. The resulting reaction mixture is allowed to warm to about 20° C to about 30° C, preferably about 25° C, and allowed to stir for an additional time period between about 30 minutes to about 2 hours, preferably about 1 hour. Boron trifluoride diethyl etherate is then added to the mixture dropwise at a temperature between about 20° C to about 30° C, preferably about 25° C.

In reaction 3 of Preparation C, the compound of C-3 is converted to the corresponding compound of formula C-2, wherein A1, X5 and A2 are as defined above, by first stirring a suspension of cerium (III) chloride in an aprotic solvent, such as tetrahyrofuran, at room temperature for time period between about 30 minutes to about 2 hours, preferably about 1 hour. The resulting suspension is cooled to a temperature between about -60° C to about -90° C, preferably about -78° C and an organolithium agent is added, preferably methyl lithium in an ether solution. The resulting organocerium complex is allowed to form for a time period between about 30 minutes to about 2 hours, preferably about 1 hour, followed by the addition of C-3 in an aprotic solvent, such as tetrahydrofuran. The resulting reaction mixture is then warmed to room temperature and allowed to stir for time period between about 16 hours to about 20 hours, preferably about 18 hours.

In reaction 1 of Preparation D, the compound of D-5, wherein R is CO2Et or CN and X is bromine or chloride, is converted to the corresponding compound of formula D-3, by reacting D-5 with an alkyl dihalide such 1,2-dibromoethane. Subsequently, the compound so formed is treated with an inorganic base such lithium hydroxide or potassium hydroxide, in a mixture of solvent such tetrahydrofuran, methanol, glycol and water to afford the compound of D-3, wherein f is 1 to 8. The reaction is stirred overnight at a temperature between 25°C and 130°C. Alternatively, to form the corresponding compound of formula D-3, wherein X is X5-A2, D-5 must first be reacted according to the procedure discussed above in reaction 1 of Preparation C.

In reaction 2 of Preparation D, the compound of D-3 is converted to the corresponding compound of formula D-1 by reacting D-3 with a base such triethylamine and diphenylphosphoryl azide in aprotic solvent such toluene. The reaction was heated to a temperature range between 80°C-110°C, preferably at 110°C for 15 min to 1 hour, preferably 30 minutes. The so formed intermediate is then treated with *tert*-butyl alcohol for overnight period at 60-110 °C, preferably 90 °C. Subsequently, the so formed carbamate is converted to the corresponding compound of formula D-1, wherein f is 1 to 8, by a treatment under acidic media using preferably trifluoroacetic acid in dichloromethane at room temperature for a period of 30 min to 5 hours, preferably 2 hours.

In reaction 3 of Preparation D, the compound of D-5, wherein R is CO2Et or CN and X is bromine or chloride, is converted to the corresponding compound of formula D-4, by reacting D-5 with an alkyl halide such Mel. Subsequently, the compound so formed is treated with an inorganic base such lithium hydroxide or potassium hydroxide, in a mixture of solvent such tetrahydrofuran, methanol, glycol and water to afford the compound of D-4. The reaction is stirred overnight at a temperature between 25°C and 130°C. Alternatively, to form the corresponding compound of formula D-4, wherein X is X5-A2, D-5 must first be reacted according to the procedure discussed above in reaction 1 of Preparation C.

In reaction 4 of Preparation D, the compound of D-4 is converted to the corresponding compound of formula D-2, by reacting D-4 with a base such triethylamine and diphenylphosphoryl azide in aprotic solvent such toluene. The reaction was heated to a temperature range between 80°C-110°C, preferably at 110°C for 15 min to 1 hour, preferably 30 minutes. The so formed intermediate is then treated with *tert*-butyl alcohol for overnight period at 60-110 °C, preferably 90 °C. Subsequently, the so formed carbamate is converted to the corresponding compound of formula D-1 by a treatment under acidic media using preferably trifluoroacetic acid in dichloromethane at room temperature for a period of 30 min to 5 hours, preferably 2 hours.

In reaction 1 of Preparation E, the compound of formula E-2, wherein X is bromide or chloride, is converted to the corresponding compound of formula E-1, by reacting E-2 with methyl magnesium bromide in ether, at a temperature between about - 60° C to about -90° C, preferably about -78° C for a time period between about 30min to about 3 hours, preferably about 2 hours. Alternatively, to form the corresponding compound of formula E-1, wherein X is X5-A2, E-2 must first be reacted according to the procedure discussed above in reaction 1 of Preparation C.

In reaction 2 of Preparation E, the compound of formula E-1 is converted to the corresponding compound of D-2 by treating E-1 with a strong acid, preferably sulfuric acid, in the presence of chloroacetonitrile. The reaction is stirred overnight at room temperature. Subsequently, the so formed compound is treated with thiourea in a polar protic solvent such ethanol for an overnight period at 80°C to form the corresponding compound of formula D-2. Alternatively, E-1 is treated with sodium azide and trifluoroacetic acid in an aprotic solvent such dichloromethane at a temperature range of - 10°C to room temperature, preferably 0°C. The so formed compound is reduced in presence of triphenylphosphine in a solution of tetrahydrofuran and water to form corresponding compound of formula D-2. The reaction is stirred at a temperature range 25-80°C, preferably at room temperature for a period of 2 hours to 24 hours, preferably 18 hours.

In reaction 1 of Scheme 1, the compounds of formula A-1 or A-2 are converted to the corresponding compounds of Formula II, wherein f is 1 to 8, or III, respectively, by adding triphosgene to a suspension of C-1 or C-2 and triethylamine in a aprotic solvent, such as tetrahydrofuran. The reaction is stirred at room temperature for a time period between about 5 minutes to about 20 minutes, preferably about 15 minutes, and a small amount of ether was added. The triethylammonium salt generated is filtered off. Separately, sodium hydride is added to a suspension of A-1 or A-2, wherein X is OH or NH, in an aprotic solvent, such as tetrahydrofuran, at 0°C or room temperature. The reaction is stirred at room temperature for a time period between about 5 minutes to about 20 minutes, preferably about 15 minutes, and the isocyanate tetrahydrofuran/ether solution so formed above is added dropwise. Alternatively, the compounds of Formula II and III may be formed by reacting the compounds of D3 or D4 with A-1 and A-2 in presence of a base such triethylamine and diphenylphosphoryl azide in aprotic solvent such toluene as described in procedure discussed above in reaction 4 of Preparation D.

In reaction 1 of Scheme 2, the compounds of formula A-1, A-2 or B-1 are converted to the corresponding compounds of Formula IV, V, VI and VII, wherein f is 1 to 8, respectively, by adding triphosgene to a suspension of C-1, C-2, D-1 or D-2 and triethylamine in a aprotic solvent, such as tetrahydrofuran or toluene. The reaction is stirred at room temperature for a time period between about 5 minutes to about 20 minutes, preferably about 15 minutes, and a small amount of ether was added. Subsequently, A-1 or A-2, wherein X is NH, is added to the isocyanate solution so formed above and the reaction is stirred at a temperature range of 25-100°C, preferably at room temperature for a period of about 2 hours to 24 hours, preferably 18 hours.

In reaction 1 of Scheme 3, the compound of formula A-3 is converted to the corresponding compounds of Formula VIII, wherein f is 1 to 8, and IX, respectively by reacting A3 with C1, C-2, D-1 or D-2 via peptide coupling using carbodiimide coupling agent such 1-ethyl-3-(3-dimethylaminopropyl) carbodiimide and 1-hydroxy-benzotriazole or 2-(1H-7-Azabenzotriazol-1-yl)-1,1,3,3-tetramethyl uronium hexafluorophosphate in solvent such tetrahydrofuran or dimethylformamide. The reaction is stirred at room temperature for overnight.

Although specific embodiments of the present disclosure will now be described with reference to the preparations and schemes, it should be understood that such embodiments are by way of example only and merely illustrative of but a small number of the many possible specific embodiments which can represent applications of the principles of the present disclosure. Various changes and modifications will be obvious to those of skill in the art given the benefit of the present disclosure and are deemed to be within the spirit and scope of the present disclosure as further defined in the appended claims.

Unless defined otherwise, all technical and scientific terms used herein have the same meaning as commonly understood by one having ordinary skill in the art to which this disclosure belongs. Although other compounds or methods can be used in practice or testing, certain preferred methods are now described in the context of the following preparations and schemes.

All pharmaceutically acceptable salts, prodrugs, tautomers, hydrates and solvates of the compounds presently disclosed are also within the scope of the present disclosure.

Presently disclosed compounds that are basic in nature are generally capable of forming a wide variety of different salts with various inorganic and/or organic acids. Although such salts are generally pharmaceutically acceptable for administration to animals and humans, it is often desirable in practice to initially isolate a compound from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds can be readily prepared using conventional techniques, e.g., by treating the base compound with a substantially equivalent amount of the chosen mineral or organic acid in an aqueous solvent medium or in a suitable organic solvent such as, for example, methanol or ethanol. Upon careful evaporation of the solvent, the desired solid salt is obtained.

Acids which can be used to prepare the pharmaceutically acceptable acid addition salts of the base compounds are those which can form non-toxic acid addition salts, i.e., salts containing pharmacologically acceptable anions, such as chloride, bromide, iodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts.

Presently disclosed compounds that are acidic in nature, e.g., contain a COOH or tetrazole moiety, are generally capable of forming a wide variety of different salts with various inorganic and/or organic bases. Although such salts are generally pharmaceutically acceptable for administration to animals and humans, it is often desirable in practice to initially isolate a compound from the reaction mixture as a pharmaceutically unacceptable salt and then simply convert the latter back to the free acid compound by treatment with an acidic reagent, and subsequently convert the free acid to a pharmaceutically acceptable base addition salt. These base addition salts can be readily prepared using conventional techniques, e.g., by treating the corresponding acidic compounds with an aqueous solution containing the desired pharmacologically acceptable cations, and then evaporating the resulting solution to dryness, preferably under reduced pressure. Alternatively, they also can be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum product yields of the desired solid salt.

Bases which can be used to prepare the pharmaceutically acceptable base addition salts of the base compounds are those which can form non-toxic base addition salts, i.e., salts containing pharmacologically acceptable cations, such as, alkali metal cations (e.g., potassium and sodium), alkaline earth metal cations (e.g., calcium and magnesium), ammonium or other water-soluble amine addition salts such as N-methylglucamine-(meglumine), lower alkanolammonium and other such bases of organic amines.

Isotopically-labeled compounds are also within the scope of the present disclosure. As used herein, an "isotopically-labeled compound" refers to a presently disclosed compound including pharmaceutical salts and prodrugs thereof, each as described herein, in which one or more atoms are replaced by an atom having an atomic mass or mass number different from the atomic mass or mass number usually found in nature. Examples of isotopes that can be incorporated into compounds presently disclosed include isotopes of hydrogen, carbon, nitrogen, oxygen, phosphorous, fluorine and chlorine, such as ²H, ³H, ¹¹C, ¹⁴C, ¹⁵N, ¹⁸O, ¹⁷O, ³¹P, ³²P, ³⁵S, ¹⁸F, and ³⁶Cl, respectively.

By isotopically-labeling the presently disclosed compounds, the compounds may be useful in drug and/or substrate tissue distribution assays. Tritiated (³H) and carbon-14 (¹⁴C) labeled compounds are particularly preferred for their ease of preparation and detectability. Further, substitution with heavier isotopes such as deuterium (²H) can afford certain therapeutic advantages resulting from greater metabolic stability, for example increased in vivo half-life or reduced dosage requirements and, hence, may be preferred in some circumstances. Isotopically labeled compounds presently disclosed, including pharmaceutical salts and prodrugs thereof, can be prepared by any means known in the art.

Stereoisomers (e.g., cis and trans isomers) and all optical isomers of a presently disclosed compound (e.g., R and S enantiomers), as well as racemic, diastereomeric and other mixtures of such isomers are within the scope of the present disclosure.

The compounds, salts, prodrugs, hydrates, and solvates presently disclosed can exist in several tautomeric forms, including the enol and imine form, and the keto and enamine form and geometric isomers and mixtures thereof. Tautomers exist as mixtures of a tautomeric set in solution. In solid form, usually one tautomer predominates. Even though one tautomer may be described, all tautomers are within the scope of the present disclosure.

Atropisomers are also within the scope of the present disclosure. Atropisomers refer to compounds that can be separated into rotationally restricted isomers.

The present disclosure also provides pharmaceutical compositions comprising at least one presently disclosed compound and at least one pharmaceutically acceptable carrier. The pharmaceutically acceptable carrier can be any such carrier known in the art including those described in, for example, Remington's Pharmaceutical Sciences, Mack Publishing Co., (A. R. Gennaro edit. 1985). Pharmaceutical compositions of the compounds presently disclosed may be prepared by conventional means known in the art including, for example, mixing at least one presently disclosed compound with a pharmaceutically acceptable carrier.

Presently disclosed pharmaceutical compositions can be used in an animal or human. Thus, a presently disclosed compound can be formulated as a pharmaceutical composition for oral, buccal, parenteral (e.g., intravenous, intramuscular or subcutaneous), topical, rectal or intranasal administration or in a form suitable for administration by inhalation or insufflation.

The compounds presently disclosed may also be formulated for sustained delivery according to methods well known to those of ordinary skill in the art. Examples of such formulations can be found in United States Patents 3,119,742, 3,492,397, 3,538,214, 4,060,598, and 4,173,626.

For oral administration, the pharmaceutical composition may take the form of, for example, a tablet or capsule prepared by conventional means with a pharmaceutically acceptable excipient(s) such as a binding agent (e.g., pregelatinized maize starch, polyvinylpyrrolidone or hydroxypropyl methylcellulose); filler (e.g., lactose, microcrystalline cellulose or calcium phosphate); lubricant (e.g., magnesium stearate, talc or silica); disintegrant (e.g., potato starch or sodium starch glycolate); and/or wetting agent (e.g., sodium lauryl sulphate). The tablets may be coated by methods well known in the art. Liquid preparations for oral administration may take the form of a, for example, solution, syrup or suspension, or they may be presented as a dry product for constitution with water or other suitable vehicle before use. Such liquid preparations may be prepared by conventional means with a pharmaceutically acceptable additive(s) such as a suspending agent (e.g., sorbitol syrup, methyl cellulose or hydrogenated edible fats); emulsifying agent (e.g., lecithin or acacia); non-aqueous vehicle (e.g., almond oil, oily esters or ethyl alcohol); and/or preservative (e.g., methyl or propyl p-hydroxybenzoates or sorbic acid).

For buccal administration, the composition may take the form of tablets or lozenges formulated in a conventional manner.

Presently disclosed compounds may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain a formulating agent such as a suspending, stabilizing and/or dispersing agent recognized by those of skill in the art. Alternatively, the active ingredient may be in powder form for reconstitution with a suitable vehicle, e.g., sterile pyrogen-free water, before use.

For topical administration, a presently disclosed compound may be formulated as an ointment or cream.

Presently disclosed compounds may also be formulated in rectal compositions such as suppositories or retention enemas, e.g., containing conventional suppository bases such as cocoa butter or other glycerides.

For intranasal administration or administration by inhalation, presently disclosed compounds may be conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the presently disclosed compound. Capsules and cartridges (made, for example, from gelatin) for use in an inhaler or insufflator may be formulated containing a powder mix of a presently disclosed compound and a suitable powder base such as lactose or starch.

A proposed dose of a presently disclosed compound for oral, parenteral or buccal administration to the average adult human for the treatment or prevention of a TPO-related disease state is about 0.1 mg to about 2000 mg. In certain embodiments, the proposed dose is from about 0.1 mg to about 200 mg of the active ingredient per unit dose. Irrespective of the amount of the proposed dose, administration of the compound can occur, for example, 1 to 4 times per day.

Aerosol formulations for the treatment or prevention of the conditions referred to above in the average adult human are preferably arranged so that each metered dose or "puff' of aerosol contains about 20mg to about 10,000mg, preferably, about 20mg to about 1000mg of a presently disclosed compound. The overall daily dose with an aerosol will be within the range from about 100mg to about 100 mg. In certain embodiments, the overall daily dose with an aerosol generally will be within the range from about 100mg to about 10 mg. Administration may be several times daily, for example 2, 3, 4 or 8 times, giving for example, 1, 2 or 3 doses each time.

Aerosol combination formulations for the treatment or prevention of the conditions referred to above in the average adult human are preferably arranged so that each metered dose or "puff' of aerosol contains from about 0.01 mg to about 1000 mg of a combination comprising a presently disclosed compound. In certain embodiments, each metered dose or "puff' of aerosol contains about 0.01 mg to about 100 mg of a combination comprising a presently disclosed compound. In certain embodiments, each metered dose or "puff' of aerosol contains about 1 mg to about 10 mg of a combination comprising a presently disclosed compound. Administration may be several times daily, for example 2, 3, 4 or 8 times, giving for example, 1, 2 or 3 doses each time.

Pharmaceutical compositions and methods of treatment or prevention comprising administering prodrugs of at least one presently disclosed compound are also within the scope of the present disclosure.

### Glucosylceramide Synthase Assay

An enzyme assay using microsomes as a source of glucosylceramide synthase activity. Fluorescent ceramide substrate is delivered to membrane-bound enzyme as a complex with albumin. After reaction, ceramide and glucosylceramide are separated and quantitated by reverse-phase HPLC with fluorescence detection.

### Procedure

### Preparation of Microsomes from A375 human melanoma cells:

Cell suspension was sonicated on ice to complete cell lysis followed by centrifugation at spin down at 10,000 g for 10 min. at 4°C. Supernatant was cleared by centrifugation again at 100,000 g for 1 hour at 4°C in the. Pellet was resuspended in lysis buffer, aliquoted and stored at -80°C.

### Glucosylceramide Synthase Assay

Substrate and microsome were combined 1:1, mix well on a plate shaker seal the plate and incubate 1 hour at room temperature in the dark

The was stop with stop solution into the reaction plate and transferred analysis plate;

### RP-HPLC Analysis

- column: MercuryMS™ (Phenomenex) replaceable cartridge (Luna C₈, 3 µm, 20 x 4 mm)
- system: Agilent 1100 with Agilent 1200 series fluorescence detector
- mobile phase: 1% formic acid in 81% methanol, 19% water, flow rate 0.5 mL/min, isocratic run, 4 min
- sample diluent: 0.1 mM C₈ ceramide (adsorption blocker) in 50% isopropanol, 50% water (v/v)
- fluorescence detection: λₑₓ = 470 nm, λₑₘ = 530 nm
- under these conditions, NBD C₆ GluCer had a retention time of about 1.7 min and NBD C₆ Cer ran at about 2.1 min; the peaks were clearly separate to the baseline and were integrated automatically by the HPLC software
- %conversion of substrate to product was used as the readout for inhibitor testing to avoid variability due to dilution error or sample evaporation

All of the exemplified compounds had an IC₅₀ value of less than 5µM in the Reporter Assay.

### EXPERIMENTAL

### General procedure A: Carbamate/urea formation with triphosgene

To a suspension of amine hydrochloride (1 equivalent) and triethylamine (3-4 equivalents) in a THF (concentration ∼ 0.2M) at room temperature was added triphosgene (0.35 equivalent). The reaction mixture was stirred for 10 min and small amount of ether (1-2 mL) was added. The triethylammonium salt was filtered off to afford a clear solution of isocyanate in THF/ether.

To a solution of alcohol (1.5 equivalents) in THF (concentration ∼ 0.2M) at room temperature was added NaH [60%, oil] (1.5 equivalents). The reaction mixture was stirred for 15 min and the above solution (isocyanate in THF/ether) was added dropwise.

In a standard workup, the reaction was quenched with brine. The solution was extracted with EtOAc and the organic layer was dried over Na₂SO₄, filtered and concentrated. The crude material was purified on combiflash (SiO₂ cartridge, CHCl₃ and 2N NH₃ in MeOH) to afford the corresponding carbamate.

Alternatively: To a suspension of amine hydrochloride A (1 equivalent) and triethylamine (3-4 equivalents) in a THF (concentration ∼ 0.2M) at room temperature was added triphosgene (0.35 equivalent). The reaction mixture was stirred for 10 min and small amount of ether (1-2 mL) was added. The triethylammonium salt was filtered off to afford a clear solution of isocyanate in THF/ether.

To a solution of amine B (1 equivalent) in THF (concentration ∼ 1.0M) at room temperature was added the above solution (isocyanate in THF/ether) dropwise. The reaction was stirred for a period of 18 h and concentrated. The crude material was purified on combiflash (SiO₂ cartridge, CHCl₃ and 2N NH₃ in MeOH) to afford the corresponding urea.

### General procedure B: Alkylation with organocerium

A suspension of CeCl₃ (4 equivalents) in THF (concentration ∼ 0.2M) was stirred at room temperature for 1 h. The suspension was cooled to -78°C and MeLi /Ether [1.6M] (4 equivalents) was added dropwise. The organocerium complex was allowed to form for a period of 1 h and a solution of nitrile (1 equivalent) in THF (concentration 2.0M) was added dropwise. The reaction mixture was warmed up to room temperature and stirred for 18 h. The solution was cooled to 0 °C and quenched with water (∼ 1 mL) followed by addition of 50% aqueous solution of ammonium hydroxide (∼ 3 mL) until precipitated formed and settled to the bottom of the flask. The mixture was filtered through a pad of celite and concentrated. The crude material was treated with a solution of HCl/dioxane [4.0M]. The intermediate arylpropan-2-amine hydrochloride was triturated in ether and used as is for the next step. Alternatively, the crude free base amine was purified on combiflash (SiO₂ cartridge, CHCl₃ and 2N NH₃ in MeOH) to afford the corresponding arylpropylamine.

### General procedure C: Urea formation with carbonyl diimidazole (CDI)

A solution of amine A (1 equivalent) and CDI (1.3 equivalent) in THF (concentration ∼ 0.15M) was stirred at reflux for 1 h. A solution of amine B (1.3 equivalent) in THF was added and the reaction mixture was stirred for an additional 1.5 h. The reaction was cooled to room temperature and diluted with ether. The desired compound precipitated and was filtered off. The crude material was purified on combiflash (basic Al₂O₃ cartridge, CHCl₃ and MeOH) or (SiO₂ cartridge, CHCl₃ and 2N NH₃ in MeOH) to afford the corresponding urea.

### General procedure D: Urea formation with triphosgene

To a suspension of amine A (1 equivalent) and triethylamine (4 equivalents) in a THF (concentration ∼ 0.15M) at room temperature was added triphosgene (0.35 equivalent). The reaction mixture was stirred for 15 min and amine B (1.1 equivalent) was added. The reaction mixture was stirred at room temperature for 18 h and then diluted with EtOAc. The organic layer was washed with aqueous NaOH [1.0 M], dried over Na₂SO₄. filtered and concentrated. The crude material was purified on combiflash (SiO₂ cartridge, CHCl₃ and 2N NH₃ in MeOH) to afford the corresponding urea.

### General procedure E: Suzuki coupling

To a solution of aryl halide (1 equivalent) in a mixture of DME/water [4:1] (concentration ∼ 0.2M) was added boronic acid (2 equivalents), palladium catalyst (0.1-0.25 equivalent) and sodium carbonate (2 equivalents). The reaction mixture was microwaved 25 min at 150 °C. After filtering through a celite plug and concentrating, the crude product was purified on combiflash (SiO₂ cartridge, CHCl₃ and 2N NH₃ in MeOH) to afford the corresponding coupling adduct.

Alternatively: To a solution of aryl halide (1 equivalent) in a mixture of toluene/water [20:1] (concentration ∼ 0.2 M) was added boronic acid (1.3-2.5 equivalents), palladium catalyst (0.05-0.15 equivalent), tricyclohexylphosphine (0.15-0.45 equivalent) and potassium phosphate (5 equivalents). The reaction mixture was microwaved 25 min at 150° C. After filtering through a celite plug and concentrating, the crude product was purified on combiflash (SiO₂ cartridge, CHCl₃ and 2N NH₃ in MeOH) to afford the corresponding coupling adduct.

### General procedure F: Hydrogenation

To a solution of substrate in methanol, ethanol or EtOAc (concentration ∼ 0.2M) was added palladium catalyst (20% w/w of substrate). The reaction mixture was stirred at room temperature under 1 atm of H₂ until completion. The reaction was filtered through a celite plug followed by two rinses with chloroform. The crude product was concentrated and purified on combiflash (SiO₂ cartridge, CHCl₃ and 2N NH₃ in MeOH) to afford the hydrogenated product. Alternatively, the final material was purified by precipitation or recrystallization.

### General procedure G: Cyclopropanation

To a mixture of arylnitrile (1 equivalent) and Ti(Oi-Pr)₄ (1.7 equivalents) stirring at -70 °C, was added dropwise EtMgBr [3.0 M in ether] (1.1 equivalents). The reaction mixture was allowed to warm to 25 °C and stirred for 1 h. To the above mixture was added BF₃·Et₂O (3 equivalents) dropwise at 25 °C. After the addition, the mixture was stirred for another 2 h, and then quenched with aqueous HCl [2M]. The resulting solution was then basified by adding aqueous NaOH [2M]. The organic material was extracted with ethyl ether. The organic layers were combined, dried over Na₂SO₄, filtered and concentrated. The crude material was purified by silica gel column chromatography (eluting with petroleum ether/EtOAc: 10/1 to 1/1) to give the corresponding 1-aryl-cyclopropanamine.

### General procedure H: Coupling via in-situ Curtius rearrangement

A mixture of acid (1 equivalent), triethylamine (2.5 equivalents), DPPA (1.0 equivalent) in toluene (concentration ∼ 0.3M) was refluxed for 30 min. The mixture was cooled to room temperature and alcohol (1 equivalent) was added. After addition, the mixture was heated at 90 °C for 18 h. The reaction was cool down to room temperature, diluted with EtOAc and washed with saturated aqueous sodium dicarbonate. The organic phase was dried over Na₂SO₄, concentrated and purified by prep-TLC (EtOAc/MeOH 5:1, containing 1% of TEA) to afford the corresponding carbamate.

### General procedure I: Amide formation using EDCI

To a solution of amine (1 equivalent) in DMF or THF (concentration ∼ 0.3M) was added EDCI (1.2-2.5 equivalents), HOBT (1.2-2.5 equivalents), DIPEA (1.2-2.5 equivalents) and triethylamine (few drops). The reaction mixture was stirred and the acid (1.2 equivalents) was added. The reaction was stirred at room temperature for 18 h and then concentrated. The residue was dissolved in EtOAc and washed with brine. The organic layer was dried over Na₂SO₄ and concentrated. The crude material was purified by prep- HPLCMS or by combiflash (SiO₂ cartridge, CHCl₃ and 2N NH₃ in MeOH).

### Preparation A

### Intermediate 1

### 2-(3-bromophenyl)propan-2-amine hydrochloride

To a solution of methyl 3-bromobenzoate (15.0 g, 69.8 mmol) in THF (140 mL) at -78 °C was added dropwise a solution of MeMgBr/diethyl ether [3.0M] (58 mL). The reaction mixture was warmed up to room temperature and stirred for 2 h. The solution was poured to an aqueous saturated solution of ammonium chloride and the organic material was extracted with EtOAc. The organic layer was dried over Na₂SO₄, filtered and concentrated to afford the corresponding alcohol (14.9 g) which was used without further purification.

To a solution of 2-(3-bromophenyl)propan-2-ol (17.2 g, 79.8 mmol) in chloroacetonitrile (160 mL) was added acetic acid (14 mL). The reaction mixture was cooled to 0 °C and H₂SO₄ (14 mL) was added dropwise. The reaction mixture was warmed to room temperature and stirred for 18 h. The reaction was then poured into ice and extracted with EtOAc. The organic layer was washed with aqueous NaOH [1.0M] solution and brine, dried over Na₂SO₄ and concentrated to afford the corresponding chloroacetamide (21.4 g) which was used without further purification.

To a solution of N-(2-(3-bromophenyl)propan-2-yl)-2-chloroacetamide (20.3 g) in ethanol (120 mL) was added acetic acid (20 mL). The reaction mixture was stirred at reflux for 18 h. The solution was cooled to room temperature and the precipitate was filtered off on a celite pad. The filtrate was concentrated and the residue was dissolved in EtOAc. The organic layer was treated with aqueous NaOH [1.0M] solution, dried over Na₂SO₄ and concentrated. The crude material was treated with a solution of HCl/dioxane [4M]. The intermediate 2-(3-bromophenyl)propan-2-amine hydrochloride was triturated in ether and used as is for the next step (7.50 g, 43%). ¹H NMR (400 MHz, CD₃OD) δ 7.69 (q, *J* = 1.8 Hz, 1H), 7.55 (ddd, *J* = 1.0, 1.8, 7.9 Hz, 1H), 7.49 (ddd, *J* = 1.0, 2.0, 8.0 Hz, 1H), 7.38 (t, *J* = 8.0 Hz, 1H), 1.71 (s, 6H) ppm.

### Preparation B

### Intermediate 2

### 2-(5-bromo-2-fluorophenyl)propan-2-amine hydrochloride

To a solution of 5-bromo-2-fluorobenzoic acid (4.85 g, 22.8 mmol) in methanol (45 mL) was added H₂SO₄ (4.5 mL). The reaction mixture was stirred at room temperature for 18 h and the solution was concentrated. The residue was treated with an aqueous NaOH [10% w/v] solution and the organic material was extracted with CHCl₃. The organic layer was dried over Na₂SO₄, filtered and concentrated to afford the corresponding ester (4.69 g, 91%) which was used without further purification.

The ester intermediate (4.69 g, 20.1 mmol) was converted to intermediate 2 using the same procedure reported in example intermediate 1 to afford the corresponding ammonium salt (3.94 g, 67% overall yield) as a white solid. ¹H NMR (400 MHz, CD₃OD) δ 7.67 - 7.57 (m, 2H), 7.21 (dd, *J* = 8.7, 12.3 Hz, 1H), 1.77 (s, 6H) ppm.

### Intermediate 3

### 2-(3-bromo-4-fluorophenyl)propan-2-amine hydrochloride

5-Bromo-2-fluorobenzoic acid was transformed to intermediate 3 using the same procedure reported in example intermediate 2 to afford the corresponding ammonium salt (2.79 g, 49% overall yield) as a white solid.

### Intermediate 4

### 2-(3-bromo-2-fluorophenyl)propan-2-amine

3-Bromo-2-fluorobenzoic acid was transformed to intermediate 4 using the same procedure reported in example intermediate 2 to afford the corresponding amine as pale yellow oil.

### Intermediate 5

### 2-(4-bromophenyl)propan-2-amine hydrochloride

Using general procedure B, bromobenzonitrile (2.00 g, 11.0 mmol) was converted to the corresponding 2-(4-bromophenyl)propan-2-amine, which was afforded as a brown oil (1.20 g, 51 %).

### Preparation C

### Intermediate 6

### 1,4-Diazabicyclo[3.2.2]nonane

To a stirred solution of 1,4-diazabicyclo[3.2.2]nonan-3-one (1.0 g, 7.2 mmol) in 1,4-dioxane (7.2 mL) at room temperature was added lithium aluminum hydride [2.0M/THF] (4.1 mL, 8.2 mmol). The reaction mixture was then heated at reflux for 6 hours before cooling to room temperature. The reaction was quenched by the stepwise addition of 200 µL of H₂O, 200 µL of 15% aqueous NaOH, and 600 µL of H₂O. The mixture was filtered through Celite which was subsequently washed with EtOAc. The combined filtrate was concentrated *in vacuo* to afford the product (0.82 g, 90%) which was used without further purification. ¹H NMR (400 MHz, CDCl₃) δ 3.28-3.25 (m, 1H), 2.99-2.95 (m, 8H), 1.86-1.80 (m, 3H), 1.69-1.64 (m, 2H) ppm.

### Preparation D

### Intermediate 7

### 2-Methylquinuclidin-3-ol

A solution of potassium carbonate (11.4g, 82.8 mmol) and quinuclidine hydrate (5.00 g, 20.4 mmol) was dissolved in H₂O (15.6 mL). When completely dissolved, dichloromethane (20.4 mL) was added and the reaction was stirred at room temperature overnight. The organic phase was separated and the aqueous phase extracted with chloroform (3 x 50 mL). The combined organic layers were dried over MgSO₄, filtered and concentrated *in vacuo.* The product was used without further purification. ¹H NMR (400 MHz, CDCl₃) 2.79 (s, 1H), 5.19 (s, 1H), 3.14-3.06 (m, 2H), 2.99-2.91 (m, 2H), 2.57-2.55 (m, 1H), 1.98-1.93 (m, 4H) ppm.

The 2-methylenequinuclidin-3-one (3.50 g) in ethanol (30 mL) was reduced over 10% Pd/C (50 wt%) under a H₂ atmosphere. When judged complete by TLC (∼3 days), the catalyst was filtered off and the filter cake washed with ethyl acetate. The solvent was removed *in vacuo* to afford the desired product (2.80 g, 80%) was obtained and used without further purification. ¹H NMR (400 MHz, CDCl₃) 3.37-3.31 (m, 1H), 3.21-3.13 (m, 2H), 3.09-3.00 (m, 1H), 2.97-2.89 (m, 1H), 2.46-2.43 (m, 1H), 2.05-1.91 (m, 4H), 1.34 (d, *J* = 7.6 Hz, 3H) ppm.

To 2-methylquinuclidin-3-one (0.50g, 3.60 mmol) in 1,4-dioxane (18 mL) at room temperature was added lithium aluminum hydride [1.0M/THF] (4.1, 4.1 mmol). The reaction mixture was stirred at room temperature for 15 minutes. The reaction was quenched by the stepwise addition of 116 µL of H₂O, 116 µL of 15% aqueous NaOH, and 348 µL of H₂O. The mixture was filtered through Celite which was subsequently washed with EtOAc. The solvent was removed *in vacuo* to afford the product (0.48 g, 95%), which was used without further purification as a 2:1 mixture of diastereomers.

### Preparation E

### Intermediate 8

### 1-azabicyclo[3.2.2]nonan-4-ol

To 1-azabicyclo[3.2.2]nonan-4-one (0.20 g, 1.4 mmol) in 1,4-dioxane (2.8 mL) at 0 °C was added lithium aluminum hydride [1.0M/THF] (1.7 mL, 1.7 mmol). The reaction mixture was maintained at 0 °C for 15 minutes. The reaction was quenched by the stepwise addition of 46 µL of H₂O, 46 µL of 15% aqueous NaOH, and 138 µL of H₂O. The mixture was filtered through Celite which was subsequently washed with EtOAc. The solvent was removed *in vacuo* to afford the product (0.19 g, 96%) which was used without further purification. ¹H NMR (400 MHz, CDCl₃) δ 3.90-3.86 (m, 1H), 3.09-3.03 (m, 1H), 2.96-2.91 (dd, *J* = 9.2, 6.8 Hz, 1H), 2.86-2.75 (m, 3H), 2.71-2.64 (m, 1H), 2.34-2.27 (bs s, 1H), 1.98-1.86 (m, 3H), 1.71-1.59 (m, 3H), 1.51-1.35 (m, 1H) ppm.

### Preparation F

### Intermediate 9

### 1-Azabicyclo[2.2.1]heptan-3-ol

To a mixture of sodium methoxide (2.00 g, 37.9 mmol) in methanol (9 mL) at 0 °C was added glycine methyl ester hydrochloride (4.76 g, 37.9 mmol) and dimethyl itaconate (5.00 g, 31.6 mmol.) The reaction was heated at reflux for 16 hours before cooling to room temperature. The solid was filtered off and washed with dichloromethane. The filtrate was concentrated and the residue diluted with 5N HCl (50 mL). The aqueous layer was extracted with dichloromethane (4 x 50mL), dried over MgSO₄, filtered and concentrated *in vacuo.* The product was used without further purification. ¹H NMR (400 MHz, CDCl₃) δ 4.04 (dd, *J* = 82.0, 17.6 Hz), 3.74-3.64 (m, 8H), 3.32-3.24 (m, 1H), 2.77-2.63 (m, 2H) ppm.

To methyl 1-(2-methoxy-2-oxoethyl)-5-oxopyrrolidine-3-carboxylate (3.40 g, 16.0 mmol) in THF (20 mL) at 0 °C was added borane-THF [1.0M/THF] (32.0 mL, 32.0 mmol). The reaction was stirred at reflux for 1 hour and then cooled to room temperature where it was allowed to stir an additional 12 hours. The reaction was quenched by the addition of a saturated solution of potassium carbonate (5.52 g in 20 mL H₂O) and heated at reflux for an additional 1 hour before cooling to room temperature. The solvent was removed *in vacuo* and the residue made acidic by the addition of 5N HCl (25 mL). The aqueous layer was extracted with dichloromethane (2 x 30 mL). The pH of the aqueous layer was then made basic by the addition of solid potassium carbonate. The aqueous layer was further extracted with dichloromethane (5 x 30 mL). The combined organic layers were dried over MgSO₄, filtered and concentrated *in vacuo.* The product was used without further purification. ¹H NMR (400 MHz, CDCl₃) δ 3.66 (s, 3H), 3.63 (s, 3H), 3.29 (ABq, 2H, *J* = 24.0, 16.8 Hz), 3.06-3.02 (m, 2H), 2.87-2.81 (m, 1H), 2.71-2.65 (m, 1H), 2.56-2.50 (m, 1H), 2.09-2.04 (m, 2H) ppm.

To a refluxing solution of potassium *tert*-butoxide (2.46 g, 22.0 mmol) in toluene (32 mL) was added dropwise a solution of methyl 1-(2-methoxy-2-oxoethyl)pyrrolidine-3-carboxylate (2.00 g, 10.0 mmol) in toluene (10 mL) over 1 hour. The reaction was allowed to stir and additional 3 hours at reflux before first cooling to room temperature then cooling to -10 °C. Acetic acid (1.3 mL) was then added with stirring. The toluene layer was extracted with 5N HCl (4 x 50mL). The combined aqueous layers were heated at 110 °C for 8 hours. The reaction was then cooled to room temperature and the volume reduced by half *in vacuo.* The pH of the reaction mixture was made basic by the addition of solid potassium carbonate. The aqueous layer was extracted with dichloromethane (5 x 50mL) and the combined organic layers were concentrated *in vacuo.* To the crude product was added ethyl ether. The solid filtered off to afford the desired product (0.30 g, 27%) which was used without further purification. ¹H NMR (400 MHz, CDCl₃) δ 3.05-2.96 (m, 3H), 2.76 (s, 2H), 2.72-2.66 (m, 2H), 2.09-2.01 (m, 1H), 1.78-1.71 (m, 1H) ppm.

The 1-azabicyclo[2.2.1]heptan-3-one (0.30 g, 2.7 mmol) in ethanol (2-3 mL) was reduced over PtO₂ (50 wt%) under a H₂ atmosphere. After stirring 4 hours, the catalyst was filtered off and the filtercake washed with ethanol. The ethanol was removed *in vacuo* to afford the desired product (0.29 g, 95%). ¹H NMR (400 MHz, CDCl₃) δ 4.36-4.35 (m, 1H), 3.10-3.05 (m, 1H), 2.95-2.88 (m, 1H), 2.72-2.66 (m, 1H), 2.63-2.57 (m, 2H), 2.48-2.44 (dd, *J* = 10.0, 3.2 Hz, 1H), 2.11-2.05 (m, 2H), 1.51-1.44 (m, 1H) ppm.

### Preparation G

### Intermediate 10

### (R)-3-methylquinuclidin-3-amine and (S)-3-methylquinuclidin-3-amine

To a well-stirred solution of MeLi [3.0M/diethyl ether] (67.0 mL, 201 mmol) in anhydrous diethyl ether (150 mL) at -78 °C was added, dropwise, a solution of quinuclidin-3-one (12.5 g, 100 mmol) in diethyl ether (100 mL). The resulting solution was maintained at -78 °C for 1 hour, then at room temperature for 18 hours. Water (60 mL) was added dropwise at 0 °C and the mixture was concentrated *in vacuo* to give a residue, which was purified by neutral aluminum oxide column chromatography (0-20% MeOH in CHCl₃) to give 3-methylquinuclidin-3-ol (10.0 g, 71%) as a light yellow solid. To stirred acetonitrile (250 mL) at 0 °C was slowly added concentrated sulfuric acid (100 mL). The resulting solution was added dropwise to a mixture of 3-methylquinuclidin-3-ol (9.10 g, 64.5 mmol) in acetonitrile (250 mL) at 0 °C. The reaction mixture was stirred at room temperature for 60 hours, then cooled with an ice bath and basified with aqueous sodium hydroxide solution to pH 10. The mixture was extracted with 5:1 (v/v) CHCl₃/*i-*PrOH. The organic layer was concentrated to afford a residue which was diluted with 2N aq. HCl and washed with 5:1 (v/v) CHCl₃/*i*-PrOH. The remaining aqueous layer was then basified with 2N NaOH and extracted with 5:1 (v/v) CHCl₃/*i*-PrOH. The combined organic layers were washed with water, dried (Na₂SO₄) and concentrated to give 9.5 g (82%) of the desired compound as a light yellow oil. The 2 enantiomers of the above intermediate are separated from each other by using chiral column on supercritical fluid chromatography (SFC) system.

A solution of the above chiral acetamide intermediate (9.50 g, 52.0 mmol) in conc. HCl (100 mL) was refluxed for 3 days, cooled with an ice bath and neutralised with aqueous sodium hydroxide solution to pH 1. The mixture was washed with 5:1 (v/v) CHCl₃/*i*-PrOH. The aqueous layer was then basified with 2N NaOH and extracted with 5:1 (v/v) CHCl₃/*i*-PrOH). The combined extracts were washed with water, dried (Na₂SO₄) and concentrated to give 5.00 g (69%) of the desired chiral compound as a light yellow semi-solid. ¹H NMR (500 MHz, DMSO-*d₆*) δ 2.72-2.39 (m, 6H), 2.01-1.96 (m, 1H), 1.67-1.61 (m, 1H), 1.43-1.36 (m, 2H), 1.23-1.17 (m, 1H), 1.09 (s, 3H) ppm. ¹³C NMR (125 MHz, DMSO-*d₆*) δ 65.3, 48.3, 46.6, 46.4, 34.2, 30.0, 24.8, 22.8 ppm. Purity: > 99% (GC-MS); retention time 6.63 min; (M) 140.1.

### Preparation H

### Intermediate 11

### 2-(3-(4-fluorophenyl)isothiazol-5-yl)propan-2-amine

To a stirred suspension of 4-fluorobenzamide (70.00 g, 503.1 mmol) in toluene (900 mL) was added chlorocarbonyl sulfenyl chloride (83.0 mL, 1.00 mol). The mixture was heated overnight at 60 °C and concentrated. The resulting tan solid was triturated with methylene chloride (200 mL), collected by suction filtration and rinsed with additional methylene chloride (4 x 70 mL). The crude product was impregnated onto silica (100 g) and chromatographed in a large filter funnel dry loaded with silica using a hexane/ethyl acetate gradient. The product 5-(4-fluorophenyl)-1,3,4-oxathiazol-2-one was afforded as an off-white solid (55.98 g, 56%).

To a stirred solution of 5-(4-fluorophenyl)-1,3,4-oxathiazol-2-one (42.80 g, 217.1 mmol) in o-dichlorobenzene (600 mL) was added ethyl propiolate (66.0 mL, 651 mmol). The mixture was heated overnight at 135 °C and concentrated. The residual oil was purified by flash chromatography using a hexane/ethyl acetate gradient to afford ethyl 3-(4-fluorophenyl)isothiazole-5-carboxylate as a pale golden solid (17.35 g, 32%). The more polar, ethyl 3-(4-fluorophenyl)isothiazole-4-carboxylate isomer (generated in ∼57/43 ratio versus the desired product) was discarded.

To a stirred and cooled (0 °C) solution of ethyl 3-(4-fluorophenyl)isothiazole-5-carboxylate (38.50 g, 153.2 mmol) in THF (400 mL) was added a solution of methylmagnesium bromide in diethyl ether (3.0 M, 128 mL, 384 mmol), dropwise over 20 minutes. After another 1.5 hours at 0 °C, the reaction was quenched by the slow addition of ethyl acetate (20 mL) and concentrated. The residue was taken up in aqueous NH₄Cl (400 mL) and extracted with ethyl acetate (2 x 150 mL). The combined extracts were dried (Na₂SO₄) and concentrated. The resulting amber syrup was purified by flash chromatography using a hexane/ethyl acetate gradient to afford 2-(3-(4-fluorophenyl)isothiazol-5-yl)propan-2-ol as soft, golden solid (29.02 g, 80%).

2-(3-(4-Fluorophenyl)isothiazol-5-yl)propan-2-ol (29.00 g, 122.2 mmol) was taken up in thionyl chloride (75 mL). The mixture was cooled (ice bath) briefly and stirred. After 4 hours the reaction was concentrated and the residue was partitioned between ethyl acetate (200 mL) and aqueous NaHCO₃ (300 mL). The organic layer was combined with a backextract of the aqueous layer (ethyl acetate, 1 x 100 mL), dried (Na₂SO₄) and concentrated to afford a mixture of 5-(2-chloropropan-2-yl)-3-(4-fluorophenyl)isothiazole product and 3-(4-fluorophenyl)-5-(prop-1-en-2-yl)isothiazole elimination byproduct (∼63/39 ratio) as a dark amber oil (29.37 g). This material was used without purification in the next reaction.

To a stirred solution of the product of the previous step in DMSO (80 mL) was added sodium azide (14.89 g, 229.0 mmol). The mixture was heated at 50 °C overnight, diluted with ethyl acetate (250 mL) and washed with water (6 x 400 mL). The organic layer was dried (Na₂SO₄) and concentrated to afford a mixture of 5-(2-azidopropan-2-yl)-3-(4-fluorophenyl)isothiazole and 3-(4-fluorophenyl)-5-(prop-1-en-2-yl)isothiazole (∼56/44 ratio) as a dark amber oil (29.10 g). This material was used without purification in the next reaction.

The product of the previous step was combined with 10% palladium on carbon (50% water; 7.50 g) and taken up in methanol (350 mL). The stirred suspension was cycled between vacuum and a nitrogen purge three times. After an additional evacuation, the reaction was backfilled with hydrogen gas (balloon reservoir) and stirred overnight. The reaction was filtered through Celite. The filtrate was combined with methanol rinsings of the Celite and concentrated. The resulting dark amber oil purified by flash chromatography using a methylene chloride/methanol gradient to afford 2-(3-(4-fluorophenyl)isothiazol-5-yl)propan-2-amine as viscous, amber oil (14.23 g, 49% over 3 steps).

Several approaches are being used or pursued for the treatment of LSDs, most of which focus on enzyme replacement therapy for use alone in disease management. Numerous approved enzyme replacement therapies are commercially available for treating LSDs (e.g., Myozyme® for Pompe disease, Aldurazyme® for Mucopolysaccharidosis I, Cerezyme® for Gaucher disease and Fabrazyme® for Fabry disease). Additionally, the inventors have identified a number of small molecules for use alone in the management of LSDs. The therapeutic methods described herein provide treatment options for the practitioner faced with management of various lysosomal storage diseases, as described in detail below.

In certain aspects of the disclosure, the compounds of the present disclosure may be used to treat a metabolic disease, such as a lysosomal storage disease (LSD), either alone or as a combination therapy with an enzyme replacement therapy. In other aspects of the disclosure, the compounds of the present discosure may be used to inhibit or reduce GCS activity in a subject diagnosed with a metabolic disease, such as an LSD, either alone or as a combination therapy with an enzyme replacement therapy. In other aspects of the disclosure, the compounds of the present disclosure may be used to reduce and/or inhibit the accumulation of a stored material (e.g., lysosomal substrate) in a subject diagnosed with a metabolic disease, such as an LSD. In certain embodiments of the foregoing aspects, the LSD is Gaucher (type 1, type 2 or type 3), Fabry, G_{M1}-gangliosidosis or G_{M2}-gangliosidoses (e.g., GM2 Activator Deficiency, Tay-Sachs and Sandhoff). Table 1 lists numerous LSDs and identifies the corresponding deficient enzyme that may be used as an ERT in the foregoing aspects of the disclosure.

In other scenarios it may be necessary to provide SMT to a patient whose condition requires the reduction of substrates in the brain and thus is not treatable by systemic administration of ERT. While direct intracerebroventricular or intrathecal administration can reduce substrate levels in the brain, systemic administration of ERT is not amenable for LSD's with Central Nervous System (CNS) involvement due to its incapacity to cross the Blood Brain Barrier (BBB) and SMT may prove beneficial in patients having residual enzymatic activities in the CNS.

In accordance with the present disclosure, SMT is provided to a patient to treat a cancer and/or metabolic disease, such as, a lysosomal storage disease. The SMT may include one or more small molecules. The SMT includes administering to the patient compounds of the present disclosure. In particular embodiments, the compound is (S)-Quinuclidin-3-yl (2-(2-(4-fluorophenyl)thiazol-4-yl)propan-2-yl)carbamate or Quinuclidin-3-yl (2-(4'-fluoro-[1,1'-biphenyl]-3-yl)propan-2-yl)carbamate, or combinations thereof.

In certain embodiments, compounds of the disclosure, such as, for example, (S)-Quinuclidin-3-yl(2-(2-(4-fluorophenyl)thiazol-4-yl)propan-2-yl)carbamate and Quinuclidin-3-yl (2-(4'-fluoro-[1,1'-biphenyl]-3-yl)propan-2-yl)carbamate may be used for treatment of virtually any storage disease resulting from a defect in the glycosphingolipid pathway (e.g. Gaucher (i.e., type 1, type 2 type 3), Fabry, G_{M1}-gangliosidosis, G_{M2}-gangliosidoses (e.g., GM2 Activator Deficiency, Tay-Sachs and Sandhoff)). In a particularly preferred embodiment, (S)-Quinuclidin-3-yl (2-(2-(4-fluorophenyl)thiazol-4-yl)propan-2-yl)carbamate or a pharmaceutically acceptable salt or prodrug thereof is used to inhibit and/or reduce the accumulation of Gb3 and/or lyso-Gb3 in a patient with Fabry disease, either alone or as a combination therapy with enzyme replacement therapy. In a preferred embodiment, the enzyme replacement therapy includes administering alpha-galactosidase A to the Fabry patient. Indeed, it has been demonstrated that a GCS inhibitor of the invention effectively reduces Gb3 and lyso-Gb3 storage in a mouse model of Fabry disease, thus supporting its use as a viable approach for the treatment of Fabry disease. See WO2012/129084. Furthermore, *in vivo* combination therapy data provided in the Examples of See WO2012/129084 strongly indicate that a combined therapeutic approach could be both additive and complementary.

In certain embodiments, compounds of the disclosure, such as, for example, (S)-Quinuclidin-3-yl (2-(2-(4-fluorophenyl)thiazol-4-yl)propan-2-yl)carbamate and Quinuclidin-3-yl (2-(4'-fluoro-[1,1'-biphenyl]-3-yl)propan-2-yl)carbamate may be used for reducing the level of GluCer and GluSph in the brain of a subject diagnosed with neuropathic Gaucher disease, either alone or in combination with ERT (e.g., glucocerebrosidase administration).

Dosage regimens for a small molecule therapy component of a combination therapy of the invention are generally determined by the skilled clinician and are expected to vary significantly depending on the particular storage disease being treated and the clinical status of the particular affected individual. The general principles for determining a dosage regimen for a given SMT of the invention for the treatment of any storage disease are well known to the skilled artisan. Guidance for dosage regimens can be obtained from any of the many well-known references in the art on this topic. Further guidance is available, inter alia, from a review of the specific references cited herein. In certain embodiments, such dosages may range from about 0.5 mg/kg to about 300 mg/kg, preferably from about 5 mg/kg to about 60 mg/kg (e.g., 5 mg/kg, 10 mg/kg, 15, mg/kg, 20 mg/kg, 25 mg/kg, 30 mg/kg, 35 mg/kg, 40 mg/kg, 45 mg/kg, 50 mg/kg, 55 mg/kg and 60 mg/kg) by intraperitoneal, oral or equivalent administration from one to five times daily. Such dosages may range from about 5 mg/kg to about 5 g/kg, preferably from about 10 mg/kg to about 1 g/kg by oral, intraperitoneal or equivalent administration from one to five times daily. In one embodiment, doses range from about 10 mg/day to about 500 mg/day (e.g., 10 mg/day, 20 mg/day, 30 mg/day, 40 mg/day, 50 mg/day, 60 mg/day, 70 mg/day, 80 mg/day, 90 mg/day, 100 mg/day, 110 mg/day, 120 mg/day, 130 mg/day, 140 mg/day, 150 mg/day, 160 mg/day, 170 mg/day, 180 mg/day, 190 mg/day, 200 mg/day, 210 mg/day, 220 mg/day, 230 mg/day, 240 mg/day, 250 mg/day, 260 mg/day, 270 mg/day, 280 mg/day, 290 mg/day, 300 mg/day). A particularly preferred oral dose range is from about 50 mg to about 100 mg, wherein the dose is administered twice daily. A particular oral dose range for a compound of the present invention is from about 5 mg/kg/day to about 600 mg/kg/day. In a particular oral dose range for a compound of the present invention is from about 1 mg/kg/day to about 120 mg/kg/day, e.g., 1 mg/kg/day , 5 mg/kg/day, 10 mg/kg/day, 15 mg/kg/day, 20 mg/kg/day, 25 mg/kg/day, 30 mg/kg/day, 35 mg/kg/day, 40 mg/kg/day , 45 mg/kg/day , 50 mg/kg/day , 55 mg/kg/day or 60 mg/kg/day, 65 mg/kg/day, 70 mg/kg/day, 75 mg/kg/day, 80 mg/kg/day, 85 mg/kg/day, 90 mg/kg/day, 95 mg/kg/day, 100 mg/kg/day, 105 mg/kg/day, 110 mg/kg/day , 115 mg/kg/day or 120 mg/kg/day.

In certain embodiments, the invention relates to combination therapies of SMT using compounds of the invention and ERT therapy for the treatment of lysosomal storage diseases. A partial list of known lysosomal storage diseases that can be treated in accordance with the disclosure is set forth in Table 1, including common disease name, material stored, and corresponding enzyme deficiency (adapted from Table 38-4 of Kolodny et al., 1998, Id.).

**TABLE 1**

| | **Lysosomal Storage Diseases** | |
|---|---|---|
| **Disease** | **Material Stored** | **Enzyme Deficiency** |
| **Sphingolipidoses** | | |
| Gaucher | Glucocerebroside, glucosyl sphingosine | Glucocerebrosidase |
| Niemann-Pick | Sphingomyelin | Sphingomyelinase |
| Niemann-Pick B | Sphingomyelin | Sphingomyelinase |
| Farber | Ceramide | Ceramidase |
| G_{M1}-gangliosidosis | G_{M1}-ganglioside, glycoprotein | G_{M1}-ganglioside-β-galactosidase |
| G_{M2}-gangliosidosis (Sandhoff) | G_{M2}-ganglioside, globoside | Hexosaminidase A and B |
| Tay-Sachs | G_{M2}-ganglioside | Hexosaminidase A |
| Krabbe | Galactosylceramide | β-Galactocerebrosidase |

| **Mucopolvsaccharidoses** | | |
|---|---|---|
| Hurler-Scheie (MPS I) | Dermatan sulfate, heparin Sulfate | α-L-iduronidase |
| Hunter (MPS II) | Dermatan sulfate, heparin sulfate | Iduronate sulfatase |

| Sanfilippo (MPS III) | | |
|---|---|---|
| Type A | Heparan sulfate | Heparan-N-sulfatase |
| Type B | Heparan sulfate | N-acetyl-α-glucosaminidase |
| Type C | Heparan sulfate | Acetyl CoA:α-glucosaminide acetyl-transferase |
| Type D | Heparan sulfate | N-acetyl-α-glucosamine-6-sulfatase |

| Marquio (MPS IV) | | |
|---|---|---|
| Type A | Keratan sulfate | Galactosamine-6-sulfatase |
| Type B | Keratan sulfate | β-galactosidase |
| Maroteaux-Lamy (MPS VI) | Dermatan sulfate | Galactosamine-4-sulfatase (aryl sulfatase B) |
| Sly (MPS VII) | Dermatan sulfate, heparan Sulfate | β-glucuronidase |
| Mucosulfatidosis | Sulfatides, mucopolysaccharides | Arylsulfatase A, B and C, other sulfatases |

| **Mucolipidoses** | | |
|---|---|---|
| Sialidoses | Sialyloligosaccharides, glycoproteins | α-neuraminidase |
| Mucolipidosis II | Sialyloligosaccharides, glycoproteins, glycolipids | High serum, low fibroblast enzymes; N-acetylglucosamine-1-phosphate transferase |
| Mucolipidosis III | Glycoproteins, glycolipids | Same as above |
| Mucolipidosis IV | Glycolipids, glycoproteins | Mcoln1 transm protein |

| **Other Diseases of Complex Carbohydrate Metabolism** | | |
|---|---|---|
| Fabry | Globotriaosylceramide(Gb3), α-galac lyso-Gb3 α-galactosidase A | |
| Schindler | O-linked glycopeptides | α-N-acetylgalactosaminidase |
| Pompe | Glycogen | α-glucosidase |
| Sialic acid storage disease | Free sialic acid | Unknown |
| Fucosidosis | Fucoglycolipids, fucosyloligosaccharides | α-fucosidase |
| Mannosidosis Aspartylglucosaminuria | Mannosyl oligosacchari des Aspartylglucosamine | α-mannosidase Aspartylglucosamine amidase |
| Wolman | Cholesteryl esters, Triglycerides | Acid lipase |

| **Neuronal Ceroid Lipofuscinoses (NCLs)*** | | |
|---|---|---|
| Infantile NCL | Granular osmophilic deposits, Saposins A and D thioesterase | Palmitoyl-protein e thioesterase (PPT1) |
| Late Infantile | Curvilinear profiles, ATP synthase subunit c | Tripeptidyl protease 1 (TPP 1) |
| Finnish variant | Fingerprint/Rectilinear profiles, ATP synthase subunit c | CLN5 |
| Variant | Fingerprint/Rectilinear profiles, ATP synthase subunit c | CLN6 |
| Juvenile | Fingerprint profile, ATP synthase subunit c | CLN3 |
| Adult | Variable | Unknown |
| Northern Epilepsy | Rectilinear profile, ATP synthase subunit c | CLN8 |
| Turkish variant | Fingerprint/Rectilinear profiles - constituents unknown | Unknown |

| **Lysosomal diseases of cholesterol transport and metabolism** | | |
|---|---|---|
| Niemann-Pick type C | Unesterified cholesterol | NPC1 or NPC2 |
| * Davidson et al., The Neuronal Ceroid Lipofuscinosis, Clinical Features and Molecular Basis of Disease. In Barranger JA and Cabrera-Salazar MA (Eds) Lysosomal Storage Disorders. 2007. pp. 371-388. Springer, New York, U.S.A. | | |

Any method known to the skilled artisan may be used to monitor disease status and the effectiveness of a combination therapy of the invention. Clinical monitors of disease status may include but are not limited to organ volume (e.g. liver, spleen), hemoglobin, erythrocyte count, hematocrit, thrombocytopenia, cachexia (wasting), and plasma chitinase levels (e.g. chitotriosidase). Chitotriosidase, an enzyme of the chitinase family, is known to be produced by macrophages in high levels in subjects with lysosomal storage diseases (see Guo et al., 1995, J. Inherit. Metab. Dis. 18, 717-722; den Tandt et al., 1996, J. Inherit. Metab. Dis. 19, 344-350; Dodelson de Kremer et al., 1997, Medicina (Buenos Aires) 57, 677-684; Czartoryska et al., 2000, Clin. Biochem. 33, 147-149; Czartoryska et al., 1998, Clin. Biochem. 31, 417-420; Mistry et al., 1997, Baillieres Clin. Haematol. 10, 817-838; Young et al., 1997, J. Inherit. Metab. Dis. 20, 595-602; Hollak et al., 1994, J. Clin. Invest. 93, 1288-1292). Chitotriosidase is preferably measured in conjunction with angiotensin converting enzyme and non tartrate resistant acid phosphatase to monitor response to treatment of Gaucher patients.

Methods and formulations for administering the combination therapies of the invention include all methods and formulations well known in the art (see, e.g., Remington's Pharmaceutical Sciences, 1980 and subsequent years, 16th ed. and subsequent editions, A. Oslo editor, Easton Pa.; Controlled Drug Delivery, 1987, 2nd rev., Joseph R. Robinson & Vincent H. L. Lee, eds., Marcel Dekker, ISBN: 0824775880; Encyclopedia of Controlled Drug Delivery, 1999, Edith Mathiowitz, John Wiley & Sons, ISBN: 0471148288; U.S. Pat. No. 6,066,626 and references cited therein; see also, references cited in sections below).

According to the invention, the following general approaches are provided for combination therapy in the treatment of lysosomal storage diseases. Each general approach involves combining enzyme replacement therapy with small molecule therapy in a manner consistent with optimizing clinical benefit while minimizing disadvantages associated with using each therapy alone.

In one embodiment of the invention, enzyme replacement therapy (alone or in combination with small molecule therapy) is administered to initiate treatment (i.e., to de-bulk the subject), and small molecule therapy is administered after the de-bulking phase to achieve and maintain a stable, long-term therapeutic effect without the need for frequent intravenous ERT injections. For example, enzyme replacement therapy may be administered intravenously (e.g. over a one to two hour period) once, on a weekly basis, once every two weeks, or once every two months, for several weeks or months, or longer (e.g., until an involved indicator organ such as spleen or liver shows a decrease in size). Moreover, the ERT phase of initial de-bulking treatment can be performed alone or in combination with a small molecule therapy. A small molecule therapeutic component is particularly preferred where the small molecule is compatible with oral administration, thus providing further relief from frequent intravenous intervention.

Alternating among ERT and SMT, or supplementing SMT with ERT as needed, provides a strategy for simultaneously taking advantage of the strengths and addressing the weaknesses associated with each therapy when used alone. An advantage of ERT, whether used for de-bulking and/or for more long-term care, is the much broader clinical experience available to inform the practitioner's decisions. Moreover, a subject can be effectively titrated with ERT during the de-bulking phase by, for example, monitoring biochemical metabolites in urine or other body samples, or by measuring affected organ volume. A disadvantage of ERT, however, is the frequency of the administration required, typically involving intravenous injection on a weekly or bi-weekly basis due to the constant re-accumulation of the substrate. The use of small molecule therapy to reduce the amount of or inhibit substrate accumulation in a patient can in turn reduce the administration frequency of ERT. For example, a bi-weekly enzyme replacement therapy dosing regimen can be offered an "ERT holiday" (e.g., using a SMT) so that frequent enzyme injections are not required therapy. Furthermore, treating a lysosomal storage disease with combination therapy can provide complementary therapeutic approaches. Indeed, a combination therapy of SMT and ERT can provide significant improvements over either therapeutic platform alone. See WO2012/129084. Combination therapy using SMT and ERT can be both additive and complementary. In one embodiment, ERT may be used as a de-bulking strategy (i.e., to initiate treatment), followed by or simultaneously supplemented with SMT using a compound of the present invention. In another embodiment, a patient is first treated with SMT using a compound of the present invention, followed by or simultaneously supplemented with ERT. In other embodiments, a SMT is used to inhibit or reduce further accumulation of substrate (or re-accumulation of substrate if used after debulking with ERT) in a patient with a lysosomal storage disease, and optionally provided ERT as needed to reduce any further substrate accumulation. In one embodiment, this invention provides a method of combination therapy for treatment of a subject diagnosed as having a lysosomal storage disease comprising alternating between administration of an enzyme replacement therapy and a small molecule therapy. In another embodiment, this invention provides a method of combination therapy for treatment of a subject diagnosed as having a lysosomal storage disease comprising simultaneously administering an enzyme replacement therapy and a small molecule therapy. In the various combination therapies of the invention, it will be understood that administering small molecule therapy may occur prior to, concurrently with, or after, administration of enzyme replacement therapy. Similarly, administering enzyme replacement therapy may occur prior to, concurrently with, or after, administration of small molecule therapy.

In any of the embodiments of the invention, the lysosomal storage disease is Fabry disease.

Further, the ERT provides an effective amount of at least one of the following enzymes; glucocerebrosidase, sphingomyelinase, ceramidase, G_{M1}-ganglioside-beta-galactosidase, hexosaminidase A, hexosaminidase B, beta-galactocerebrosidase, alpha-L-iduronidase, iduronate sulfatase, heparan-N-sulfatase, N-acetyl-alpha-glucosaminidase, acetyl CoA:alpha-glucosaminide acetyl-transferase, N-acetyl-alpha-glucosamine-6-sulfatase, galactosamine-6-sulfatase, beta-galactosidase, galactosamine-4-sulfatase (arylsulfatase B), beta-glucuronidase, arylsulfatase A, arylsulfatase C, alpha-neuraminidase, N-acetyl-glucosamine-1-phosphate transferase, alpha-galactosidase A, alpha-N-acetylgalactosaminidase, alpha-glucosidase, alpha-fucosidase, alpha-mannosidase, aspartylglucosamine amidase, acid lipase, palmitoyl-protein thioesterase (CLN-1), PPT1, TPP1, CLN3, CLN5, CLN6, CLN8, NPC1 or NPC2 .

In accordance with the invention, the SMT and/or ERT produce a diminution in at least one of the following stored materials; glucocerebroside, sphingomyelin, ceramide, G_{M1}-ganglioside, G_{M2}-ganglioside, globoside, galactosylceramide, dermatan sulfate, heparan sulfate, keratan sulfate, sulfatides, mucopolysaccharides, sialyloligosaccharides, glycoproteins, sialyloligosaccharides, glycolipids, globotriaosylceramide, O-linked glycopeptides, glycogen, free sialic acid, fucoglycolipids, fucosyloligosaccharides, mannosyloligosaccharides, aspartylglucosamine, cholesteryl esters, triglycerides, granular osmophilic deposits - Saposins A and D, ATP synthase subunit c, NPC1 or NPC2.

In certain embodiments of the invention, the small molecule therapy comprises administering to the subject an effective amount of (S)-Quinuclidin-3-yl (2-(2-(4-fluorophenyl)thiazol-4-yl)propan-2-yl)carbamate:

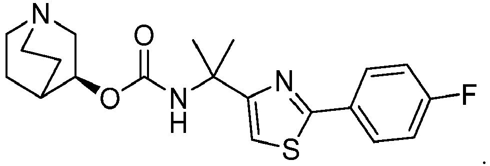

In other embodiments, the small molecule therapy comprises administering to the subject an effective amount of Quinuclidin-3-yl (2-(4'-fluoro-[1,1'-biphenyl]-3-yl)propan-2-yl)carbamate:

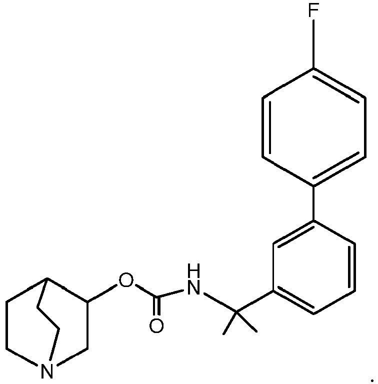

The small molecule therapy may include administering to a subject one or more compounds. In certain embodiments, at least one of the compounds is a compound of the present invention, such as (S)-Quinuclidin-3-yl (2-(2-(4-fluorophenyl)thiazol-4-yl)propan-2-yl)carbamate and/or Quinuclidin-3-yl (2-(4'-fluoro-[1,1'-biphenyl]-3-yl)propan-2-yl)carbamate.

Enzyme replacement therapy can provoke unwanted immune responses. Accordingly, immunosuppressant agents may be used together with an enzyme replacement therapy component of a combination therapy of the invention. Such agents may also be used with a small molecule therapy component, but the need for intervention here is generally less likely. Any immunosuppressant agent known to the skilled artisan may be employed together with a combination therapy of the invention. Such immunosuppressant agents include but are not limited to cyclosporine, FK506, rapamycin, CTLA4-Ig, and anti-TNF agents such as etanercept (see e.g. Moder, 2000, Ann. Allergy Asthma Immunol. 84, 280-284; Nevins, 2000, Curr. Opin. Pediatr. 12, 146-150; Kurlberg et al., 2000, Scand. J. Immunol. 51, 224-230; Ideguchi et al., 2000, Neuroscience 95, 217-226; Potteret al., 1999, Ann. N.Y. Acad. Sci. 875, 159-174; Slavik et al., 1999, Immunol. Res. 19, 1-24; Gaziev et al., 1999, Bone Marrow Transplant. 25, 689-696; Henry, 1999, Clin. Transplant. 13, 209-220; Gummert et al., 1999, J. Am. Soc. Nephrol. 10, 1366-1380; Qi et al., 2000, Transplantation 69, 1275-1283). The anti-IL2 receptor (.alpha.-subunit) antibody daclizumab (e.g. Zenapax.TM.), which has been demonstrated effective in transplant patients, can also be used as an immunosuppressant agent (see e.g. Wiseman et al., 1999, Drugs 58, 1029-1042; Beniaminovitz et al., 2000, N. Engl J. Med. 342, 613-619; Ponticelli et al., 1999, Drugs R. D. 1, 55-60; Berard et al., 1999, Pharmacotherapy 19, 1127-1137; Eckhoff et al., 2000, Transplantation 69, 1867-1872; Ekberg et al., 2000, Transpl. Int. 13, 151-159). Additional immunosuppressant agents include but are not limited to anti-CD2 (Branco et al., 1999, Transplantation 68, 1588-1596; Przepiorka et al., 1998, Blood 92, 4066-4071), anti-CD4 (Marinova-Mutafchieva et al., 2000, Arthritis Rheum. 43, 638-644; Fishwild et al., 1999, Clin. Immunol. 92, 138-152), and anti-CD40 ligand (Hong et al., 2000, Semin. Nephrol. 20, 108-125; Chirmule et al., 2000, J. Virol. 74, 3345-3352; Ito et al., 2000, J. Immunol. 164, 1230-1235).

Any combination of immunosuppressant agents known to the skilled artisan can be used together with a combination therapy of the invention. One immunosuppressant agent combination of particular utility is tacrolimus (FK506) plus sirolimus (rapamycin) plus daclizumab (anti-IL2 receptor .alpha.-subunit antibody). This combination is proven effective as an alternative to steroids and cyclosporine, and when specifically targeting the liver. Moreover, this combination has recently been shown to permit successful pancreatic islet cell transplants. See Denise Grady, The New York Times, Saturday, May 27, 2000, pages A1 and A11. See also A. M. Shapiro et al., Jul. 27, 2000, "Islet Transplantation In Seven Patients With Type 1 Diabetes Mellitus Using A Glucocorticoid-Free Immunosuppressive Regimen", N. Engl. J. Med. 343, 230-238; Ryan et al., 2001, Diabetes 50, 710-719. Plasmaphoresis by any method known in the art may also be used to remove or deplete antibodies that may develop against various components of a combination therapy.

Immune status indicators of use with the invention include but are not limited to antibodies and any of the cytokines known to the skilled artisan, e.g., the interleukins, CSFs and interferons (see generally, Leonard et al., 2000, J. Allergy Clin. Immunol. 105, 877-888; Oberholzer et al., 2000, Crit. Care Med. 28 (4 Suppl.), N3-N12; Rubinstein et al., 1998, Cytokine Growth Factor Rev. 9, 175-181). For example, antibodies specifically immunoreactive with the replacement enzyme can be monitored to determine immune status of the subject. Among the two dozen or so interleukins known, particularly preferred immune status indicators are IL-1.alpha., IL-2, IL-4, IL-8 and IL-10. Among the colony stimulating factors (CSFs), particularly preferred immune status indicators are G-CSF, GM-CSF and M-CSF. Among the interferons, one or more alpha, beta or gamma interferons are preferred as immune status indicators.

In the sections which follow, various components that may be used for eight specific lysosomal storage diseases are provided (i.e., Gaucher (including types 1, 2 and 3), Fabry, Niemann-Pick B, Hunter, Morquio, Maroteaux-Lamy, Pompe, and Hurler-Scheie). In subsequent sections, further enabling disclosure for enzyme replacement therapy and small molecule therapy components of a combination therapy of the invention are provided.

### Gaucher

As noted above, Gaucher's disease is caused by the deficiency of the enzyme glucocerebrosidase (beta-D-glucosyl-N-acylsphingosine glucohydrolase, EC 3.2.1.45) and accumulation of glucocerebroside (glucosylceramide). For an enzyme replacement therapy component of a combination therapy of the disclosure for the treatment of Gaucher's disease, a number of references are available which set forth satisfactory dosage regimens and other useful information relating to treatment (see Morales, 1996, Gaucher's Disease: A Review, The Annals of Pharmacotherapy 30, 381-388; Rosenthal et al., 1995, Enzyme Replacement Therapy for Gaucher Disease: Skeletal Responses to Macrophage-targeted Glucocerebrosidase, Pediatrics 96, 629-637; Barton et al., 1991, Replacement Therapy for Inherited Enzyme Deficiency--Macrophage-targeted Glucocerebrosidase for Gaucher's Disease, New England Journal of Medicine 324, 1464-1470; Grabowski et al., 1995, Enzyme Therapy in Type 1 Gaucher Disease: Comparative Efficacy of Mannose-terminated Glucocerebrosidase from Natural and Recombinant Sources, Annals of Internal Medicine 122, 33-39; Pastores et al., 1993, Enzyme Therapy in Gaucher Disease Type 1: Dosage Efficacy and Adverse Effects in 33 Patients treated for 6 to 24 Months, Blood 82, 408-416); and Weinreb et al., Am. J. Med.;113(2):112-9 (2002).

In one embodiment of the disclosure, an ERT dosage regimen of from 2.5 units per kilogram (U/kg) three times a week to 60 U/kg once every two weeks is provided, where the enzyme is administered by intravenous infusion over 1-2 hours. A unit of glucocerebrosidase is defined as the amount of enzyme that catalyzes the hydrolysis of one micromole of the synthetic substrate para-nitrophenyl-p-D-glucopyranoside per minute at 37 °C. In another embodiment of the disclosure, a dosage regimen of from 1 U/kg three times a week to 120 U/kg once every two weeks is provided. In yet another embodiment of the disclosure, a dosage regimen of from 0.25 U/kg daily or three times a week to 600 U/kg once every two to six weeks is provided.

Since 1991, alglucerase (Ceredase®) has been available from Genzyme Corporation. Alglucerase is a placentally-derived modified form of glucocerebrosidase. In 1994, imiglucerase (Cerezyme®) also became available from Genzyme Corporation. Imiglucerase is a modified form of glucocerebrosidase derived from expression of recombinant DNA in a mammalian cell culture system (Chinese hamster ovary cells). Imiglucerase is a monomeric glycoprotein of 497 amino acids containing four N-linked glycosylation sites. Imiglucerase has the advantages of a theoretically unlimited supply and a reduced chance of biological contaminants relative to placentally-derived aglucerase. These enzymes are modified at their glycosylation sites to expose mannose residues, a maneuver which improves lysosomal targeting via the mannose-6-phosphate receptor. Imiglucerase differs from placental glucocerebrosidase by one amino acid at position 495 where histidine is substituted for arginine. Several dosage regimens of these products are known to be effective (see Morales, 1996, Id.; Rosenthal et al., 1995, Id.; Barton et al., 1991, Id.; Grabowski et al., 1995, Id.; Pastores et al., 1993, Id.). For example, a dosage regimen of 60 U/kg once every two weeks is of clinical benefit in subjects with moderate to severe disease. The references cited above and the package inserts for these products should be consulted by the skilled practitioner for additional dosage regimen and administration information. See also U.S. Pat. Nos. 5,236,838 and 5,549,892 assigned to Genzyme Corporation.

As noted above, Gaucher Disease results from a deficiency of the lysosomal enzyme glucocerebrosidase (GC). In the most common phenotype of Gaucher disease (type 1), pathology is limited to the reticuloendothelial and skeletal systems and there are no neuropathic symptoms. See Barranger, Glucosylceramide lipidosis: Gaucher disease. In: Scriver CR BA, Sly WS, Valle D, editor. The Metabolic Basis of Inherited Disease. New York: McGraw-Hill. pp. 3635-3668 (2001). In neuropathic Gaucher disease (nGD), subdivided into type 2 and type 3 Gaucher disease, the deficiency of glucocerebrosidase (GC) causes glucosylceramide (GluCer; GL-1) and glucosylsphingosine (GluSph) to accumulate in the brain, leading to neurologic impairment. Type 2 Gaucher disease is characterized by early onset, rapid progression, extensive pathology in the viscera and central nervous system, and death usually by 2 years of age. Type 3 Gaucher disease, also known as subacute nGD, is an intermediate phenotype with varying age of onset and different degrees of severity and rates of progression. Goker-Alpan et al., The Journal of Pediatrics 143: 273-276 (2003). A recent development has produced the K14 lnl/lnl mouse model of type 2 Gaucher disease (hereinafter, the "K14 mouse"); this mouse model closely recapitulates the human disease showing ataxia, seizures, spasticity and a reduced median lifespan of only 14 days. Enquist et al., PNAS 104: 17483-17488 (2007).

As in patients with nGD, several mouse models of the disease have increased levels of GluCer and GluSph in the brain due to the deficiency in GC activity. Liu et al., PNAS 95: 2503-2508 (1998) and Nilsson, J. Neurochem 39: 709-718 (1982). The "K14" mice display a neuropathic phenotype that shares many pathologic features with type 2 Gaucher disease, such as neurodegeneration, astrogliosis, microglial proliferation, and increased levels of GluCer and GluSph in specific brain regions. Enquist et al. (2007).

Clinical management of patients affected by nGD poses a challenge for treating physicians both because of the severity of type 2 disease and the inability of the current therapies to cross the blood brain barrier (BBB). Current treatment of non-nGD relies on the intravenous delivery of recombinant human glucocerebrosidase (Imiglucerase; Cerezyme™) to replace the missing enzyme or the administration of glucosylceramide synthase inhibitors to attenuate substrate (GL-1) production. However, these drugs do not cross the blood brain barrier, and thus are not expected to provide therapeutic benefit for nGD patients. Current small molecule glucosylceramide synthase inhibitors in the clinic are not likely to address the neuropathic phenotypes of nGD. An evaluation of a compound of the present invention, Quinuclidin-3-yl (2-(4'-fluoro-[1,1'-biphenyl]-3-yl)propan-2-yl)carbamate (hereinafter, "Gz161"), in the K14 mouse model of type 2 Gaucher disease demonstrated that it could indeed reduce brain GluCer and GluSph. See Examples 122-125 of WO2012/129084. It also reduced brain neuropathology and extended the lifespan of this model. Moreover, a combined approach using both enzyme replacement and small molecule substrate reduction may represent a superior therapy for type 2 Gaucher disease.

### Fabry

As noted previously, Fabry's disease is caused by the deficiency of the lysosomal enzyme alpha-galactosidase A. The enzymatic defect leads to systemic deposition of glycosphingolipids having terminal alpha-galactosyl moieties, predominantly globotriaosylceramide (GL3 or Gb3) and, to a lesser extent, galabiosylceramide and blood group B glycosphingolipids.

Several assays are available to monitor disease progression and to determine when to switch from one treatment modality to another. In one embodiment, an assay to determine the specific activity of alpha-galactosidase A in a tissue sample may be used. In another embodiment, an assay to determine the accumulation of Gb3 may be used. In another embodiment, the practitioner may assay for deposition of glycosphingolipid substrates in body fluids and in lysosomes of vascular endothelial, perithelial and smooth muscle cells of blood vessels. Other clinical manifestations which may be useful indicators of disease management include proteinuria, or other signs of renal impairment such as red cells or lipid globules in the urine, and elevated erythrocyte sedimentation rate. One can also monitor anemia, decreased serum iron concentration, high concentration of beta-thromboglobulin, and elevated reticulocyte counts or platelet aggregation. Indeed, any approach for monitoring disease progression which is known to the skilled artisan may be used (See generally Desnick RJ et al., 1995, .alpha.-Galactosidase A Deficiency: Fabry Disease, In: The Metabolic and Molecular Bases of Inherited Disease, Scriver et al., eds., McGraw-Hill, N.Y., 7.sup.th ed., pages 2741-2784). A preferred surrogate marker is pain for monitoring Fabry disease management. Other preferred methods include the measurement of total clearance of the enzyme and/or substrate from a bodily fluid or biopsy specimen. A preferred dosage regimen for enzyme replacement therapy in Fabry disease is 1-10 mg/kg i.v. every other day. A dosage regimen from 0.1 to 100 mg/kg i.v. at a frequency of from every other day to once weekly or every two weeks can be used.

### Niemann-Pick B

As previously noted, Niemann-Pick B disease is caused by reduced activity of the lysosomal enzyme acid sphingomyelinase and accumulation of membrane lipid, primarily sphingomyelin. An effective dosage of replacement acid sphingomyelinase to be delivered may range from about 0.01 mg/kg to about 10 mg/kg body weight at a frequency of from every other day to weekly, once every two weeks, or once every two months. In other embodiments of the disclosure an effective dosage may range from about 0.03 mg/kg to about 1 mg/kg; from about 0.03 mg/kg to about 0.1 mg/kg; and/or from about 0.3 mg/kg to about 0.6 mg/kg. In a particular embodiment of the disclosure, a patient is administering acid sphingomyelinase in an escalating dose regimen at the following sequential doses: 0.1 mg/kg; 0.3 mg/kg; 0.6 mg/kg; and 1.0 mg/kg, wherein each dose of acid sphingomyelinase is administered at least twice, and each dose is administered at two week intervals, and wherein the patient is monitored for toxic side effects before elevating the dose to the next level (See U.S. Patent Application Publication No. 2011/0052559.

### Hurler-Scheie (MPS I)

Hurler, Scheie, and Hurler-Scheie disease, also known as MPS I, are caused by inactivation of alpha-iduronidase and accumulation of dermatan sulfate and heparan sulfate. Several assays are available to monitor MPS I disease progression. For example, alpha-iduronidase enzyme activity can be monitored in tissue biopsy specimens or cultured cells obtained from peripheral blood. In addition, a convenient measure of disease progression in MPS I and other mucopolysaccharidoses is the urinary excretion of the glycosaminoglycans dermatan sulfate and heparan sulfate (see Neufeld et al., 1995, Id.). In a particular embodiment of the disclosure, alpha-iduronidase enzyme is administered once weekly as an intravenous infusion at a dosage of 0.58 mg/kg of body weight.

### Hunter (MPS II)

Hunter's disease (a.k.a. MPS II) is caused by inactivation of iduronate sulfatase and accumulation of dermatan sulfate and heparan sulfate. Hunter's disease presents clinically in severe and mild forms. A dosage regimen of therapeutic enzyme from 1.5 mg/kg every two weeks to 50 mg/kg every week is preferred.

### Morquio (MPS IV)

Morquio's syndrome (a.k.a. MPS IV) results from accumulation of keratan sulfate due to inactivation of either of two enzymes. In MPS IVA the inactivated enzyme is galactosamine-6-sulfatase and in MPS IVB the inactivated enzyme is beta-galactosidase. A dosage regimen of therapeutic enzyme from 1.5 mg/kg every two weeks to 50 mg/kg every week is preferred.

### Maroteaux-Lamy (MPS VI)

Maroteaux-Lamy syndrome (a.k.a. MPS VI) is caused by inactivation of alactosamine-4-sulfatase (arylsulfatase B) and accumulation of dermatan sulfate. A dosage regimen of from 1.5 mg/kg every two weeks to 50 mg/kg every week is a preferred range of effective therapeutic enzyme provided by ERT. Optimally, the osage employed is less than or equal to 10 mg/kg per week. A preferred surrogate marker for MPS VI disease progression is roteoglycan levels.

### Pompe

Pompe's disease is caused by inactivation of the acid alpha-glucosidase enzyme and accumulation of glycogen. The acid alpha-glucosidase gene resides on human chromosome 17 and is designated GAA. H. G. Hers first proposed the concept of inborn lysosomal disease based on his studies of this disease, which he referred to as type II glycogen storage disease (GSD II) and which is now also termed acid maltase deficiency (AMD) (see Hers, 1965, Gastroenterology 48, 625). In a particular embodiment of the disclosure, GAA is administered every 2 weeks as an intravenous infusion at a dosage of 20 mg/kg body weight.

Several assays are available to monitor Pompe disease progression. Any assay known to the skilled artisan may be used. For example, one can assay for intra-lysosomal accumulation of glycogen granules, particularly in myocardium, liver and skeletal muscle fibers obtained from biopsy. Alpha-glucosidase enzyme activity can also be monitored in biopsy specimens or cultured cells obtained from peripheral blood. Serum elevation of creatine kinase (CK) can be monitored as an indication of disease progression. Serum CK can be elevated up to ten-fold in infantile-onset patients and is usually elevated to a lesser degree in adult-onset patients. See Hirschhorn R, 1995, Glycogen Storage Disease Type II: Acid alpha-glucosidase (Acid Maltase) Deficiency, In: The Metabolic and Molecular Bases of Inherited Disease, Scriver et al., eds., McGraw-Hill, N.Y., 7.sup.th ed., pages 2443-2464.

### Enzyme Replacement Therapy

The following sections set forth specific disclosure and alternative embodiments available for the enzyme replacement therapy component of a combination therapy of the invention. Generally, dosage regimens for an enzyme replacement therapy component of a combination therapy of the invention are generally determined by the skilled clinician. Several examples of dosage regimens for the treatment of Gaucher's disease with glucocerebrosidase are provided above. The general principles for determining a dosage regimen for any given ERT component of a combination therapy of the invention for the treatment of any LSD will be apparent to the skilled artisan from publically available information, such as, for example, a review of the specific references cited in the sections for each specific LSD. An ERT may be administered to a patient by intravenous infusion. Intracerebroventricular and/or intrathecal infusion may be used (e.g., in addition to intravenous infusion) to administer ERT to a patient diagnosed with a lysosomal storage disease having CNS manifestations.

Any method known in the art may be used for the manufacture of the enzymes to be used in an enzyme replacement therapy component of a combination therapy of the invention. Many such methods are known and include but are not limited to the Gene Activation technology developed by Shire plc (see U.S. Pat. Nos. 5,968,502 and 5,272,071).

### Small Molecule Therapy

The following section also sets forth specific disclosures and alternative embodiments available for the small molecule therapy component of a combination therapy of the invention. Dosage regimens for a small molecule therapy component of a combination therapy of the invention are generally determined by the skilled clinician and are expected to vary significantly depending on the particular storage disease being treated and the clinical status of the particular affected individual. The general principles for determining a dosage regimen for a given SMT component of any combination therapy of the invention for the treatment of any storage disease are well known to the skilled artisan. Guidance for dosage regimens can be obtained from any of the many well-known references in the art on this topic. Further guidance is available, inter alia, from a review of the specific references cited herein.

Generally, compounds of the present invention, such as, for example, (S)-Quinuclidin-3-yl (2-(2-(4-fluorophenyl)thiazol-4-yl)propan-2-yl)carbamate and Quinuclidin-3-yl (2-(4'-fluoro-[1,1'-biphenyl]-3-yl)propan-2-yl)carbamate may be used in the combination therapies of the disclosure for treatment of virtually any storage disease resulting from a lesion in the glycosphingolipid pathway (e.g. Gaucher, Fabry, G_{M1}-gangliosidosis and G_{M2}-gangliosidoses (e.g., GM2 Activator Deficiency, Tay-Sachs and Sandhoff)). Likewise, aminoglycosides (e.g. gentamicin, G418) may be used in the combination therapies of the disclosure for any storage disease individual having a premature stop-codon mutation (i.e., nonsense mutation). Such mutations are particularly prevalent in Hurler syndrome. A small molecule therapy component of a combination therapy of the disclosure is particularly preferred where there is a central nervous system manifestation to the storage disease being treated (e.g., Sandhoff, Tay-Sachs, Niemann-Pick Type A, and Gaucher types 2 and 3), since small molecules can generally cross the blood-brain barrier with ease when compared to other therapies.

Preferred dosages of substrate inhibitors used in a combination therapy of the invention are easily determined by the skilled artisan. In certain embodiments, such dosages may range from about 0.5 mg/kg to about 300 mg/kg, preferably from about 5 mg/kg to about 60 mg/kg (e.g., 5 mg/kg, 10 mg/kg, 15, mg/kg, 20 mg/kg, 25 mg/kg, 30 mg/kg, 35 mg/kg, 40 mg/kg, 45 mg/kg, 50 mg/kg, 55 mg/kg and 60 mg/kg) by intraperitoneal, oral or equivalent administration from one to five times daily. Such dosages may range from about 5 mg/kg to about 5 g/kg, preferably from about 10 mg/kg to about 1 g/kg by oral, intraperitoneal or equivalent administration from one to five times daily. In one embodiment, doses range from about 10 mg/day to about 500 mg/day (e.g., 10 mg/day, 20 mg/day, 30 mg/day, 40 mg/day, 50 mg/day, 60 mg/day, 70 mg/day, 80 mg/day, 90 mg/day, 100 mg/day, 110 mg/day, 120 mg/day, 130 mg/day, 140 mg/day, 150 mg/day, 160 mg/day, 170 mg/day, 180 mg/day, 190 mg/day, 200 mg/day, 210 mg/day, 220 mg/day, 230 mg/day, 240 mg/day, 250 mg/day, 260 mg/day, 270 mg/day, 280 mg/day, 290 mg/day, 300 mg/day). A particularly preferred oral dose range is from about 50 mg to about 100 mg, wherein the dose is administered twice daily. A particular oral dose range for a compound of the present invention is from about 5 mg/kg/day to about 600 mg/kg/day. In a particular oral dose range for a compound of the present invention is from about 1 mg/kg/day to about 100 mg/kg/day, e.g., 1 mg/kg/day , 5 mg/kg/day, 10 mg/kg/day, 15 mg/kg/day, 20 mg/kg/day, 25 mg/kg/day, 30 mg/kg/day, 35 mg/kg/day, 40 mg/kg/day , 45 mg/kg/day , 50 mg/kg/day , 55 mg/kg/day or 60 mg/kg/day, 65 mg/kg/day, 70 mg/kg/day, 75 mg/kg/day, 80 mg/kg/day, 85 mg/kg/day, 90 mg/kg/day, 95 mg/kg/day or 100 mg/kg/day.

A rotating combination of therapeutic platforms (i.e., enzyme replacement and small molecule therapy) is preferred. However, subjects may also be treated by overlapping both approaches as needed, as determined by the skilled clinician. Examples of treatment schedules may include but are not limited to: (1) SMT followed by ERT; (2) ERT followed by SMT; and (3) ERT and SMT provided at about the same time. As noted previously, temporal overlap of therapeutic platforms may also be performed, as needed, depending on the clinical course of a given storage disease in a given subject.

Treatment intervals for various combination therapies can vary widely and may generally be different among different storage diseases and different individuals depending on how aggressively storage products are accumulated. For example, Fabry storage product accumulation may be slow compared to rapid storage product accumulation in Pompe. Titration of a particular storage disease in a particular individual is carried out by the skilled artisan by monitoring the clinical signs of disease progression and treatment success.

The various macromolecules that accumulate in lysosomal storage diseases are not uniformly distributed, but instead are deposited in certain preferred anatomic sites for each disease. However, an exogenously supplied enzyme is generally taken up by cells of the reticuloendothelial system and sorted to the lysosomal compartment where it acts to hydrolyze the accumulated substrate. Moreover, cellular uptake of therapeutic enzyme can be augmented by certain maneuvers to increase lysosomal targeting (see e.g. U.S. Pat. No. 5,549,892 by Friedman et al., assigned to Genzyme Corporation, which describes recombinant glucocerebrosidase having improved pharmacokinetics by virtue of remodeled oligosaccharide side chains recognized by cell surface mannose receptors which are endocytosed and transported to lysosomes).

Some treatment modalities target some affected organs better than others. In Fabry, for example, if ERT does not reach the kidney or the heart well enough for a satisfactory clinical outcome, SMT can be used to reduce the substrate levels in those tissues. It has been shown that SMT can effectively reduce Gb3 levels (i.e., the substrate accumulated in Fabry patients) in the urine of a Fabry mouse model to a greater extent than ERT. See WO2012/129084, which is hereby incorporated by reference in its entirety. The kidneys are believed to be the major source of urine Gb3. In contrast, ERT effectively reduced the Gb3 levels in the plasma to a greater extent than SMT. See WO2012/129084. Therefore, a combination therapy of ERT and SMT provides a complementary therapeutic strategy that takes advantage of the strengths and addresses the weaknesses associated with each therapy employed alone. SMT is able to cross the BBB, providing a powerful approach, when combined with ERT, for treating LSDs having CNS manifestations, such as Niemann Pick Type A and Neuropathic Gaucher disease (nGD). Moreover, substrate reduction by SMT combined with enzyme replacement address the storage problem at separate and distinct intervention points which may enhance clinical outcome.

It will be understood that reference to simultaneous or concurrent administration of two or more therapies does not require that they be administered at the same time, just that they be acting in the subject at the same time.

### EXAMPLES

It should be appreciated that the invention should not be construed to be limited to the examples that are now described.

### Example 1: Generation of FD iPSC

To generate induced pluripotent stem cell (iPSC) lines from Fabry disease (FD) patients, skin fibroblasts from two Caucasian symptomatic boys affected by FD were used. Both patients had no detectable α-galactosidase A (a-Gal A) activity. One donor, 10 year old, was hemizygous for a single nucleotide change in GLA exon 3 gene (G485A). The second donor, 17 year old, was hemizygous for a single nucleotide change in GLA exon 5 gene (C658T). Each of the mutations introduces a stop codon (W162X and R220X) and are known to be associated with the classical severe presentation of FD. See Altarescu GM. et al., Clin. Genet. 60:46-51 (2001); Germain DP. et al., Mol. Med. 8:306-12 (2002); Meaney C. et al., Hum. Mol. Genet. 3:1019-20 (1994); and Maki N. et al., Clin. Nephrol. 61:185-90 (2004).

A single polycistronic cassette encoding the four reprogramming factors, Oct4, Sox2, Klf4 and c-Myc, in a lentivirus, was used to generate more than 40 iPSC clones from both samples. The iPSC cells were characterized in order to check the pluripotent status and the extinction of the expression of reprogramming factors (data not shown). Globotriaosylceramide (GL-3) is required in this type of pluripotent cell. However, GL-3 accumulation was not detected in Fabry iPSC maintained in exponential growth phase even after several months in culture (immunohistological, electron microscopy, and mass spectrometry-based analyses data not shown). Although these cells produce GL-3 and are unable to degrade it (a-Gal A deficiency) while they are rapidly dividing, GL-3 dilution may occur during successive divisions and compensate for the GL-3 synthesis, thereby protecting the cells against GL-3 accumulation.

### Example 2: Ability to generate Fabry iPSC-derived cardiomyocytes

A protocol of cardiac differentiation with sequential addition of cytokine cocktails in hypoxic conditions was used to generate spontaneously beating embryoid bodies (EBs) containing an enriched population of cardiomyocytes which appear 10-16 days after the start of differentiation. All of the tested iPSC (17 lines) were able to generate beating EBs. Three of the iPSCs from the two patients were selected for further analysis of the phenotype of the Fabry iPSC-derived cardiomyocytes: G485A-10, G485A-56 and C658T-4. The phenotype was compared to that of wild type (WT) iPS-derived cardiomyocytes generated under the same conditions in the laboratory.

An immunohistological approach was used to characterize and quantify the cells in the beating EBs. One month after the start of the differentiation, EBs were picked and mouse fibroblasts (MEF) were added in order to have well isolated EBs in the paraffin block and to facilitate handling of the samples during inclusion, sectioning and mounting on glass slide. Antibodies directed against several cardiac contractile proteins, such as actin, myosins, troponins or α-actinin, were used to identify the cardiomyocytes contained in the beating EBs. As with the WT EBs, more than 90% of the cells express high levels of different cardiac contractile proteins (Figure 1A). These results indicate that the "Fabry origin" of the cells do not affect the capability of the Fabry iPSCs to effectively generate cardiomyocytes.

### Example 3: Fabry phenotype

One month old beating EBs were dissociated and plated on culture dishes to generate small two-dimensional clusters of cells or single cells layers. Four to seven days after plating, the cells were analyzed to study the organisation of the contractile fibers in more detail and the potential accumulation of GL-3.

In WT iPSC-derived cardiomyocytes, all the tested contractile proteins are present and functionally organized. The contractile fibers are oriented in the long axis and takes up the entire volume of the cells (Figure 1B). In Fabry iPSC-derived cardiomyocytes, the contractile proteins are also present and functionally organized into fibers, but these fibers are less numerous and, for a significant part of the cells, are localized to the periphery at the vicinity to the plasma membrane (Figure 1B). This result suggests that part of the contractile fibers were not able to get organized in the Fabry cells during cardiac differentiation or disappeared when the Fabry phenotype progressed and the remaining fibers were prevented from occupying the center of the cell. This phenotype is very similar to that already described in FD heart biopsies where ultrastructural electron microscopic examinations revealed frequent displacement of cardiac myofibrils to the periphery of the cell due to cytoplasmic inclusions and focal areas of myofibrillar lysis and myofilament degradation with troponin I degradation products. Chimenti C. et al., Am. J. Pathol. 172:1482-1490 (2008).

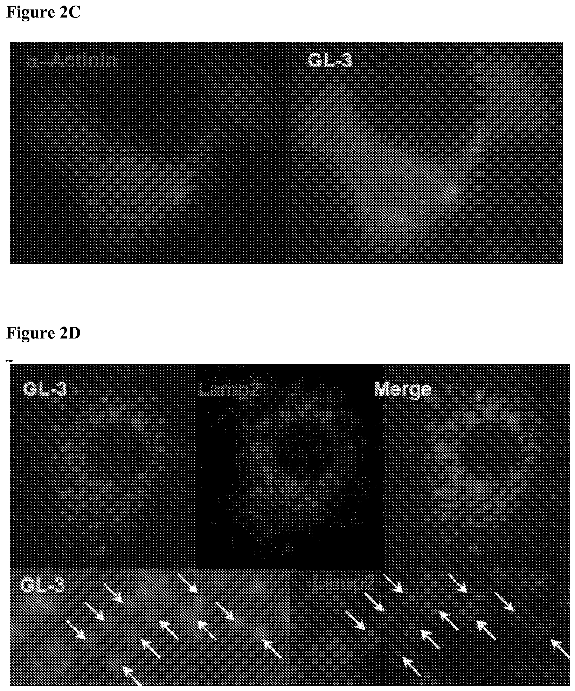

Immunocytological (ICC) study with an antibody directed against GL-3 indicated that there was an accumulation of sphingolipid in the FD cells (Figure 2A). GL-3 appeared to be more elevated in cells having observable vacuoles during bright-field microscopic examination (Figures 2B). The signal generated by the antibody consisted of a multitude of puncta scattered in the cytoplasm. Co-expression of cardiac fiber contractile protein and GL-3 was verified and demonstrated that the FD iPSC-derived cardiomyocytes have accumulated GL-3 one month after the start of the differentiation protocol (Figure 2C).

An ICC approach was also used to demonstrate that accumulated GL-3 is localized in the lysosomal compartments, as in FD patient cells. Antibodies directed to GL-3 and lysosome-associated membrane protein 2 (LAMP2/CD107b), a glycoprotein marker of the lysosomes membranes, were used. Fluorescence signals generated by the two antibodies gave punctate staining with a higher density near the nuclei, and with a decreased density near the cytoplasmic membrane. When merged, the two signals overlapped and demonstrated that GL-3 accumulation and storage occurred in lysosomal structures (Figure 2D).

This result was confirmed by electron microscopic characterisation of the FD cardiomyocytes. EBs derived from Fabry and WT iPSCs contained cells with variable degree of cardiomyocyte differentiation as evidenced by the following features: myofibril (1-2 µm wide) organized into sarcomeres with prominent Z-bands; abundant glycogen-rich sarcoplasm; and many mitochondria and intercellular junctions, including desmosomes and gap-junctions (intercalated disk-like structures). Cardiomyocytes in Fabry EBs contained lysosomal substrate storage inclusions (Figure 3). These 700-800 nm diameter inclusions are similar to the lamellated or onion-shaped structures, called zebra bodies, typical of GL-3 accumulation in Fabry patient cells. Elleder et al., Virchows Arch. A Pathol. Anat. Histopathol. 417:449-455 (1990).

In addition, the EBs that were of 16, 23, 30 and 45 days old EBs from the start of the cardiomyocyte differentiation were analyzed for their GL-3 content by mass spectrometry. GL-3 content was determined by quantitating nine distinct GL-3 isoforms and normalizing total GL-3 levels to total phosphatidylcholine (PC) for each replicate. Because PC is a major cell membrane component, its amount should not vary greatly in living cells. When these values were averaged across all of the clones at each time point, an accumulation of GL-3 was observed during the time course (Figure 4). By the second week of differentiation, the amount of GL-3 nearly doubled. By the third week, Fabry EBs had a 5.5 fold increase of GL-3 as compared to the wild type control. At day 30, they had an approximately 11 to 12 fold increase of GL-3 content. Two weeks later (day 45) the amount of GL-3 was not significantly different. Because the cardiomyocytes stop dividing once they begin to beat, these results indicate that GL-3 accumulated rapidly when the cells no longer divide and dilute the sphingolipid present in the daughter cells. Globotriaosylsphingosine (lyso-GL-3) was not observed (data not shown). Overall, the mass spectrometry-based analysis supports the ICC results and show that GL-3 accumulates in Fabry EBs.

### Example 4: Substrate reduction therapy

GCS452 [(S)-quinuclidin-3-yl (2-(2-(4-fluorophenyl)thiazol-4-yl)propan-2-yl) carbamate], a selective glucosylceramide synthase inhibitor, was tested to evaluate its potential to prevent GL-3 accumulation in Fabry iPSC-derived cardiomyocytes. EBs were treated eighteen days after induction of cardiac differentiation. At this time point, accumulation was low in the FD cardiomyocytes (Figure 4). The FD and WT cardiomyocytes were treated with 1µM GCS452 for 15 days. While untreated FD cardiomyocytes showed a 12 to 15-fold increase in GL-3 compared to WT, GL-3 levels in treated FD cells were close to the physiological levels of WT untreated cells. These results suggest that GCS452 can prevent additional GL-3 accumulation and could serve to ameliorate the abundant levels of this sphingolipid in the FD cardiomyocytes (Figure 5A).

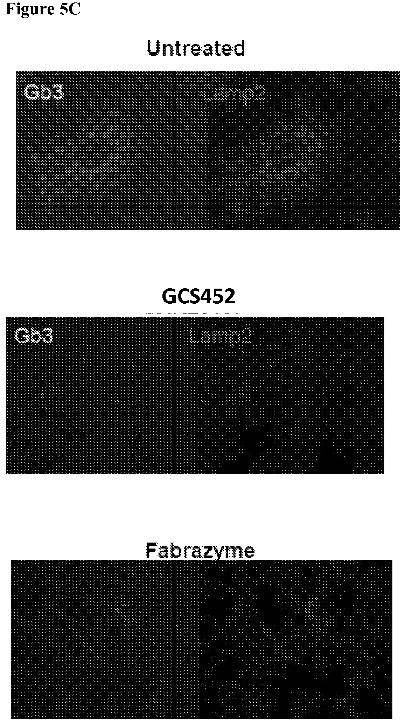

It was next determined whether the compound could reduce levels of accumulated GL-3 when significant GL-3 accumulation is present at the start of treatment (Figure 4). Twenty eight post-start of differentiation, beating EBs were treated with 1µM of GCS452 for 15 days. The EBs were dissociated and plated at the end of the treatment, and ICC analysis was performed. A significant reduction of GL-3 labeling was observed in the treated cells, up to the virtual disappearance of the punctate signals characteristic of sphingolipid accumulation (Figure 5C). Interestingly, a concomitant decrease in Lamp2 signal intensity was observed, suggesting that the elevated level of Lamp2 in untreated FD cardiomyocytes is due to GL-3 accumulation and that most of the lysosomes contained predominantly GL-3. GL-3 reduction in treated Fabry EBs was confirmed by mass spectrometry. While there was a 13 to 18 fold increase of GL-3 in untreated FD cardiomyocytes compared to WT, GL-3 levels increased by 4.5 to 5.5 fold in treated FD cardiomyocytes after 15 days of treatment with GCS452. This corresponds to a 2.3 to 2.8 fold reduction in accumulated GL-3 as compared to GL-3 levels at the start point (T0) of treatment (Figure 5B).

Electron microscopic examination of EBs revealed rare to relatively few storage inclusions in treated Fabry EBs as compared to untreated EBs at two different time points (18 and 28 days post-start of differentiation). Wild type EBs-treated or untreated did not show substrate storage inclusions (data not show).

In parallel, it was investigated whether agalsidase beta could clear accumulated GL-3 in one month old Fabry cardiomyocytes. EBs were treated with 0.3µg/mL of agalsidase beta for 5 days and GL-3 content was analyzed as described for GCS452. GL-3 vacuoles were not detected in treated Fabry cardiomyocytes using ICC (Figure 5C). Complete clearance was confirmed by mass spectrometry. Treated Fabry cardiomyocytes had a 6.4 to 18 fold reduction in GL-3 levels as compared to untreated Fabry cells, which was similar to the level in wild type untreated cardiomyocytes (Figure 5B).

### Example 5: Electrophysiology of Fabry derived cardiomyocytes

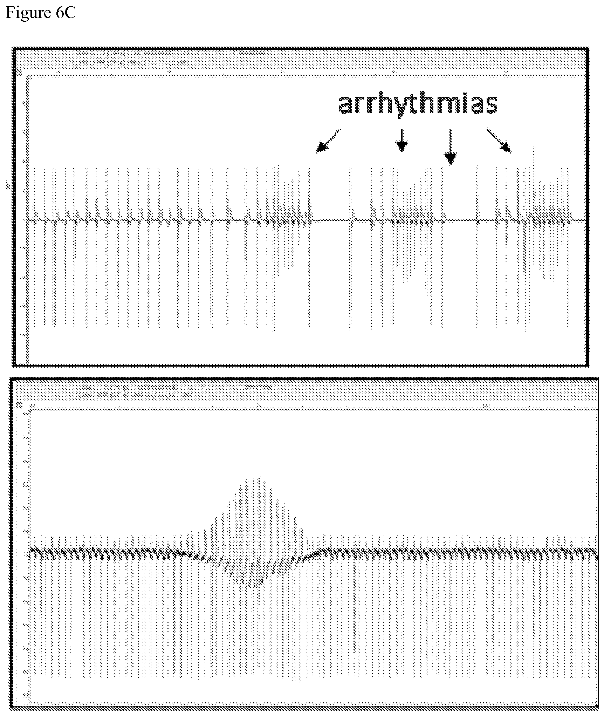

One month old iPSC-derived cardiomyocytes were platted on the electrodes of a MicroElectrode Array (MEA; Figure 6A) to investigate the electrical signal associated with the contractile activity of the cells. In wild type iPSC-derived cardiomyocytes, the electric signal is stable and reproducible over time (Figure 6B). Altered electrophysiology with high frequency, arrthythmias or sporadic modification of the intensity of the signal without modification of the rhythm was observed in Fabry cells (Figure 6C).

The electrophysiology of Fabry cardiomyocytes after treatment with agalsidase beta are observed to determine whether cardiomyocytes can recover wild type functional activity after GL-3 clearance.

### Example 6: Lower doses of agalsidase beta and GCS452

18 and 30 days old cardiomyocytes are treated with 2 doses of GCS452 (0.1µM and 1µM) for 15 days. 30 day old cardiomyocytes are treated with 2 doses of agalsidase beta (0.3µg/ml and 3µg/ml agalsidase beta) for 5 days. Mass spectrometry is used to evaluate the GL-3 contained in the cells.

### Example 7: GL-3 clearance after 6 weeks treatment with GCS452

Twenty eight old Fabry iPSC-derived cardiomyocytes are treated with 1µM GCS452 for 6 weeks. Mass spectrometry is used to evaluate the GL-3 contained in the cells.

### Results

Alpha-galactosidase A deficiency in Fabry patients result in the progressive accumulation of cellular GL-3 in a variety of cell types, including vascular endothelial cells and cardiomyocytes in the heart. See Aerts et al., PNAS USA 105:2812-2817 (2008). Although ERT with agalsidase beta effectively clears microvasculature deposits of GL-3 in the heart, it has no effect on cardiomyocyte GL-3 accumulation. See Thurberg B.L. et al, Circulation 119:2561-2567 (2009). As GL-3 accumulation is not observed in cardiomyocytes in the Fabry mouse, it is desirable to develop a relevant human *in vitro* system for studying cardiomyocyte pathology in FD that could be used to study alternative therapeutic strategies for GL-3 accumulation. See Ohshima T. et al., PNAS 96:2540-2544 (1997).

Several protocols have been described to differentiate human embryonic stem cells (ESC) into beating cardiomyocytes. However, such protocols utilize normal nonpathogenic ESC's that are not suitable for studying disease specific alterations associated with conditions such as FD. In 2007, Takahashi K. et al., Cell 131:861-872 (2007) reported the generation of human ESC-like cells, called induced pluripotent stem cells (iPSC), by reprogramming terminally differentiated cells. As a result of this finding, iPSC lines can be generated from patients and with appropriate protocols can be used to generate differentiated cells displaying disease specific phenotypes. Using this approach, several iPSC lines were generated using fibroblasts from two symptomatic patients with FD carrying different inactivating mutations in the GLA gene known to be associated with the classical phenotype of the disease (See Altarescu GM. et al., Clin. Genet. 60:46-51 (2001); Germain DP. et al., Mol. Med. 8:306-12 (2002); Meaney C. et al., Hum. Mol. Genet. 3:1019-20 (1994); and Maki N. et al., Clin. Nephrol. 61:185-90 (2004)). The ability to generate beating cardiomyocytes from control and Fabry derived iPSC lines was similar with more than 90% of the cells expressing cardiac contractile fiber proteins. Thus it appears that GLA inactivation does not significantly affect either the ability to reprogram fibroblasts into iPSCs or the ability to differentiate these cells into cardiomyocytes.

GL-3 accumulation in iPSC-derived cardiomyocytes was evaluated using three complementary methods: immunohistochemistry with an antiCD77/GL-3 antibodies, quantitative LC/MS and electron microscopy. Based on quantitative assessment by LC/MS, cellular GL-3 levels increase as the iPSCs differentiate from pluripotent cells to non dividing cardiomyocytes. At the end of differentiation cellular GL-3 levels in Fabry derived cardiomyocytes was greater than 10-fold higher than that observed in control cultures. Increased GL-3 in FD iPSC-derived cardiomyocytes was confirmed by immunohistochemistry with an anti-CD77/GL-3 antibody that revealed punctate staining within the cytoplasm that colocalized with a lysosomal marker, LAMP2. Ultrastructural examination by electron microscopy confirmed structural features typical of mature cardiomyocytes and demonstrated the presence of electron dense storage inclusions (zebra bodies) in the FD iPSC derived cultures.

Despite the accumulation of GL-3, FD iPSC-derived cells expressed several classical markers of mature cardiomyocytes, including the contractile protein α-actinin. However, unlike control cells where contractile fibers run across the long axis in an organized fashion, the fibers were generally less numerous in one month old FD iPSC-derived cardiomyocytes and often found at the periphery of the cell close to the plasma membrane. This disorganization of contractile fibers may be due to the presence of enlarged lipid laden lysosomes in FD cardiomyocytes that displace fibers from the center of the cell.

The phenotypic changes observed in diseased cardiomyocytes were reproducible and similar in cells generated from both Fabry patient iPSC lines. As these cells harbour different inactivating mutations in the GLA gene, the phenotype likely results from a lack of α-galactosidase enzymatic activity in differentiated cardiomyocytes. Importantly, the lysosomal accumulation of GL-3 and disorganization of contractile fibers observed in FD iPSC derived cardiomyocytes closely resembles the cardiac myocyte pathology described in biopsies from Fabry patients. See Chimenti C. et al., Am. J. Pathol. 172:1482-1490 (2008).

Given the strong phenotypic similarities to the human disease, Fabry iPSC-derived cardiomyocytes were used to evaluate the effects of ERT and SRT on cellular GL-3 accumulation. The addition of agalsidase beta to iPSC-derived cardiomyocytes resulted in clearance of GL-3, indicating that the recombinant enzyme is able to access and clear lysosomal substrate in cultured cells. This finding suggests that the apparent inability of agalsidase beta to clear cardiomyocyte GL-3 in Fabry patients is not due to an inherent defect in enzymatic activity in cardiomyocytes. However, levels of GL-3 in cultured iPSC derived cardiomyocytes could be less than that observed in cardiac tissue from Fabry patients, given the chronic nature of the disease and the lack of cardiomyocyte cell turnover *in vivo.* Modest reductions in left ventricular mass have been reported in agalsidase beta treated Fabry patients suggesting a positive impact of enzyme replacement on cardiomyocyte GL-3 storage. See Weidemann F. et al., Circulation 108:1299-301 (2003) and Germain D. et al., Genetic and Medicine 23:1-8 (2013). However, as a semi-quantitative visual method was used to quantitate GL-3 microscopically in cardiac biopsies, it is conceivable that the method was not sensitive enough to see limited changes in storage.

Substrate reduction therapy (SRT) is an alternative approach that aims to reduce the synthesis of GL-3 by inhibiting GCS, the rate limiting metabolic enzyme in glycosphingolipid synthesis. In the Fabry mouse model, GCS inhibitors have been shown to reduce the levels of GL-3 in plasma, urine and tissues. See Marshall J. et al., PLos One 5:e15033 (2010). To determine whether GCS inhibitors can reduce GL-3 levels in cardiomyocytes, the effects of GCS inhibition on cellular GL-3 levels was evaluated, either at the beginning of GL-3 accumulation or when significant GL-3 storage is already present in Fabry iPSC-derived cardiomyocytes. The addition of the GCS inhibitor to cardiomyocyte cultures had no effect on viability. However, the marked accumulation of GL-3 that occurs between day 18 and day 33 in untreated cardiomyocyte cultures was prevented by GCS inhibition. Importantly, in one month old cardiomyocytes that had significant GL-3 storage at the start of treatment, maintaining the cells in culture for two weeks in the presence of the GCS inhibitor resulted in a greater than 50% reduction of GL-3 storage as measured by LC/MS, as well as a corresponding decrease in GL-3/LAMP2 content. This observation in cardiomyocytes is quite provocative as it suggests that alternative pathways for GL-3 clearance exist and that it is possible to reduce GL-3 levels in non-dividing cardiac cells. Additional work is needed to explore alpha-galactosidase independent mechanisms of GL-3 clearance from these cells, but alternative catabolic pathways or the efflux of substrate from the cell are both areas of interest.

As such, described herein is the successful generation of Fabry disease iPSC-derived cardiomyocytes. These FD cardiomyocytes have phenotypic characteristics that resemble the pathological features associated with the disease. This *in vitro* model can be used to explore both the pathogenic mechanisms of cardiac manifestations in Fabry disease, as well as potential strategies for therapeutic intervention. With respect to the latter, the above data indicates that both agalsidase beta and GCS inhibition have a positive impact on GL-3 storage in cardiomyocytes. Given the promising *in vitro* data with GCS inhibition in this disease model and its distinct mechanism of action, SRT provides an alternative or complementary approach to ERT for treating cardiac manifestations of Fabry disease.

The results reported herein were obtained using the following methods and materials.

### Fibroblasts culture

The two human untransformed Fabry fibroblasts GM00107 and GM00881, were commercially available and obtained from the Coriell Institute for Medical Research. They are from two clinically affected male patients. For clone GM00107, the patient is hemizygous for a G>A change at nucleotide 485 in exon 3 of the GLA gene (c.485G>A), resulting in a stop codon (W162X). The age of sampling was 10 years. For clone GM00881, the subject is hemizygous for a C>T change at nucleotide 658 in exon 5 of the GLA gene (c.658C>T) resulting in a stop codon (R220X). The age of sampling was 17 years. The fibroblasts were thawed and cultured in accordance to the Coriell Institute recommendations in MEM (Gibco, Carlsbad, CA) with 1x MEM non-essential amino acids (Gibco, Carlsbad, CA), 1x Penicillin-Streptomycin (Gibco, Carlsbad, CA) and 15% foetal bovine serum, 37°C, 5% CO2.

### iPSCs generation

Human STEMCCA Cre-excisable constitutive polycistronic (OKSM) lentivirus reprogramming kit (Millipore, Billerica, MA) was use to generate the Fabry iPSC. This vector includes the reprogramming transcription factors Oct-4, Klf4, SOX-2, and c-Myc. The kit was used in accordance to the recommendations of the provider. At day 0, the fibroblasts were seeded at the optimal density. On days 1 and 2, they were infected with the virus. On day 6, infected cells were plated on human feeder fibroblasts (HFF) and cultured in Knock-Out DMEM (Gibco, Carlsbad, CA), 20% KOSR (Gibco, Carlsbad, CA), 1x penicillin-streptomycin (Gibco, Carlsbad, CA), 1x Glutamax (Gibco, Carlsbad, CA), 10mM 2- mercaptoethanol (Gibco, Carlsbad, CA), supplemented with 12ng/mL FGF-2 (Miltenyi Biotech, Bergisch Gladbach). iPSC colonies were weekly passaged manually during the period of selection and expansion. Collagenase IV (STEMCELL, Palo Alto, CA) passaging was carried to generate batch of iPSC for cardiac differentiation.

### Cardiac differentiation of human iPSC

A 3D protocol of differentiation adapted from Yang L. et al., Nature 453:524-528 (2008) was used to generate iPSC-derived cardiomyocytes. For the first twelve days, the cells were maintained under hypoxic conditions (5% oxygen) and 5% CO₂. The cells were then cultured in air environment and 5% CO2 to complete the differentiation. The basal medium used consisted of StemPro 34 SFM (Gibco, Carlsbad, CA), Nutrient supplement (Gibco, Carlsbad, CA), 0.5 ng/mL BMP4 (R&D Systems, Minneapolis, MN), Glutamax (Gibco, Carlsbad, CA) and 1x penicillin-streptomycin (Gibco, Carlsbad, CA), complemented with different cytokine cocktails over time.

At day 0, six day old iPSC cultures were treated 45 minutes with collagenase IV (STEMCELL, Palo Alto, CA). Floating colonies were transferred and maintained for 7 days in ultra-low attachment culture dishes (Corning, Lowell, MA) to generate embryoid bodies (EBs). From day 1 to day 4, 10 ng/mL BMP4, 6 ng/mL bFGF and 3 ng/mL activin A (R&D Systems, Minneapolis, MN) were added in the basal medium, and then replaced by 150 ng/mL DKK1 (Peprotech, Rocky Hill, NJ) and 10 ng/mL VEGF (R&D Systems, Minneapolis, MN) from day 4 to 8. At day 7, the EBs were plated on matrigel (BD Biosciences, Franklin Lakes, NJ) coated dishes. From day 8 to day 16, the basal medium was supplemented with 10 ng/mL VEGF, 150 ng/mL DKK1 and 6 ng/mL bFGF. Beyond, the cardiomyocytes were maintained in the basal medium.

### Immunohistochemistry (IHC)

One month old beating EBs were harvested and mouse fibroblasts were added in order to facilitate handling of the samples during inclusion, cut and deposition on glass slide. Samples were fixed in a solution of phosphate-buffered saline 3.7% formalin, then dehydrated and embedded in paraffin. 5 µm thick sections were prepared. Classical IHC was performed using Ventana automatic instrument (Discovery XT, Ventana Medical Systems, Inc, Oro Valley, AZ). Sections were dewaxed, and antigen retrieval Cell Conditioning 1 (CC1) buffer (Ventana Medical Systems, Inc, Oro Valley, AZ) was first applied during 36 minutes. Afterwards, the samples were incubated with the primary antibodies for 1 hour at 37°C, followed by glutaraldehyde post-fixation. For detection, biotinylated secondary antibodies were applied for 32 minutes at room temperature. The immunostaining was detected by DAB Map Kit (Ventana Medical Systems, Inc, Oro Valley, AZ) giving brown color.

For all immunostainings, sections were subsequently counterstained with Hematoxylin for 4 minutes and bluing reagent for 4 minutes (Ventana Medical Systems, Inc, Oro Valley, AZ). Stained slides were dehydrated and cover-slipped with cytoseal XYL (Richard-Allan Scientific, Kalamazoo, Michigan).

Primary antibodies used were mouse monoclonal antibody IgG1, directed against α-Actin, at the final dilution 1/200 (Lifespan, Providence, RI), mouse monoclonal IgG2b antibody directed against Troponin 1, at the final dilution 1/500 (Millipore, Billerica, MA) and rabbit polyclonal anti α-Actinin, at the final dilution 1/50 (Lifespan, Providence, RI) and secondary antibody used were biotinylated goat anti-mouse IgG1, (Southern Biotech, Birmingham, AL), biotinylated goat anti-mouse IgG, (Jackson Immunoresearch Laboratories, West Grove, PA) and goat anti-rabbit immunoglobulin (IgG) (Jackson Immunoresearch Laboratories, West Grove, PA), respectively. Secondary antibodies were added at the final dilution 1/200.

### Immunocytochemistry (ICC)

One month old beating EBs were harvested and dissociated by using the embryoid body dissociation kit (Miltenyi Biotech, Bergisch Gladbach). Dissociated cells were plated on matrigel coated dishes. Five days latter, cells were fixed using 8% de formaldehyde solution (Sigma-Aldrich, St. Louis, MO) in DPBS (Gibco, Carlsbad, CA) and permeabilized in 0.05% Tween 20 (Sigma Life Science, St. Louis, MO) 20 minutes at room temperature.

The samples were incubated with the primary antibodies over night at 4°C. Secondary antibodies were applied one hour at room temperature.

Primary antibodies used were mouse monoclonal IgG1 directed against - Actinin, at the final dilution 1/1500 (Sigma Life Science, St. Louis, MO), rat monoclonal IgM directed against GL-3/CD77, at the final dilution 1/300 (Abcam, Cambridge, US) and mouse monoclonal IgG1 directed against LAMP2, at the final dilution 1/500 (Santa Cruz Biotechnology, Dallas, TX). The secondary antibodies were Alexa Fluor 488 goat anti-mouse IgG (Molecular Probes, Carlsbad, CA) Alexa Fluor 555 goat anti-mouse IgG (Molecular Probes, Carlsbad, CA) and Alexa Fluor 488 goat anti-rat IgG (Molecular Probes, Carlsbad, CA). They were used at the final dilution 1/1000.

### Electron microscopy

One month old beating EBs were fixed in 3% gluteraldehyde in 0.2M cacodylate buffer (Electron Microscopy Sciences, Hatfield, PA) and post-fixed in 1% osmium tetroxide and embedded in epon resin. Semi-thin sections (1 micron) were stained with Richardson's stain and examined by light microscope. Ultrathin sections (60-80 nm) were stained with uranyl-acetate and examined with a JEOL JEM-1400 transmission electron microscope (JEOL USA, Inc., Peabody, MA).

### Mass spectrometry

Pellets containing EBs were extracted in a solution of acetonitrile:methanol (1:1). The extracts were directly injected onto a 2.1x100mm, 1.7 µM BEH Hilic column (Waters), resolved with a Waters Acquity UPLC system, and analyzed for C16, C18, C20, C22, C23, C24, C24:1, C24(OH), and C24:1(OH) GL-3 isoforms and lyso-GL-3 in multiple-reaction monitoring (MRM) and positive-ion mode with an API-4000 mass spectrometer (ABSciex, Framingham, MA). Total GL-3 levels were quantitated using an external calibration curve prepared by serially diluting GL-3 natural standard (Matreya #1067) in the same extraction solution. Extracts were further diluted with acetonitrile and injected onto a 2.0x100mm, 3 µM Luna Hilic column (Phenomonex), resolved using a Waters Acquity UPLC, and analyzed for total phosphatidylcholine (PC) in precursor-ion and positive-ion mode with an API-4000 mass spectrometer. Total PC levels were also determined by calibrating against an external calibration curve prepared by serially diluting PC natural standard (Matreya #1044). Total GL-3 levels were normalized to the total PC levels for each sample.

## Claims

1. A compound for use in a method for treating globotriaosylceramide (GL-3) accumulation in the heart of a subject having or at risk of having Fabry disease, wherein administration of the compound inhibits GL-3 accumulation in a cardiomyocyte of the subject, wherein said compound is represented by the following structural formula, 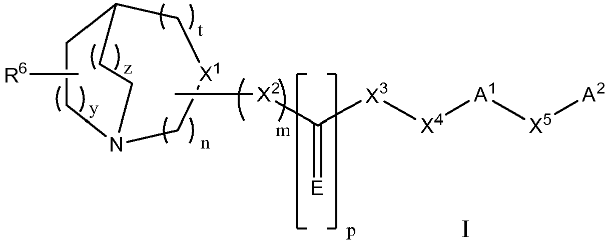 or a pharmaceutically acceptable salt or prodrug thereof, wherein:
n is 1, 2 or 3;
m is 0 or 1;
p is 0 or 1;
t is 0, 1 or 2;
y is 1 or 2;
z is 0, 1 or 2;
E is S, O, NH, NOH, NNO₂, NCN, NR, NOR or NSO₂R;
X¹ is CR¹ when m is 1 or N when m is 0;
X² is O, -NH, -CH₂-, SO₂, NH-SO₂;CH(C₁-C₆) alkyl or -NR² ;
X³ is O, -NH, -CH₂-, CO, - CH(C₁-C₆) alkyl, SO₂NH, -CO-NH- or -NR³;
X⁴ is CR⁴R⁵, CH₂ CR⁴R⁵ or CH₂ -(C₁-C₆) alkyl-CR⁴R⁵;
X⁵ is a direct bond, O, S, SO₂, CR⁴R⁵; (C₁-C₆)alkyl, (C₁-C₆)alkyloxy, (C₁-C₆)alkenyl, (C₁-C₆)alkenyloxy;
R is (C₆-C₁₂)aryl, (C₂-C₉)heteroaryl, (C₁-C₆)alkyl, (C₂-C₉)heteroaryl(C₁-C₆)alkyl;
R¹ is H, CN, (C₁-C₆)alkylcarbonyl, or (C₁-C₆)alkyl;
R² and R³ are each independently -H, (C₁-C₆)alkyl optionally substituted by one or more substituents selected from the group consisting of halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl, or optionally when X² is -NR² and X³ is -NR³, R² and R³ may be taken together with the nitrogen atoms to which they are attached form a non-aromatic heterocyclic ring optionally substituted by with one or more substituents selected from halogen, (C₁-C₆)alkyl, (C₆-C₁₂)aryl, (C₂-C₉)heteroaryl, (C₁-C₆)alkyl(C₆-C₁₂)aryl, halo(C₆-C₁₂)aryl, and halo(C₂-C₉)heteroaryl;
R⁴ and R⁵ are independently selected from H, (C₁-C₆)alkyl, or taken together with the carbon to which they are attached to form a spiro (C₃-C₁₀)cycloalkyl ring or spiro (C₃-C₁₀)cycloalkoxy ring;
R⁶ is -H, halogen, -CN, (C₆-C₁₂)aryl, (C₆-C₁₂)aryloxy, (C₁-C₆)alkyloxy; (C₁-C₆)alkyl optionally substituted by one to four halo or (C₁-C₆)alkyl;
A¹ is (C₂-C₆)alkynyl; (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkenyl, amino, (C₁-C₆)alkylamino, (C₁-C₆)dialkylamino, (C₁-C₆)alkoxy, nitro, CN, -OH, (C₁-C₆)alkyloxy optionally substituted by one to three halo; (C₁-C₆)alkoxycarbonyl, and (C₁-C₆) alkylcarbonyl;
A² is H, (C₃-C₁₀)cycloalkyl, (C₆-C₁₂)aryl, (C₂-C₉)heteroaryl, (C₂-C₉)heterocycloalkyl or benzo(C₂-C₉)heterocycloalkyl optionally substituted with one or more substituents selected from the group consisting of halo, (C₁-C₆)alkyl optionally substituted by one to three halo; (C₁-C₆)alkylenyl, amino, (C₁-C₆) alkylamino, (C₁-C₆)dialkylamino, (C₁-C₆)alkoxy, O(C3-C6 cycloalkyl), (C₃-C₆) cycloalkoxy, nitro, CN, OH, (C₁-C₆)alkyloxy optionally substituted by one to three halo; (C₃-C₆) cycloalkyl, (C₁-C₆) alkoxycarbonyl, (C₁-C₆) alkylcarbonyl, (C₁-C₆) haloalkyl;
with the proviso that the sum of n + t + y + z is not greater than 6;
with the proviso that when p is 0; X² is NH-SO₂ and X³ is NH;
with the proviso that when n is 1; t is 0; y is 1; z is 1; X² is NH; E is O; X³ is NH; A² is H and X⁵ is a direct bond; A¹ is not unsubstituted phenyl, halophenyl or isopropenyl phenyl;
with the proviso that when n is 1; t is 0; y is 1; z is 1; X² is O; E is O; X³ is NH; A¹ is (C₆-C₁₂)aryl and X⁵ is a direct bond; A² is H and R⁴ is H then R⁵ is not cyclohexyl; and
with the proviso that when n is 1; t is 0; y is 1; z is 1; X² is NH; E is O; X³ is CH₂; R⁴ and R⁵ are both hydrogen; A² is H and X⁵ is a direct bond; then A¹ is not unsubstituted phenyl.

2. The compound for use according to claim 1, wherein administration of the compound inhibits GL-3 accumulation in the lysosome of the cardiomyocyte.

3. The compound for use according to claim 1, wherein administration of the compound reduces GL-3 accumulation in a cardiomyocyte of the subject.

4. The compound for use according to claim 3, wherein administration of the compound reduces GL-3 accumulation in the lysosome of the cardiomyocyte.

5. A compound for use in a method for treating globotriaosylceramide (GL-3) accumulation in the heart of a subject having or at risk of having Fabry disease, wherein administration of the compound prevents GL-3 accumulation in a cardiomyocyte of the subject, wherein the compound is as defined in claim 1.

6. The compound for use according to claim 5, wherein administration of the compound prevents GL-3 accumulation in the lysosome of the cardiomyocyte.

7. The compound for use according to any one of claims 1-6, wherein administration of the compound inhibits glucosylceramide synthase (GCS) activity.

8. A compound for use in a method for reducing globotriaosylceramide (GL-3) in the heart of a subject having or at risk of having Fabry disease, wherein the cardiomyocytes of the subject has increased GL-3 as compared to healthy cardiomyocytes, and wherein administration of the compound reduces GL-3 in the cardiomyocytes, wherein said compound is as defined in claim 1.

9. The compound for use according to claim 8, wherein the increased GL-3 is present in the lysosomes of the cardiomyocytes.

10. The compound for use according to claim 8 or claim 9, wherein administration of the compound reduces GL-3 in the lysosome of the cardiomyocytes.

11. The compound for use according to any one of claims 8-10, wherein the cardiomyocytes of the subject comprise accumulated GL-3.

12. The compound for use according to claim 11, wherein the accumulated GL-3 is present in the lysosomes of the cardiomyocytes.

13. The compound for use according to any one of claims 8-12, wherein administration of the compound inhibits glucosylceramide synthase (GCS) activity.

14. The compound for use according to any one of claims 1-13, wherein the compound is 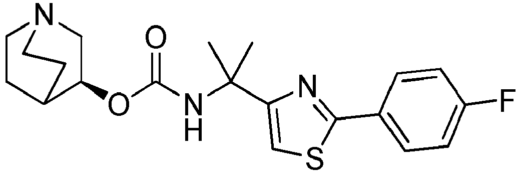 or a pharmaceutically acceptable salt or prodrug thereof.

15. The compound for use according to any one of claims 1-13, wherein the compound is 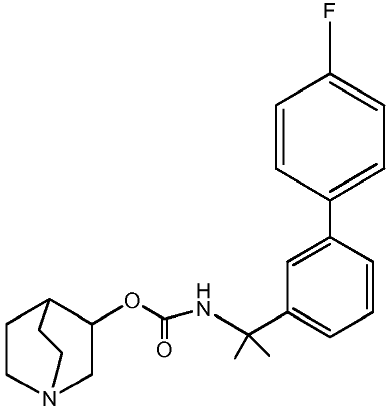 or a pharmaceutically acceptable salt or prodrug thereof.

## Patentansprüche

1. Verbindung zur Verwendung bei einer Methode zur Behandlung der Akkumulation von Globotriaosylceramid (GL-3) im Herzen eines Individuums, das an Morbus Fabry leidet oder bei dem das Risiko besteht, dass es an Morbus Fabry leidet, wobei durch die Verabreichung der Verbindung die GL-3-Akkumulation in einem Kardiomyozyten des Individuums inhibiert wird, wobei die Verbindung durch die folgende Strukturformel wiedergegeben wird, 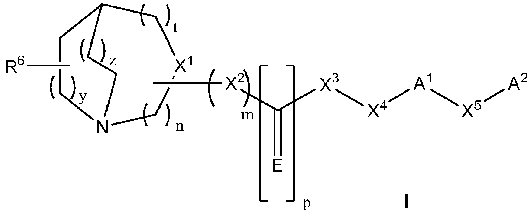 oder ein pharmazeutisch akzeptables Salz oder Prodrug davon, wobei:
n für 1, 2 oder 3 steht;
m für 0 oder 1 steht;
p für 0 oder 1 steht;
t für 0, 1 oder 2 steht;
y für 1 oder 2 steht;
z für 0, 1 oder 2 steht;
E für S, O, NH, NOH, NNO₂, NCN, NR, NOR oder NSO₂R steht;
X¹ für CR¹ steht, wenn m für 1 steht, oder für N steht, wenn m für 0 steht;
X² für O, -NH, -CH₂-, SO₂, NH-SO₂, CH-(C₁-C₆)-Alkyl oder -NR² steht;
X³ für O, -NH, -CH₂-, CO, -CH-(C₁-C₆)-Alkyl, SO₂NH, -CO-NH- oder -NR³ steht;
X⁴ für CR⁴R⁵, CH₂, CR⁴R⁵ oder CH₂- (C₁-C₆)-Alkyl-CR⁴R⁵ steht;
X⁵ für eine direkte Bindung, O, S, SO₂, CR⁴R⁵, (C₁-C₆)-Alkyl, (C₁-C₆) -Alkyloxy, (C₁-C₆) -Alkenyl, (C₁-C₆)-Alkenyloxy steht;
R für (C₆-C₁₂)-Aryl, (C₂-C₉)-Heteroaryl, (C₁-C₆) - Alkyl, (C₂-C₉)-Heteroaryl- (C₁-C₆) -alkyl steht;
R¹ für H, CN, (C₁-C₆) -Alkylcarbonyl oder (C₁-C₆) - Alkyl steht;
R² und R³ jeweils unabhängig für -H, (C₁-C₆)-Alkyl, das gegebenenfalls durch einen oder mehrere Substituenten aus der Gruppe bestehend aus Halogen, (C₁-C₆) -Alkyl, (C₆-C₁₂)-Aryl, (C₂-C₉)-Heteroaryl, (C₁-C₆) -Alkyl- (C₆-C₁₂)-aryl, Halogen (C₆-C₁₂)-aryl und Halogen (C₂-C₉)-heteroaryl substituiert ist, stehen oder gegebenenfalls dann, wenn X² für - NR² steht und X³ für -NR³ steht, R² und R³ zusammen mit den Stickstoffatomen, an die sie gebunden sind, einen nicht aromatischen heterocyclischen Ring bilden, der gegebenenfalls durch einen oder mehrere Substituenten, die aus Halogen, (C₁-C₆)-Alkyl, (C₆-C₁₂)-Aryl, (C₂-C₉)-Heteroaryl, (C₁-C₆)-Alkyl-(C₆-C₁₂)-aryl, Halogen (C₆-C₁₂)-aryl und Halogen(C₂-C₉)-heteroaryl ausgewählt sind, substituiert ist;
R⁴ und R⁵ unabhängig aus H, (C₁-C₆) -Alkyl ausgewählt sind oder zusammen mit dem Kohlenstoff, an den sie gebunden sind, einen Spiro-(C₃-C₁₀)-cycloalkylring oder Spiro-(C₃-C₁₀)-cycloalkoxyring bilden;
R⁶ für -H, Halogen, -CN, (C₆-C₁₂)-Aryl, (C₆-C₁₂)-Aryloxy, (C₁-C₆) -Alkyloxy, (C₁-C₆) -Alkyl, das gegebenenfalls durch ein bis vier Halogene oder (C₁-C₆)-Alkyl substituiert ist, steht;
A¹ für (C₂-C₆)-Alkinyl, (C₃-C₁₀)-Cycloalkyl, (C₆-C₁₂)-Aryl, (C₂-C₉)-Heteroaryl, (C₂-C₉)-Heterocycloalkyl oder Benzo-(C₂-C₉)-heterocycloalkyl, das gegebenenfalls durch einen oder mehrere Substituenten aus der Gruppe bestehend aus Halogen, (C₁-C₆) -Alkyl, das gegebenenfalls durch ein bis drei Halogene substituiert ist, substituiert ist, (C₁-C₆) -Alkenyl, Amino, (C₁-C₆)-Alkylamino, (C₁-C₆) -Dialkylamino, (C₁-C₆) -Alkoxy, Nitro, CN, -OH, (C₁-C₆)-Alkyloxy, das gegebenenfalls durch ein bis drei Halogene substituiert ist; (C₁-C₆) -Alkoxycarbonyl und (C₁-C₆) -Alkylcarbonyl substituiert ist, steht;
A² für H, (C₃-C₁₀)-Cycloalkyl, (C₆-C₁₂)-Aryl, (C₂-C₉)-Heteroaryl, (C₂-C₉)-Heterocycloalkyl oder Benzo-(C₂-C₉)-heterocycloalkyl, das gegebenenfalls durch einen oder mehrere Substituenten aus der Gruppe bestehend aus Halogen, (C₁-C₆)-Alkyl, das gegebenenfalls durch ein bis drei Halogene substituiert ist, (C₁-C₆)-Alkylenyl, Amino, (C₁-C₆)-Alkylamino, (C₁-C₆)-Dialkylamino, (C₁-C₆) -Alkoxy, O-(C₃-C₆)-Cycloalkyl, (C₃-C₆) -Cycloalkoxy, Nitro, CN, OH, (C₁-C₆)-Alkyloxy, das gegebenenfalls durch ein bis drei Halogene substituiert ist; (C₃-C₆)-Cycloalkyl, (C₁-C₆)-Alkoxycarbonyl, (C₁-C₆)-Alkylcarbonyl, (C₁-C₆)-Halogenalkyl substituiert ist, steht;
mit der Maßgabe, dass die Summe von n + t + y + z nicht größer als 6 ist;
mit der Maßgabe, dass dann, wenn p für 0 steht, X² für NH-SO₂ steht und X³ für NH steht;
mit der Maßgabe, dass dann, wenn n für 1 steht, t für 0 steht, y für 1 steht, z für 1 steht, X² für NH steht, E für O steht, X³ für NH steht, A² für H steht und X⁵ für eine direkte Bindung steht, A¹ nicht für unsubstituiertes Phenyl, Halogenphenyl oder Isopropenylphenyl steht;
mit der Maßgabe, dass dann, wenn n für 1 steht, t für 0 steht, y für 1 steht, z für 1 steht, X² für O steht, E für O steht, X³ für NH steht, A¹ für (C₆-C₁₂)-Aryl steht und X⁵ für eine direkte Bindung steht, A² für H steht und R⁴ für H steht, R⁵ nicht für Cyclohexyl steht; und
mit der Maßgabe, dass dann, wenn n für 1 steht, t für 0 steht, y für 1 steht, z für 1 steht, X² für NH steht, E für O steht, X³ für CH₂ steht, R⁴ und R⁵ beide für Wasserstoff stehen, A² für H steht und X⁵ für eine direkte Bindung steht, A¹ nicht für unsubstituiertes Phenyl steht.

2. Verbindung zur Verwendung nach Anspruch 1, wobei durch die Verabreichung der Verbindung die GL-3-Akkumulation im Lysosom des Kardiomyozyten inhibiert wird.

3. Verbindung zur Verwendung nach Anspruch 1, wobei durch die Verabreichung der Verbindung die GL-3-Akkumulation in einem Kardiomyozyten des Individuums reduziert wird.

4. Verbindung zur Verwendung nach Anspruch 3, wobei durch die Verabreichung der Verbindung die GL-3-Akkumulation im Lysosom des Kardiomyozyten reduziert wird.

5. Verbindung zur Verwendung bei einer Methode zur Behandlung der Akkumulation von Globotriaosylceramid (GL-3) im Herzen eines Individuums, das an Morbus Fabry leidet oder bei dem das Risiko besteht, dass es an Morbus Fabry leidet, wobei durch die Verabreichung der Verbindung die GL-3-Akkumulation in einem Kardiomyozyten des Individuums verhindert wird, wobei die Verbindung wie in Anspruch 1 definiert ist.

6. Verbindung zur Verwendung nach Anspruch 5, wobei durch die Verabreichung der Verbindung die GL-3-Akkumulation im Lysosom des Kardiomyozyten verhindert wird.

7. Verbindung zur Verwendung nach einem der Ansprüche 1-6, wobei durch die Verabreichung der Verbindung die Aktivität von Glucosylceramidsynthase (GCS) inhibiert wird.

8. Verbindung zur Verwendung bei einer Methode zur Verringerung von Globotriaosylceramid (GL-3) im Herzen eines Individuums, das an Morbus Fabry leidet oder bei dem das Risiko besteht, dass es an Morbus Fabry leidet, wobei die Kardiomyozyten des Individuums eine erhöhte Menge an GL-3 im Vergleich zu gesunden Kardiomyozyten aufweisen und wobei durch die Verabreichung der Verbindung GL-3 in den Kardiomyozyten reduziert wird, wobei die Verbindung wie in Anspruch 1 definiert ist.

9. Verbindung zur Verwendung nach Anspruch 8, wobei die erhöhte Menge an GL-3 in den Lysosomen der Kardiomyozyten vorliegt.

10. Verbindung zur Verwendung nach Anspruch 8 oder Anspruch 9, wobei durch die Verabreichung der Verbindung GL-3 im Lysosom der Kardiomyozyten reduziert wird.

11. Verbindung zur Verwendung nach einem der Ansprüche 8-10, wobei die Kardiomyozyten des Individuums akkumuliertes GL-3 umfassen.

12. Verbindung zur Verwendung nach Anspruch 11, wobei das akkumulierte GL-3 in den Lysosomen der Kardiomyozyten vorliegt.

13. Verbindung zur Verwendung nach einem der Ansprüche 8-12, wobei durch die Verabreichung der Verbindung die Aktivität von Glucosylceramidsynthase (GCS) inhibiert wird.

14. Verbindung zur Verwendung nach einem der Ansprüche 1-13, wobei es sich bei der Verbindung um 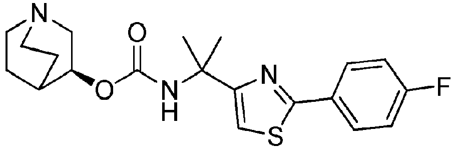 oder ein pharmazeutisch akzeptables Salz oder Prodrug davon handelt.

15. Verbindung zur Verwendung nach einem der Ansprüche 1-13, wobei es sich bei der Verbindung um 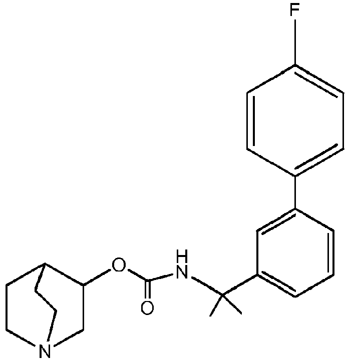 oder ein pharmazeutisch akzeptables Salz oder Prodrug davon handelt.

## Revendications

1. Composé pour une utilisation dans un procédé pour le traitement de l'accumulation de globotriaosylcéramide (GL-3) dans le cœur d'un sujet possédant ou risquant d'avoir la maladie de Fabry, une administration du composé inhibant l'accumulation de GL-3 dans un cardiomyocyte du sujet, ledit composé étant représenté par la formule structurale suivante, 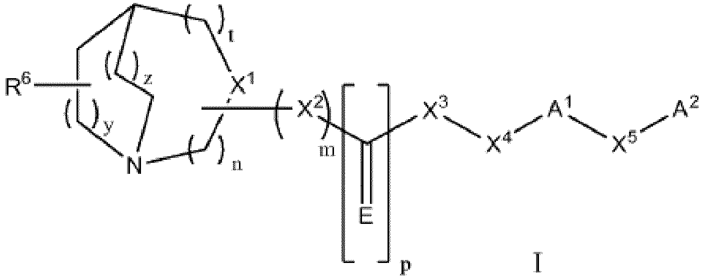 ou sel ou promédicament pharmaceutiquement acceptable correspondant :
n étant 1, 2 ou 3 ;
m étant 0 ou 1 ;
p étant 0 ou 1 ;
t étant 0, 1 ou 2 ;
y étant 1 ou 2 ;
z étant 0, 1 ou 2 ;
E étant S, O, NH, NOH, NNO₂, NCN, NR, NOR ou NSO₂R ;
X¹ étant CR¹ lorsque m est 1 ou N lorsque m est 0 ;
X² étant O, -NH, -CH₂-, SO₂, NH-SO₂ ; CH-C₁₋₆-alkyle ou -NR² ;
X³ étant O, -NH, -CH₂-, CO, -CH-C₁₋₆-alkyle, SO₂NH, -CO-NH- ou -NR³ ;
X⁴ étant CR⁴R⁵, CH₂CR⁴R⁵, ou CH₂-C₁₋₆-alkyl-CR⁴R⁵ ;
X⁵ étant une liaison directe, O, S, SO₂, CR⁴R⁵ ; C₁₋₆-alkyle, C₁₋₆-alkyloxy, C₁₋₆-alcényle, C₁₋₆-alcényloxy ;
R étant C₆₋₁₂-aryle, C₂₋₉-hétéroaryle, C₁₋₆-alkyle, C₂₋₉-hétéroaryl-C₁₋₆-alkyle ;
R¹ étant H, CN, C₁₋₆-alkylcarbonyle, ou C₁₋₆-alkyle ;
R² et R³ étant chacun indépendamment -H, C₁₋₆-alkyle éventuellement substitué par un ou plusieurs substituants choisis dans le groupe constitué par halogène, C₁₋₆-alkyle, C₆₋₁₂-aryle, C₂₋₉-hétéroaryle, C₁₋₆-alkyl-C₆₋₁₂-aryle, halogéno-C₆₋₁₂-aryle, et halogéno-C₂₋₉-hétéroaryle, ou éventuellement lorsque X² est -NR² et X³ est -NR³, R² et R³ peuvent être pris conjointement avec les atomes d'azote auxquels ils sont fixés pour former un cycle hétérocyclique non aromatique éventuellement substitué par un ou plusieurs substituants choisis parmi halogène, C₁₋₆-alkyle, C₆₋₁₂-aryle, C₂₋₉-hétéroaryle, C₁₋₆-alkyl-C₆₋₁₂-aryle, halogéno-C₆₋₁₂-aryle, et halogéno-C₂₋₉-hétéroaryle ;
R⁴ et R⁵ étant indépendamment choisis parmi H, C₁₋₆-alkyle, ou pris conjointement avec le carbone auquel ils sont fixés pour former un cycle spiro-C₃₋₁₀-cycloalkyle ou un cycle spiro-C₃₋₁₀-cycloalcoxy ;
R⁶ étant -H, halogène, -CN, C₆₋₁₂-aryle, C₆₋₁₂-aryloxy, C₁₋₆-alkyloxy ; C₁₋₆-alkyle éventuellement substitué par un à quatre halogéno ou C₁₋₆-alkyle ;
A¹ étant C₂₋₆-alcynyle ; C₃₋₁₀-cycloalkyle, C₆₋₁₂-aryle, C₂₋₉-hétéroaryle, C₂₋₉-hétérocycloalkyle ou benzo-C₂₋₉-hétérocycloalkyle éventuellement substitué par un ou plusieurs substituants choisis dans le groupe constitué par halogéno, C₁₋₆-alkyle éventuellement substitué par un à trois halogéno ; C₁₋₆-alcényle, amino, C₁₋₆-alkylamino, C₁₋₆-dialkylamino, C₁₋₆-alcoxy, nitro, CN, -OH, C₁₋₆-alkyloxy éventuellement substitué par un à trois halogéno ; C₁₋₆-alcoxycarbonyle, et C₁₋₆-alkylcarbonyle ;
A² étant H, C₃₋₁₀-cycloalkyle, C₆₋₁₂-aryle, C₂₋₉-hétéroaryle, C₂₋₉-hétérocycloalkyle ou benzo-C₂₋₉-hétérocycloalkyle éventuellement substitué par un ou plusieurs substituants choisis dans le groupe constitué par halogéno, C₁₋₆-alkyle éventuellement substitué par un à trois halogéno ; C₁₋₆-alkylényle, amino, C₁₋₆-alkylamino, C₁₋₆-dialkylamino, C₁₋₆-alcoxy, O(C₃₋₆-cycloalkyle), C₃₋₆-cycloalcoxy, nitro, CN, OH, C₁₋₆-alkyloxy éventuellement substitué par un à trois halogéno ; C₃₋₆-cycloalkyle, C₁₋₆-alcoxycarbonyle, C₁₋₆-alkylcarbonyle, C₁₋₆-halogénoalkyle ;
étant entendu que la somme de n + t + y + z n'est pas supérieure à 6 ;
étant entendu que lorsque p est 0 ; X² est NH-SO₂ et X³ est NH ;
étant entendu que lorsque n est 1 ; t est 0 ; y est 1 ; z est 1 ; X² est NH ; E est O ; X³ est NH ; A² est H et X⁵ est une liaison directe ; A¹ n'est pas phényle non substitué, halogénophényle ou isopropénylphényle ;
étant entendu que lorsque n est 1 ; t est 0 ; y est 1 ; z est 1 ; X² est O ; E est O ; X³ est NH ; A¹ est C₆₋₁₂-aryle et X⁵ est une liaison directe ; A² est H et R⁴ est H alors R⁵ n'est pas cyclohexyle ; et
étant entendu que lorsque n est 1 ; t est 0 ; y est 1 ; z est 1 ; X² est NH ; E est O ; X³ est CH₂ ; R⁴ et R⁵ sont tous deux hydrogène ; A² est H et X⁵ est une liaison directe ; alors A¹ n'est pas phényle non substitué.

2. Composé pour une utilisation selon la revendication 1, une administration du composé inhibant l'accumulation de GL-3 dans le lysosome du cardiomyocyte.

3. Composé pour une utilisation selon la revendication 1, une administration du composé réduisant l'accumulation de GL-3 dans un cardiomyocyte du sujet.

4. Composé pour une utilisation selon la revendication 3, une administration du composé réduisant l'accumulation de GL-3 dans le lysosome du cardiomyocyte.

5. Composé pour une utilisation dans un procédé pour le traitement de l'accumulation de globotriaosylcéramide (GL-3) dans le cœur d'un sujet possédant ou risquant d'avoir la maladie de Fabry, une administration du composé prévenant l'accumulation de GL-3 dans un cardiomyocyte du sujet, le composé étant tel que défini dans la revendication 1.

6. Composé pour une utilisation selon la revendication 5, une administration du composé prévenant l'accumulation de GL-3 dans le lysosome du cardiomyocyte.

7. Composé pour une utilisation selon l'une quelconque des revendications 1 à 6, une administration du composé inhibant l'activité de la glucosylcéramide synthase (GCS).

8. Composé pour une utilisation dans un procédé pour la réduction de globotriaosylcéramide (GL-3) dans le cœur d'un sujet possédant ou risquant d'avoir la maladie de Fabry, les cardiomyocytes du sujet possédant une quantité plus élevée de GL-3 par comparaison avec des cardiomyocytes sains, et une administration du composé réduisant le GL-3 dans les cardiomyocytes, ledit composé étant tel que défini dans la revendication 1.

9. Composé pour une utilisation selon la revendication 8, la quantité plus élevée de GL-3 étant présente dans les lysosomes des cardiomyocytes.

10. Composé pour une utilisation selon la revendication 8 ou la revendication 9, une administration du composé réduisant le GL-3 dans le lysosome des cardiomyocytes.

11. Composé pour une utilisation selon l'une quelconque des revendications 8 à 10, les cardiomyocytes du sujet comprenant du GL-3 accumulé.

12. Composé pour une utilisation selon la revendication 11, le GL-3 accumulé étant présent dans les lysosomes des cardiomyocytes.

13. Composé pour une utilisation selon l'une quelconque des revendications 8 à 12, une administration du composé inhibant l'activité de la glucosylcéramide synthase (GCS).

14. Composé pour une utilisation selon l'une quelconque des revendications 1 à 13, le composé étant 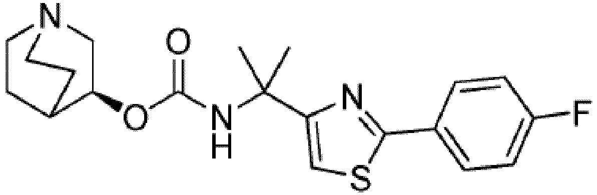 ou sel ou promédicament pharmaceutiquement acceptable correspondant.

15. Composé pour une utilisation selon l'une quelconque des revendications 1 à 13, le composé étant 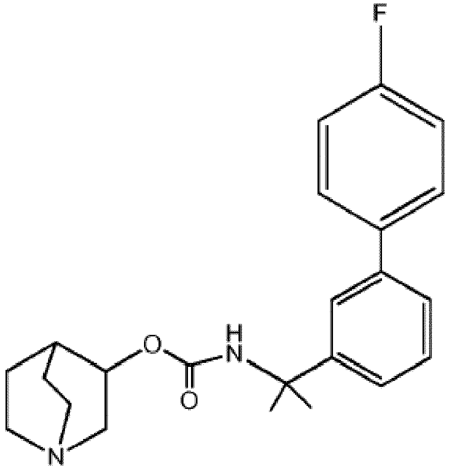 ou sel ou promédicament pharmaceutiquement acceptable correspondant.
